(19) Europäisches Patentamt
European Patent Office
Office européen des brevets

(11) EP 3 356 404 B1

(12) **EUROPEAN PATENT SPECIFICATION**

(45) Date of publication and mention of the grant of the patent:
**18.08.2021 Bulletin 2021/33**

(21) Application number: **16774499.4**

(22) Date of filing: **29.09.2016**

(51) Int Cl.:
***A61P 35/00*** *(2006.01)* ***A61P 37/04*** *(2006.01)*
***C07K 16/28*** *(2006.01)*

(86) International application number:
**PCT/EP2016/073248**

(87) International publication number:
**WO 2017/055443 (06.04.2017 Gazette 2017/14)**

(54) **ANTI-PD1 ANTIBODIES AND METHODS OF USE**

ANTI-PD1-ANTIKÖRPER UND VERFAHREN ZUR VERWENDUNG

ANTICORPS ANTI-PD1 ET PROCÉDÉS D'UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Validation States:
**MA**

(30) Priority: **02.10.2015 EP 15188061**

(43) Date of publication of application:
**08.08.2018 Bulletin 2018/32**

(73) Proprietor: **F. Hoffmann-La Roche AG**
**4070 Basel (CH)**

(72) Inventors:
- **SEEBER, Stefan**
**82404 Sindelsdorf (DE)**
- **LIFKE, Valeria**
**82377 Penzberg (DE)**
- **FISCHER, Jens**
**82362 Weilheim in Oberbayern (DE)**
- **WEISER, Barbara**
**82377 Penzberg (DE)**
- **WUENSCHE, Ildiko**
**82467 Garmisch-Partenkirchen (DE)**
- **PLOETTNER, Oliver**
**82205 Gilching (DE)**
- **ZWICK, Adrian**
**82377 Penzberg (DE)**
- **GEORGES, Guy**
**82392 Habach (DE)**
- **DENGL, Stefan**
**82538 Geretsried (DE)**
- **LEVITSKI, Viktor**
**8903 Birmensdorf (CH)**
- **KLEIN, Christian**
**8906 Bonstetten (CH)**
- **CODARRI DEAK, Laura**
**8804 Au (CH)**
- **FENN, Sebastian**
**82547 Achmuehle/Eurasburg (DE)**
- **BENZ, Joerg**
**79618 Rheinfelden (DE)**

(74) Representative: **Burger, Alexander et al**
**Roche Diagnostics GmbH**
**Patentabteilung**
**Nonnenwald 2**
**82377 Penzberg (DE)**

(56) References cited:
**WO-A1-2011/110604 WO-A1-2011/110621**
**WO-A1-2012/145493 WO-A2-2010/036959**

- **PAOLO A. ASCIERTO ET AL: "2015: The Year of Anti-PD-1/PD-L1s Against Melanoma and Beyond", EBIOMEDICINE, vol. 2, no. 2, 1 February 2015 (2015-02-01), pages 92-93, XP055255406, ISSN: 2352-3964, DOI: 10.1016/j.ebiom.2015.01.011**
- **G. K. PHILIPS ET AL: "Therapeutic uses of anti-PD-1 and anti-PD-L1 antibodies", INTERNATIONAL IMMUNOLOGY., vol. 27, no. 1, 16 October 2014 (2014-10-16), pages 39-46, XP055217958, GB ISSN: 0953-8178, DOI: 10.1093/intimm/dxu095**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 356 404 B1

- KIM C. OHAEGBULAM ET AL: "Human cancer immunotherapy with antibodies to the PD-1 and PD-L1 pathway", TRENDS IN MOLECULAR MEDICINE, vol. 21, no. 1, 1 January 2015 (2015-01-01), pages 24-33, XP055249717, GB ISSN: 1471-4914, DOI: 10.1016/j.molmed.2014.10.009
- S. L. TOPALIAN ET AL: "Survival, Durable Tumor Remission, and Long-Term Safety in Patients With Advanced Melanoma Receiving Nivolumab", JOURNAL OF CLINICAL ONCOLOGY, vol. 32, no. 10, 3 March 2014 (2014-03-03) , pages 1020-1030, XP055218601, US ISSN: 0732-183X, DOI: 10.1200/JCO.2013.53.0105
- J. R. BRAHMER ET AL: "Phase I Study of Single-Agent Anti-Programmed Death-1 (MDX-1106) in Refractory Solid Tumors: Safety, Clinical Activity, Pharmacodynamics, and Immunologic Correlates", JOURNAL OF CLINICAL ONCOLOGY, vol. 28, no. 19, 1 June 2010 (2010-06-01), pages 3167-3175, XP055124332, ISSN: 0732-183X, DOI: 10.1200/JCO.2009.26.7609
- AGATA Y ET AL: "EXPRESSION OF THE PD-1 ANTIGEN ON THE SURFACE OF STIMULATED MOUSE T AND B LYMPHOCYTES", INTERNATIONAL IMMUNOLOGY, OXFORD UNIVERSITY PRESS, GB, vol. 8, no. 5, 1 May 1996 (1996-05-01), pages 765-772, XP000971773, ISSN: 0953-8178

**Description**

## FIELD OF THE INVENTION

The present invention relates to anti-PD1 antibodies and methods of using the same.

## BACKGROUND

PD-1

Co-stimulation or the provision of two distinct signals to T-cells is a widely accepted model of lymphocyte activation of resting T lymphocytes by antigen-presenting cells (APCs) (Lafferty et al., Aust. J. Exp. Biol. Med. Sci. 53: 27-42 (1975)).

This model further provides for the discrimination of self from non-self and immune tolerance (Bretscher et al., Science 169: 1042-1049 (1970); Bretscher, P.A., P.N.A.S. USA 96: 185-190 (1999); Jenkins et al., J. Exp. Med. 165: 302-319 (1987)). The primary signal, or antigen specific signal, is transduced through the T-cell receptor (TCR) following recognition of foreign antigen peptide presented in the context of the major histocompatibility-complex (MHC). The second or co-stimulatory signal is delivered to T-cells by co-stimulatory molecules expressed on antigen-presenting cells (APCs), and induces T-cells to promote clonal expansion, cytokine secretion and effector function (Lenschow et al., Ann. Rev. Immunol. 14:233 (1996)). In the absence of co-stimulation, T-cells can become refractory to antigen stimulation, do not mount an effective immune response, and further may result in exhaustion or tolerance to foreign antigens.

The simple two-signal model can be an oversimplification because the strength of the TCR signal actually has a quantitative influence on T-cell activation and differentiation (Viola et al., Science 273: 104-106 (1996); Sloan-Lancaster, Nature 363: 156-159 (1993)). Moreover, T-cell activation can occur even in the absence of co-stimulatory signals if the TCR signal strength is high. More importantly, T-cells receive both positive and negative secondary co-stimulatory signals. The regulation of such positive and negative signals is critical to maximize the host's protective immune responses, while maintaining immune tolerance and preventing autoimmunity.

Negative secondary signals seem necessary for induction of T-cell tolerance, while positive signals promote T-cell activation. While the simple two-signal model still provides a valid explanation for naive lymphocytes, a host's immune response is a dynamic process, and co-stimulatory signals can also be provided to antigen-exposed T-cells.

The mechanism of co-stimulation is of therapeutic interest because the manipulation of co-stimulatory signals has shown to provide a means to either enhance or terminate cell-based immune response. Recently, it has been discovered that T cell dysfunction or anergy occurs concurrently with an induced and sustained expression of the inhibitory receptor, programmed death 1 polypeptide (PD-1). As a result, therapeutic targeting of PD-1 is an area of intense interest.

The protein Programmed Death 1 (PD-1) is an inhibitory member of the CD28 family of receptors, that also includes CD28, CTLA-4, ICOS and BTLA. PD-1 is expressed on activated B cells, T cells, and myeloid cells (Agata et al, supra; Okazaki et al (2002) Curr. Opin. Immunol. 14: 391779-82; Bennett et al. (2003) J Immunol 170:711-8). The initial members of the family, CD28 and ICOS, were discovered by functional effects on augmenting T cell proliferation following the addition of monoclonal antibodies (Hutloff etal (1999) Nature 397:263-266; Hansen et al (1980) Immunogenics 10:247-260). PD-1 was discovered through screening for differential expression in apototic cells (Ishida et al (1992) EMBO J 11 :3887-95). The other members of the family, CTLA-4, and BTLA were discovered through screening for differential expression in cytotoxic T lymphocytes and TH1 cells, respectively. CD28, ICOS and CTLA-4 all have an unpaired cysteine residue allowing for homodimerization. In contrast, PD-1 is suggested to exist as a monomer, lacking the unpaired cysteine residue characteristic in other CD28 family members.

The PD-1 gene is a 55 kDa type I transmembrane protein that is part of the Ig gene superfamily (Agata et al. (1996) bit Immunol 8:765-72). PD-1 contains a membrane proximal immunoreceptor tyrosine inhibitory motif (ITIM) and a membrane distal tyrosine- based switch motif (ITSM) (Thomas, MX. (\995) J Exp A4edW,: 1953-6; Vivier, E and Daeron, M (1997) Immunol Today 18:286-91). Although structurally similar to CTLA-4, PD-1 lacks the MYPPPY motif that is critical for B7-1 and B7-2 binding. Two ligands for PD-1 have been identified, PD-L1 and PD-L2, that have been shown to downregulate T cell activation upon binding to PD-1 (Freeman et al (2000) J Exp Med 192: 1027-34; Latchman et al (2001) Nat Immunol 2:261-8; Carter etal (2002) Eur J Immunol 32:634-43). Both PD-L1 and PD-L2 are B7 homologs that bind to PD-1, but do not bind to other CD28 family members. One ligand for PD-1, PD-L1 is abundant in a variety of human cancers (Dong et al (2002) Nat. Med 8:787-9). The interaction between PD-1 and PD-L1 results in a decrease in tumor infiltrating lymphocytes, a decrease in T-cell receptor mediated proliferation, and immune evasion by the cancerous cells (Dong et al. (2003) J. Mol. Med. 81:281-7; Blank et al. (2005) Cancer Immunol. Immunother. 54:307-314; Konishi et al. (2004) Clin. Cancer Res. 10:5094-100). Immune suppression can be reversed by inhibiting the local interaction of PD-1 with PD-L1, and the effect is additive when the interaction of PD-1 with PD-L2 is blocked as well (Iwai et al. (2002) Proc. Nat 7. Acad. ScL USA 99: 12293-7; Brown et al. (2003) J. Immunol. 170:1257-66).

PD1 is an inhibitory member of the CD28 family expressed on activated B cells, T cells, and myeloid cells (Agata etal, supra; Okazaki et al. (2002) Curr Opin Immunol 14: 391779-82; Bennett et al. (2003) J Immunol YWJ1 1-8). PD-I deficient animals develop various autoimmune phenotypes, including autoimmune cardiomyopathy and a lupus-like syndrome with arthritis and nephritis (Nishimura et al. (1999) Immunity H: 141-51; Nishimura et al. (2001) Science 291:319-22). Additionally, PD1 has been found to play a role in autoimmune encephalomyelitis, systemic lupus erythematosus, graft-versus-host disease (GVHD), type I diabetes, and rheumatoid arthritis (Salama et al. (2003) J Exp Med 198:71-78: Prokunina and Alarcon-Riquelme (2004) Hum Mol Genet 13_:R143; Nielsen et al. (2004) Lupus 11:510). In a murine B cell tumor line, the ITSM of PD1 was shown to be essential to block BCR-mediated Ca<2+>-flux and tyrosine phosphorylation of downstream effector molecules (Okazaki etal. (2001) PNAS 98: 13866-71).

Various patent applications disclose production of anti-PD-1 antibodies and/or methods of enhancing immune responses with an agent (including an anti-PD-1 antibody) that interferes with PD-L1 binding and/or PD-1 signaling, including the following: US2003/0039653, US2004/0213795, US2006/0110383, US2007/0065427, US2007/0122378, US2012/237522, WO2004/072286, WO2006/121168, WO2006/133396, WO2007/005874, WO2008/083174, WO2008/156712, WO2009/024531, WO2009/014708, WO2009/114335, WO2010/027828, WO2010/027423, WO2010/036959, WO2010/029435, WO2010/029434, WO2010/063011, WO2010/089411, WO2011/066342, WO2011/110604, WO2011/110621, and WO2012/145493.

## SUMMARY

The invention is defined in the appended claims and any other aspects set forth herein not falling within the scope of the claims are for information only.

The invention provides

The invention further provides an isolated antibody that binds to human PD1, wherein the antibody

i) comprises a VH sequence of SEQ ID NO:57 and a VL sequence of SEQ ID NO:58, or

ii) comprises a VH sequence of SEQ ID NO:57 and a VL sequence of SEQ ID NO:59, or

iii) comprises a VH sequence of SEQ ID NO:57 and a VL sequence of SEQ ID NO:60, or

iv) comprises a VH sequence of SEQ ID NO:57 and a VL sequence of SEQ ID NO:61.

In one embodiment the anti-PDl antibody is a full length IgG1 antibody with mutations L234A, L235A and P329G (numbering according to the EU index of Kabat)

In one embodiment the anti-PD1 antibody according to the invention is a monoclonal antibody.

In one embodiment the anti-PDl antibody according to the invention is a human, humanized, or chimeric antibody.

In one embodiment the anti-PDl antibody according to the invention which is an antibody fragment that binds to PD1.

In one embodiment the anti-PDl antibody according to the invention which is Fab fragment.

The invention provides an isolated nucleic acid encoding the antibody according to any one of the preceding claims.

The invention provides a host cell comprising such nucleic acid.

The invention provides a method of producing an antibody comprising culturing the host cell so that the antibody is produced.

The invention provides such method of producing an antibody, further comprising recovering the antibody from the host cell.

The invention provides a pharmaceutical formulation comprising the antibody described herein and a pharmaceutically acceptable carrier.

The invention provides the antibody described herein for use as a medicament.

The invention provides the antibody described herein for use in treating cancer.

## BRIEF DESCRIPTION OF THE FIGURES

**Figure 1:** Blockade of PD1 with chimeric PD1-0103 strongly enhances IFN-gamma secretion by allogenic stimulated primary human T cells.

**Figure 2:** Blockade of PD1 with chimeric PD1-0103 strongly increases interferon-gamma (IFN-g) secretion by allogenic stimulated primary human T cells.

**Figure 3:** Blockade of PD1 with chimeric PD1-0103 strongly increases tumor necrosis factor alpha (TNF) secretion by allogenic stimulated primary human T cells.

**Figure 4:** 4A) frequency of CD4 T cells producing Granzyme B and 4B) Amount of IFN-$\gamma$ detected by absorbance (Optical Density, O.D.) in the supernatant of the MLR in presence of increasing concentrations of different anti-PD-1 antibodies.

**Figure 5:** 5A) Impact of PD1/PD-L1 blockade on reactivation of suppressed T cell receptor signalig in presence of different anti-PD-1 antibodies 5B) Impact of PD1/PD-L1 blockade on reactivation of suppressed T cell receptor signalig in presence of different anti-PD-1 antibodies.

**Figure 6:** Structure of PD1-ECD in complex with Fab of PD1-0103.

**Figure 7:** Structure of PD1-ECD complex with Fab PD1-0103: *Glycosylation at ASN58 on PD1 is involved in the interaction.*

**Figure 8:** Structure of PD1-ECD complex Structure of PD1-ECD complex with Fab PD1-0103: *View on epitope/paratop.*

**Figure 9:** Contacts PD1 core sugar side chain at Asn58 - Fab PD1-0103 Heavy chain: contacts identified by distance cutoff of 5Å.

**Figure 10:** Residues of PD1-ECD that are interacting with the *antibody-Sequence view with detailed contact properties - PD-1.*

**Figure 11:** Residues of the antibody that are interacting with PD1-ECD *-Sequence view with detailed contact properties - heavy chain.*

**Figure 12:** Residues of the antibody that are interacting with *PD1-ECD-Sequence view with detailed contact properties - light chain.*

**Figure 13A:** Binding of different antibodies to PD1 aglycosylated at Asn58 (left) and to PD1 glycosylated at Asn58 (right) (Biacore sensorgramms).

**Figure 13B:** Binding of different antibodies to PD1 aglycosylated at Asn58 and to PD1 glycosylated at Asn58 - On-off-rate mab determined by Biacore.

**Figure 14A:** In vivo tumor growth inhibition of PD1-0103-0312 (aPD-1) compared to nivolumab in combination with a bispecific CEA-CD3 antibody - at high doses.

**Figure 14B:** In vivo tumor growth inhibition of PD1-0103-0312 (aPD-1) compared to nivolumab in combination with a bispecific CEA-CD3 antibody - at high doses.

## DETAILED DESCRIPTION OF EMBODIMENTS OF THE INVENTION

The invention is defined in the appended claims and any other aspects set forth herein not falling within the scope of the claims are for information only.

An "acceptor human framework" for the purposes herein is a framework comprising the amino acid sequence of a light chain variable domain (VL) framework or a heavy chain variable domain (VH) framework derived from a human immunoglobulin framework or a human consensus framework, as defined below. An acceptor human framework "derived from" a human immunoglobulin framework or a human consensus framework may comprise the same amino acid sequence thereof, or it may contain amino acid sequence changes. In some embodiments, the number of amino acid changes are 10 or less, 9 or less, 8 or less, 7 or less, 6 or less, 5 or less, 4 or less, 3 or less, or 2 or less. In some embodiments, the VL acceptor human framework is identical in sequence to the VL human immunoglobulin framework sequence or human consensus framework sequence.

When used herein, the term "PD1", "human PD1", "PD-1" or "human PD-1" refers to the human protein PD1 (SEQ ID NO: 68) (protein without signal sequence) / (SEQ ID NO: 70)( protein with signal sequence). As used herein, an antibody "binding to human PD1", "specifically binding to human PD1", "that binds to human PD1" or "anti-PD1 antibody" refers to an antibody specifically binding to the human PD1 antigen or its Extracellular Domain (ECD) with a binding affinity of a $K_D$-value of 1.0 x $10^{-8}$ mol/1 or lower, in one embodiment of a $K_D$-value of 1.0 x $10^{-9}$mol/l or lower, in one embodiment of a $K_D$-value of 1.0 x $10^{-9}$ mol/l to 1.0 x $10^{-13}$ mol/l. The binding affinity is determined with a standard binding assay, such as surface plasmon resonance technique (BIAcore®, GE-Healthcare Uppsala, Sweden) e.g. using the PD1 extracellular domain.

Human PD1 has N-linked glycosylation sites at PD-1 residues 49, 58, 74 of SEQ ID NO. 70 (see e.g.D.Y. Lin et al, PNAS 105 (2008) 3011-3016)). The core sugar chain (N-linked glycosylation) tree at position Asn58 of PD-1 has the following structure with respect to the monosaccharides. In one embodiment the core sugar chain at Asn58 of PD1 refers to the first 5 sugars (monosaccharides) which are attached to PD1 at Asn58.

Asn58-N-GlcNAc(FUC) - GlcNAc- - BMA - MAN ( see Figure 9) wherein the following abbreviations are used.

[GlcNAc]= NGA = N-acetyl-beta-D-galactosamine = 2-(acetylamino)-2-deoxy-beta-D-galactopyranose

[FUC] = alpha-L-fucose

[BMA] = beta-D-mannopyranose

[MAN] = alpha-D-mannopyranose

The first GlcNAC in the sugar chain is fucosylated which abbreviated as GlcNAc(FUC).

In one embodiment the core sugar chain at Asn58 of PD1 refers to the first 5 sugars (monosaccharides) GlcNAc, FUC, GlcNAc, BMA, MAN which are attached to PD1 at Asn58.

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, $F(ab')_2$; diabodies; linear antibodies; single-chain antibody molecules (e.g. scFv); and multispecific antibodies formed from antibody fragments.

An "antibody that binds to the same epitope" as a reference antibody refers to an antibody that blocks binding of the reference antibody to its antigen in a competition assay by 50% or more, and conversely, the reference antibody blocks binding of the antibody to its antigen in a competition assay by 50% or more. An exemplary competition assay is provided herein.

The term "chimeric" antibody refers to an antibody in which a portion of the heavy and/or light chain is derived from a particular source or species, while the remainder of the heavy and/or light chain is derived from a different source or species.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., $IgG_1$, $IgG_2$, $IgG_3$, $IgG_4$, $IgA_1$, and $IgA_2$. The heavy chain constant domains that correspond to the different classes of immunoglobulins are called $\alpha$, $\delta$, $\varepsilon$, $\gamma$, and $\mu$, respectively.

The term "cytotoxic agent" as used herein refers to a substance that inhibits or prevents a cellular function and/or causes cell death or destruction. Cytotoxic agents include, but are not limited to, radioactive isotopes (e.g., At211, 1131, 1125, Y90, Re186, Re188, Sm153, Bi212, P32, Pb212 and radioactive isotopes of Lu); chemotherapeutic agents or drugs (e.g., methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed below.

An "effective amount" of an agent, e.g., a pharmaceutical formulation, refers to an amount effective, at dosages and for periods of time necessary, to achieve the desired therapeutic or prophylactic result.

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat, E.A. et al., Sequences of Proteins of Immunological Interest, 5th ed., Public Health Service, National Institutes of Health, Bethesda, MD (1991), NIH Publication 91-3242.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The terms "full length antibody," "intact antibody," and "whole antibody" are used herein interchangeably to refer to an antibody having a structure substantially similar to a native antibody structure or having heavy chains that contain an Fc region as defined herein.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

A "human consensus framework" is a framework which represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat, E.A. et al., Sequences of Proteins of Immunological Interest, 5th ed., Bethesda MD (1991), NIH Publication 91-3242, Vols. 1-3. In one embodiment, for the VL, the subgroup is subgroup kappa I as in Kabat et al., *supra.* In one embodiment, for the VH, the subgroup is subgroup III as in Kabat et al., *supra.*

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

The term "hypervariable region" or "HVR" as used herein refers to each of the regions of an antibody variable domain which are hypervariable in sequence ("complementarity determining regions" or "CDRs") and/or form structurally defined loops ("hypervariable loops") and/or contain the antigen-contacting residues ("antigen contacts"). Generally, antibodies comprise six HVRs: three in the VH (HI, H2, H3), and three in the VL (L1, L2, L3). Exemplary HVRs herein include:

(a) hypervariable loops occurring at amino acid residues 26-32 (L1), 50-52 (L2), 91-96 (L3), 26-32 (H1), 53-55 (H2), and 96-101 (H3) (Chothia and Lesk, J. Mol. Biol. 196:901-917 (1987));

(b) CDRs occurring at amino acid residues 24-34 (L1), 50-56 (L2), 89-97 (L3), 31-35b (H1), 50-65 (H2), and 95-102 (H3) (Kabat et al., Sequences of Proteins of Immunological Interest, 5th Ed. Public Health Service, National Institutes of Health, Bethesda, MD (1991));

(c) antigen contacts occurring at amino acid residues 27c-36 (L1), 46-55 (L2), 89-96 (L3), 30-35b (H1), 47-58 (H2), and 93-101 (H3) (MacCallum et al. J. Mol. Biol. 262: 732-745 (1996)); and

(d) combinations of (a), (b), and/or (c), including HVR amino acid residues 46-56 (L2), 47-56 (L2), 48-56 (L2), 49-56 (L2), 26-35 (H1), 26-35b (H1), 49-65 (H2), 93-102 (H3), and 94-102 (H3).

Unless otherwise indicated, HVR residues and other residues in the variable domain (e.g., FR residues) are numbered herein according to Kabat et al., *supra.*

An "immunoconjugate" is an antibody conjugated to one or more heterologous molecule(s), including but not limited to a cytotoxic agent.

An "individual" or "subject" is a mammal. Mammals include, but are not limited to, domesticated animals (e.g.,

cows, sheep, cats, dogs, and horses), primates (e.g., humans and non-human primates such as monkeys), rabbits, and rodents (e.g., mice and rats). In certain embodiments, the individual or subject is a human.

An "isolated" antibody is one which has been separated from a component of its natural environment. In some embodiments, an antibody is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity see, e.g., Flatman, S. et al., J. Chromatogr. B 848 (2007) 79-87.

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

"Isolated nucleic acid encoding an anti-PD1 antibody" refers to one or more nucleic acid molecules encoding antibody heavy and light chains (or fragments thereof), including such nucleic acid molecule(s) in a single vector or separate vectors, and such nucleic acid molecule(s) present at one or more locations in a host cell.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

A "naked antibody" refers to an antibody that is not conjugated to a heterologous moiety (e.g., a cytotoxic moiety) or radiolabel. The naked antibody may be present in a pharmaceutical formulation. (Include if Prior art has immunoconjugates)

"Native antibodies" refer to naturally occurring immunoglobulin molecules with varying structures. For example, native IgG antibodies are heterotetrameric glycoproteins of about 150,000 daltons, composed of two identical light chains and two identical heavy chains that are disulfide-bonded. From N- to C-terminus, each heavy chain has a variable region (VH), also called a variable heavy domain or a heavy chain variable domain, followed by three constant domains (CH1, CH2, and CH3). Similarly, from N- to C-terminus, each light chain has a variable region (VL), also called a variable light domain or a light chain variable domain, followed by a constant light (CL) domain. The light chain of an antibody may be assigned to one of two types, called kappa ($\kappa$) and lambda ($\lambda$), based on the amino acid sequence of its constant domain.

The term "package insert" is used to refer to instructions customarily included in commercial packages of therapeutic products, that contain information about the indications, usage, dosage, administration, combination therapy, contraindications and/or warnings concerning the use of such therapeutic products.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

100 times the fraction X/Y

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject., A pharmaceutically acceptable carrier includes, but is not limited to, a buffer, excipient, stabilizer, or preservative.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies of the invention are used to delay development of a disease or to slow the progression of a disease.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, e.g., Kindt, T.J. et al. Kuby Immunology, 6th ed., W.H. Freeman and Co., N.Y. (2007), page 91) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See e.g., Portolano, S. et al., J. Immunol. 150 (1993) 880-887; Clackson, T. et al., Nature 352 (1991) 624-628).

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors".

## I. COMPOSITIONS AND METHODS

In one aspect, the invention is based, in part, on the finding that the selected anti-PD1 antibodies of the invention bind to certain epitopes of PD1 , and have ability to increase the activation of different immune cells (e.g. T-cells, B-cells, NK cells, dendritic cells (DC), monocytes and macrophages). E.g. they increase immunemodulating cytokines ( e.g. interferon gamma and granzyme B) release (secretion). Other immunemodulating cytokines which are or can be increased are e.g tumor necrosis factor alpha (TNF alpha) secretion and IL-12. As used herein the the terms interferon-gamma (IFN-gamma) tumor necrosis factor alpha (TNF alpha) secretion, IL-12 etc refer to the human cytokines.

In certain embodiments, antibodies that bind to PD1 are provided. Antibodies of the invention are useful, e.g., for the diagnosis or treatment of cancer.

### A. Exemplary Anti-PD1Antibodies

In one aspect, the invention provides isolated antibodies that bind to human PD1.

In certain embodiments, an anti-PD1 is provided wherein the antibody:

i) competes for binding to PD-1 with an anti-PDl antibody comprising the VH and VL of PD1-0103, and

ii) binds to a human and cynomolguoes PD-1; and

iii) enhances the interferon-gamma (IFN-gamma) secretion by allogenic stimulated T cells by 85% or more (in one preferred embodiment by 90% or more, in one preferred embodiment by 95% or more) at an antibody concentration of 10μg/ml (wherein the secretion without antibody is set as 0% (basal level of IFN gamma) and the secretion with 20 EU/ ml recombinant human IL-2 is set as 100% (in a (allogenic) Mixed lymphocyte reaction (MLR) assay according to Example 3); and/ or

iv) enhances the tumor necrosis factor alpha (TNF alpha) secretion by allogenic stimulated T cells by 200% or more (in one preferred embodiment by 250% or more) at an antibody concentration of 10μg/ml (wherein the secretion without antibody is set as 0% (basal level of IFN gamma) and the secretion with 20 EU/ ml recombinant human IL-2 is is set as 100% (in a (allogenic) Mixed lymphocyte reaction (MLR) assay according to Example 3).

In one aspect, the disclosure provides an anti-PD1 antibody comprising (a) HVR-H1 comprising the amino acid sequence of SEQ ID NO: 1; (b) HVR-H2 comprising the amino acid sequence of SEQ ID NO:2; (c) HVR-H3 comprising the amino acid sequence of SEQ ID NO:3; (d) HVR-L1 comprising the amino acid sequence of SEQ ID NO:4; (e) HVR-L2 comprising the amino acid sequence of SEQ ID NO:5; and (f) HVR-L3 comprising the amino acid sequence of SEQ ID NO:6.

In another aspect, an antibody comprises (a) a VH domain comprising (i) HVR-H1 comprising the amino acid sequence of SEQ ID NO:1, (ii) HVR-H2 comprising the amino acid sequence of SEQ ID NO:2, and (iii) HVR-H3 comprising an amino acid sequence selected from SEQ ID NO:3; and (b) a VL domain comprising (i) HVR-L1 comprising the amino acid sequence of SEQ ID NO:4; (ii) HVR-L2 comprising the amino acid sequence of SEQ ID NO:5 and (iii) HVR-L3 comprising the amino acid sequence of SEQ ID NO:6.

Such an anti-PD1 antibody comprises

i) a VH sequence of SEQ ID NO:7 and a VL sequence of SEQ ID NO:8;

ii) or humanized variant of the VH and VL of the antibody under i).

In one embodiment such anti-PDl antibody comprises

i) a VH sequence of SEQ ID NO:57 and a VL sequence of SEQ ID NO:58; or

ii) a VH sequence of SEQ ID NO:57 and a VL sequence of SEQ ID NO:59; or

iii) a VH sequence of SEQ ID NO:57 and a VL sequence of SEQ ID NO:60; or

iv) a VH sequence of SEQ ID NO:57 and a VL sequence of SEQ ID NO:61.

In one embodiment such anti-PDl antibody comprises a VH sequence of SEQ ID NO:57 and a VL sequence of SEQ ID NO:58.

In one embodiment such anti-PDl antibody comprises a VH sequence of SEQ ID NO:57 and a VL sequence of SEQ ID NO:59.

In one embodiment such anti-PDl antibody comprises a VH sequence of SEQ ID NO:57 and a VL sequence of SEQ ID NO:60.

In one embodiment such anti-PDl antibody comprises a VH sequence of SEQ ID NO:57 and a VL sequence of SEQ ID NO:61.

In one aspect, the disclosure provides an anti-PDl antibody comprising at least one, two, three, four, five, or six HVRs selected from (a) HVR-H1 comprising the amino acid sequence of SEQ ID NO:9; (b) HVR-H2 comprising the amino acid sequence of SEQ ID NO:10; (c) HVR-H3 comprising the amino acid sequence of SEQ ID NO:11; (d) HVR-L1 comprising the amino acid sequence of SEQ ID NO:12; (e) HVR-L2 comprising the amino acid sequence of SEQ ID NO:13; and (f) HVR-L3 comprising the amino acid sequence of SEQ ID NO:14.

In one aspect, the disclosure provides an anti-PD1 antibody comprising (a) HVR-H1 comprising the amino acid sequence of SEQ ID NO:9; (b) HVR-H2 comprising the amino acid sequence of SEQ ID NO:10; (c) HVR-H3 comprising the amino acid sequence of SEQ ID NO:11; (d) HVR-L1 comprising the amino acid sequence of SEQ ID NO:12; (e) HVR-L2 comprising the amino acid sequence of SEQ ID NO:13; and (f) HVR-L3 comprising the amino acid sequence of SEQ ID NO:14.

In another aspect, an antibody comprises (a) a VH domain comprising at least one, at least two, or all three VH HVR sequences selected from (i) HVR-H1 comprising the amino acid sequence of SEQ ID NO:9, (ii) HVR-H2 comprising the amino acid sequence of SEQ ID NO:10, and (iii) HVR-H3 comprising an amino acid sequence selected from SEQ ID NO:11; and (b) a VL domain comprising at least one, at least two, or all three VL HVR sequences selected from (i) HVR-L1 comprising the amino acid sequence of SEQ ID NO:12; (ii) HVR-L2 comprising the amino acid sequence of SEQ ID NO:13 and (c) HVR-L3 comprising the amino acid sequence of SEQ ID NO:14.

In another aspect, an antibody comprises (a) a VH domain comprising (i) HVR-H1 comprising the amino acid sequence of SEQ ID NO:9, (ii) HVR-H2 comprising the amino acid sequence of SEQ ID NO:10, and (iii) HVR-H3 comprising an amino acid sequence selected from SEQ ID NO:11; and (b) a VL domain comprising (i) HVR-L1 comprising the amino acid sequence of SEQ ID NO:12; (ii) HVR-L2 comprising the amino acid sequence of SEQ ID NO:13 and (iii) HVR-L3 comprising the amino acid sequence of SEQ ID NO:14.

Such an anti-PD1 antibody comprises

i) comprises a VH sequence of SEQ ID NO:15 and a VL sequence of SEQ ID NO:16;

ii) or humanized variant of the VH and VL of the antibody under i).

In one aspect, the disclosure provides an anti-PDI antibody comprising at least one, two, three, four, five, or six HVRs selected from (a) HVR-H1 comprising the amino acid sequence of SEQ ID NO:17; (b) HVR-H2 comprising the amino acid sequence of SEQ ID NO:18; (c) HVR-H3 comprising the amino acid sequence of SEQ ID NO:19; (d) HVR-L1 comprising the amino acid sequence of SEQ ID NO:20; (e) HVR-L2 comprising the amino acid sequence of SEQ ID NO:21; and (f) HVR-L3 comprising the amino acid sequence of SEQ ID NO:22.

In one aspect, the disclosure provides an anti-PD1 antibody comprising (a) HVR-H1 comprising the amino acid sequence of SEQ ID NO: 17; (b) HVR-H2 comprising the amino acid sequence of SEQ ID NO:18; (c) HVR-H3 comprising the amino acid sequence of SEQ ID NO:19; (d) HVR-L1 comprising the amino acid sequence of SEQ ID NO:20; (e) HVR-L2 comprising the amino acid sequence of SEQ ID NO:21; and (f) HVR-L3 comprising the amino acid sequence of SEQ ID NO:22.

In another aspect, an antibody comprises (a) a VH domain comprising at least one, at least two, or all three VH HVR sequences selected from (i) HVR-H1 comprising the amino acid sequence of SEQ ID NO:17, (ii) HVR-H2 comprising the amino acid sequence of SEQ ID NO:18, and (iii) HVR-H3 comprising an amino acid sequence selected from SEQ ID NO:19; and (b) a VL domain comprising at least one, at least two, or all three VL HVR sequences selected from (i) HVR-L1 comprising the amino acid sequence of SEQ ID NO:20; (ii) HVR-L2 comprising the amino acid sequence of SEQ ID NO:21 and (c) HVR-L3 comprising the amino acid sequence of SEQ ID NO:22.

In another aspect, an antibody comprises (a) a VH domain comprising (i) HVR-H1 comprising the amino acid sequence of SEQ ID NO: 17, (ii) HVR-H2 comprising the amino acid sequence of SEQ ID NO:18, and (iii) HVR-H3 comprising an amino acid sequence selected from SEQ ID NO:19; and (b) a VL domain comprising (i) HVR-L1 comprising the amino acid sequence of SEQ ID NO:20; (ii) HVR-L2 comprising the amino acid sequence of SEQ ID NO:21 and (iii) HVR-L3 comprising the amino acid sequence of SEQ ID NO:22.

Such an anti-PD1 antibody comprises

i) comprises a VH sequence of SEQ ID NO:23 and a VL sequence of SEQ ID NO:24;

ii) or humanized variant of the VH and VL of the antibody under i).

In one aspect, the disclosure provides an anti-PDI antibody comprising at least one, two, three, four, five, or six HVRs selected from (a) HVR-H1 comprising the amino acid sequence of SEQ ID NO:25; (b) HVR-H2 comprising the amino acid sequence of SEQ ID NO:26; (c) HVR-H3 comprising the amino acid sequence of SEQ ID NO:27; (d) HVR-L1 comprising the amino acid sequence of SEQ ID NO:28; (e) HVR-L2 comprising the amino acid sequence of SEQ ID NO:29; and (f) HVR-L3 comprising the amino acid sequence of SEQ ID NO:30.

In one aspect, the disclosure provides an anti-PD1 antibody comprising (a) HVR-H1 comprising the amino acid sequence of SEQ ID NO:25; (b) HVR-H2 comprising the amino acid sequence of SEQ ID NO:26; (c) HVR-H3 comprising the amino acid sequence of SEQ ID NO:27; (d) HVR-L1 comprising the amino acid sequence of SEQ ID NO:28; (e) HVR-L2 comprising the amino acid sequence of SEQ ID NO:29; and (f) HVR-L3 comprising the amino acid sequence of SEQ ID NO:30.

In another aspect, an antibody comprises (a) a VH domain comprising at least one, at least two, or all three VH HVR sequences selected from (i) HVR-H1 comprising the amino acid sequence of SEQ ID NO:25, (ii) HVR-H2 comprising the amino acid sequence of SEQ ID NO:26, and (iii) HVR-H3 comprising an amino acid sequence selected from SEQ ID NO:27; and (b) a VL domain comprising at least one, at least two, or all three VL HVR sequences selected from (i) HVR-L1 comprising the amino acid sequence of SEQ ID NO:28; (ii) HVR-L2 comprising the amino acid sequence of SEQ ID NO:29 and (c) HVR-L3 comprising the amino acid sequence of SEQ ID NO:30.

In another aspect, an antibody comprises (a) a VH domain comprising (i) HVR-H1 comprising the amino acid sequence of SEQ ID NO:25, (ii) HVR-H2 comprising the amino acid sequence of SEQ ID NO:26, and (iii) HVR-H3 comprising an amino acid sequence selected from SEQ ID NO:27; and (b) a VL domain comprising (i) HVR-L1 comprising the amino acid sequence of SEQ ID NO:28; (ii) HVR-L2 comprising the amino acid sequence of SEQ ID NO:29 and (iii) HVR-L3 comprising the amino acid sequence of SEQ ID NO:30.

Such an anti-PD1 antibody comprises

i) comprises a VH sequence of SEQ ID NO:31 and a VL sequence of SEQ ID NO:32;

ii) or humanized variant of the VH and VL of the antibody under i).

In one aspect, the disclosure provides an anti-PDI antibody comprising at least one, two, three, four, five, or six HVRs selected from (a) HVR-H1 comprising the amino acid sequence of SEQ ID NO:33; (b) HVR-H2 comprising the amino acid sequence of SEQ ID NO:34; (c) HVR-H3 comprising the amino acid sequence of SEQ ID NO:35; (d) HVR-L1 comprising the amino acid sequence of SEQ ID NO:36; (e) HVR-L2 comprising the amino acid sequence of SEQ ID NO:37; and (f) HVR-L3 comprising the amino acid sequence of SEQ ID NO:38.

In one aspect, the disclosure provides an anti-PD1 antibody comprising (a) HVR-H1 comprising the amino acid sequence of SEQ ID NO:33; (b) HVR-H2 comprising the amino acid sequence of SEQ ID NO:34; (c) HVR-H3 comprising the amino acid sequence of SEQ ID NO:35; (d) HVR-L1 comprising the amino acid sequence of SEQ ID NO:36; (e) HVR-L2 comprising the amino acid sequence of SEQ ID NO:37; and (f) HVR-L3 comprising the amino acid sequence of SEQ ID NO:38.

In another aspect, an antibody comprises (a) a VH domain comprising at least one, at least two, or all three VH HVR sequences selected from (i) HVR-H1 comprising the amino acid sequence of SEQ ID NO:33, (ii) HVR-H2 comprising the amino acid sequence of SEQ ID NO:34, and (iii) HVR-H3 comprising an amino acid sequence selected from SEQ ID NO:35; and (b) a VL domain comprising at least one, at least two, or all three VL HVR sequences selected from (i) HVR-L1 comprising the amino acid sequence of SEQ ID NO:36; (ii) HVR-L2 comprising the amino acid sequence of SEQ ID NO:37 and (c) HVR-L3 comprising the amino acid sequence of SEQ ID NO:38.

In another aspect, an antibody comprises (a) a VH domain comprising (i) HVR-H1 comprising the amino acid sequence of SEQ ID NO:33, (ii) HVR-H2 comprising the amino acid sequence of SEQ ID NO:34, and (iii) HVR-H3 comprising an amino acid sequence selected from SEQ ID NO:35; and (b) a VL domain comprising (i) HVR-L1 comprising the amino acid sequence of SEQ ID NO:36; (ii) HVR-L2 comprising the amino acid sequence of SEQ ID NO:37 and (iii) HVR-L3 comprising the amino acid sequence of SEQ ID NO:38.

Such an anti-PD1 antibody comprises

i) comprises a VH sequence of SEQ ID NO:39 and a VL sequence of SEQ ID NO:40;

ii) or humanized variant of the VH and VL of the antibody under i).

In one aspect, the disclosure provides an anti-PDI antibody comprising at least one, two, three, four, five, or six HVRs selected from (a) HVR-H1 comprising the amino acid sequence of SEQ ID NO:41; (b) HVR-H2 comprising the amino acid sequence of SEQ ID NO:42; (c) HVR-H3 comprising the amino acid sequence of SEQ ID NO:43; (d) HVR-L1 comprising the amino acid sequence of SEQ ID NO:44; (e) HVR-L2 comprising the amino acid sequence of SEQ ID NO:45; and (f) HVR-L3 comprising the amino acid sequence of SEQ ID NO:46.

In one aspect, the disclosure provides an anti-PD1 antibody comprising (a) HVR-H1 comprising the amino acid sequence of SEQ ID NO:41; (b) HVR-H2 comprising the amino acid sequence of SEQ ID NO:42; (c) HVR-H3 comprising the amino acid sequence of SEQ ID NO:43; (d) HVR-L1 comprising the amino acid sequence of SEQ ID NO:44; (e) HVR-L2 comprising the amino acid sequence of SEQ ID NO:45; and (f) HVR-L3 comprising the amino acid sequence of SEQ ID NO:46.

In another aspect, an antibody comprises (a) a VH domain comprising at least one, at least two, or all three VH HVR sequences selected from (i) HVR-H1 comprising the amino acid sequence of SEQ ID NO:41, (ii) HVR-H2 comprising the amino acid sequence of SEQ ID NO:42, and (iii) HVR-H3 comprising an amino acid sequence selected from SEQ ID NO:43; and (b) a VL domain comprising at least one, at least two, or all three VL HVR sequences selected from (i) HVR-L1 comprising the amino acid sequence of SEQ ID NO:44; (ii) HVR-L2 comprising the amino acid sequence of SEQ ID NO:45 and (c) HVR-L3 comprising the amino acid sequence of SEQ ID NO:46.

In another aspect, an antibody comprises (a) a VH domain comprising (i) HVR-H1 comprising the amino acid sequence of SEQ ID NO:41, (ii) HVR-H2 comprising the amino acid sequence of SEQ ID NO:42, and (iii) HVR-H3 comprising an amino acid sequence selected from SEQ ID NO:43; and (b) a VL domain comprising (i) HVR-L1 comprising the amino acid sequence of SEQ ID NO:44; (ii) HVR-L2 comprising the amino acid sequence of SEQ ID NO:45 and (iii) HVR-L3 comprising the amino acid sequence of SEQ ID NO:46.

Such an anti-PD1 antibody comprises

i) comprises a VH sequence of SEQ ID NO:47 and a VL sequence of SEQ ID NO:48;

ii) or humanized variant of the VH and VL of the antibody under i).

In one aspect, the disclosure provides an anti-PDl antibody comprising at least one, two, three, four, five, or six HVRs selected from (a) HVR-H1 comprising the amino acid sequence of SEQ ID NO:49; (b) HVR-H2 comprising the amino acid sequence of SEQ ID NO:50; (c) HVR-H3 comprising the amino acid sequence of SEQ ID NO:51; (d) HVR-L1 comprising the amino acid sequence of SEQ ID NO:52; (e) HVR-L2 comprising the amino acid sequence of SEQ ID NO:53; and (f) HVR-L3 comprising the amino acid sequence of SEQ ID NO:54.

In one aspect, the disclosure provides an anti-PDl comprising (a) HVR-H1 comprising the amino acid sequence of SEQ ID NO:49; (b) HVR-H2 comprising the amino acid sequence of SEQ ID NO:50; (c) HVR-H3 comprising the amino acid sequence of SEQ ID NO:51; (d) HVR-L1 comprising the amino acid sequence of SEQ ID NO:52; (e) HVR-L2 comprising the amino acid sequence of SEQ ID NO:53; and (f) HVR-L3 comprising the amino acid sequence of SEQ ID NO:54.

In another aspect, an antibody comprises (a) a VH domain comprising at least one, at least two, or all three VH HVR sequences selected from (i) HVR-H1 comprising the amino acid sequence of SEQ ID NO:49, (ii) HVR-H2 comprising the amino acid sequence of SEQ ID NO:50, and (iii) HVR-H3 comprising an amino acid sequence selected from SEQ ID NO:51; and (b) a VL domain comprising at least one, at least two, or all three VL HVR sequences selected from (i) HVR-L1 comprising the amino acid sequence of SEQ ID NO:52; (ii) HVR-L2 comprising the amino acid sequence of SEQ ID NO:53 and (c) HVR-L3 comprising the amino acid sequence of SEQ ID NO:54.

In another aspect, an antibody comprises (a) a VH domain comprising (i) HVR-H1 comprising the amino acid sequence of SEQ ID NO:49, (ii) HVR-H2 comprising the amino acid sequence of SEQ ID NO:50, and (iii) HVR-H3 comprising an amino acid sequence selected from SEQ ID NO:51; and (b) a VL domain comprising (i) HVR-L1 comprising the amino acid sequence of SEQ ID NO:52; (ii) HVR-L2 comprising the amino acid sequence of SEQ ID NO:53 and (iii) HVR-L3 comprising the amino acid sequence of SEQ ID NO:54.

Such an anti-PD1 antibody comprises

i) comprises a VH sequence of SEQ ID NO:47 and a VL sequence of SEQ ID NO:48;

ii) or humanized variant of the VH and VL of the antibody under i).

In one aspect, the disclosure provides an anti-PDl antibody (e.g. an antibody that binds to human PD1) comprising

A) (a) HVR-H1 comprising the amino acid sequence of SEQ ID NO: 1; (b) HVR-H2 comprising the amino acid sequence of SEQ ID NO:2; (c) HVR-H3 comprising the amino acid sequence of SEQ ID NO:3; (d) HVR-L1 comprising the amino acid sequence of SEQ ID NO:4; (e) HVR-L2 comprising the amino acid sequence of SEQ ID NO:5; and (f) HVR-L3 comprising the amino acid sequence of SEQ ID NO:6; or

In another aspect the disclosure provides an anti-PD1 antibody (e.g. an antibody that binds to human PD1) comprising

(a) a VH domain comprising (i) HVR-H1 comprising the amino acid sequence of SEQ ID NO: 1, (ii) HVR-H2 comprising the amino acid sequence of SEQ ID NO:2, and (iii) HVR-H3 comprising an amino acid sequence selected from SEQ ID NO:3; and (b) a VL domain comprising (i)HVR-L1 comprising the amino acid sequence of SEQ ID NO:4; (ii) HVR-L2 comprising the amino acid sequence of SEQ ID NO:5 and (iii) HVR-L3 comprising the amino acid sequence of SEQ ID NO:6.

In one aspect, the 2. disclosure provides an antibody that binds to human PD1 that

A)

i) comprises a VH sequence of SEQ ID NO:7 and a VL sequence of SEQ ID NO:8;
ii) or humanized variant of the VH and VL of the antibody under i);

or B)

i) comprises a VH sequence of SEQ ID NO:57 and a VL sequence of SEQ ID NO:58.

ii) comprises a VH sequence of SEQ ID NO:57 and a VL sequence of SEQ ID NO:59.
iii) comprises a VH sequence of SEQ ID NO:57 and a VL sequence of SEQ ID NO:60.
iv) comprises a VH sequence of SEQ ID NO:57 and a VL sequence of SEQ ID NO:61.

or C)

i) comprises a VH sequence of SEQ ID NO:15 and a VL sequence of SEQ ID NO: 16;
ii) or humanized variant of the VH and VL of the antibody under i);

or D)

i) comprises a VH sequence of SEQ ID NO:23 and a VL sequence of SEQ ID NO:24;
ii) or humanized variant of the VH and VL of the antibody under i);.

or E)

i) comprises a VH sequence of SEQ ID NO:31 and a VL sequence of SEQ ID NO:32;
ii) or humanized variant of the VH and VL of the antibody under i);

or F)

i) comprises a VH sequence of SEQ ID NO:39 and a VL sequence of SEQ ID NO:40;
ii) or humanized variant of the VH and VL of the antibody under i);

or G)

i) comprises a VH sequence of SEQ ID NO:47 and a VL sequence of SEQ ID NO:48;
ii) or humanized variant of the VH and VL of the antibody under i);

or H)

i) comprises a VH sequence of SEQ ID NO:55 and a VL sequence of SEQ ID NO:56;
ii) or humanized variant of the VH and VL of the antibody under i).

In one aspect, the disclosure provides an antibody that binds to human PD1 that

i) comprises a VH sequence of SEQ ID NO:7 and a VL sequence of SEQ ID NO:8;
ii) or humanized variant of the VH and VL of the antibody under i);

In one aspect, the invention provides an antibody that binds to human PD1 that comprises a VH sequence of SEQ ID NO:57 and a VL sequence of SEQ ID NO:58. In one aspect, the invention provides an antibody that binds to human PD1 that comprises a VH sequence of SEQ ID NO:57 and a VL sequence of SEQ ID NO:59. In one aspect, the invention provides an antibody that binds to human PD1 that comprises a VH sequence of SEQ ID NO:57 and a VL sequence of SEQ ID NO:60. In one aspect, the invention provides an antibody that binds to human PD1 that comprises a VH sequence of SEQ ID NO:57 and a VL sequence of SEQ ID NO:61.

In another aspect the 2. disclosure provides an anti-PD1 antibody (e.g. an antibody that binds to human PD1) comprising

A) (a) HVR-H1 comprising the amino acid sequence of SEQ ID NO: 1; (b) HVR-H2 comprising the amino acid sequence of SEQ ID NO:2; (c) HVR-H3 comprising the amino acid sequence of SEQ ID NO:3; (d) HVR-L1 comprising the amino acid sequence of SEQ ID NO:4; (e) HVR-L2 comprising the amino acid sequence of SEQ ID NO:5; and (f) HVR-L3 comprising the amino acid sequence of SEQ ID NO:6; or

B) (a) HVR-H1 comprising the amino acid sequence of SEQ ID NO:9; (b) HVR-H2 comprising the amino acid sequence of SEQ ID NO:10; (c) HVR-H3 comprising the amino acid sequence of SEQ ID NO:11; (d) HVR-L1 comprising the amino acid sequence of SEQ ID NO:12; (e) HVR-L2 comprising the amino acid sequence of SEQ ID NO:13; and (f) HVR-L3 comprising the amino acid sequence of SEQ ID NO:14; or

C) (a) HVR-H1 comprising the amino acid sequence of SEQ ID NO:17; (b) HVR-H2 comprising the amino acid sequence of SEQ ID NO:18; (c) HVR-H3 comprising the amino acid sequence of SEQ ID NO:19; (d) HVR-L1 comprising the amino acid sequence of SEQ ID NO:20; (e) HVR-L2 comprising the amino acid sequence of SEQ ID NO:21; and (f) HVR-L3 comprising the amino acid sequence of SEQ ID NO:22; or

D) (a) HVR-H1 comprising the amino acid sequence of SEQ ID NO:25; (b) HVR-H2 comprising the amino acid sequence of SEQ ID NO:26; (c) HVR-H3 comprising the amino acid sequence of SEQ ID NO:27; (d) HVR-L1 comprising the amino acid sequence of SEQ ID NO:28; (e) HVR-L2 comprising the amino acid sequence of SEQ ID NO:29; and (f) HVR-L3 comprising the amino acid sequence of SEQ ID NO:30; or

E) (a) HVR-H1 comprising the amino acid sequence of SEQ ID NO:33; (b) HVR-H2 comprising the amino acid sequence of SEQ ID NO:34; (c) HVR-H3 comprising the amino acid sequence of SEQ ID NO:35; (d) HVR-L1 comprising the amino acid sequence of SEQ ID NO:36; (e) HVR-L2 comprising the amino acid sequence of SEQ ID NO:37; and (f) HVR-L3 comprising the amino acid sequence of SEQ ID NO:38; or

F) (a) HVR-H1 comprising the amino acid sequence of SEQ ID NO:41; (b) HVR-H2 comprising the amino acid sequence of SEQ ID NO:42; (c) HVR-H3 comprising the amino acid sequence of SEQ ID NO:43; (d) HVR-L1 comprising the amino acid sequence of SEQ ID NO:44; (e) HVR-L2 comprising the amino acid sequence of SEQ ID NO:45; and (f) HVR-L3 comprising the amino acid sequence of SEQ ID NO:46; or

G) (a) HVR-H1 comprising the amino acid sequence of SEQ ID NO:49; (b) HVR-H2 comprising the amino acid sequence of SEQ ID NO:50; (c) HVR-H3 comprising the amino acid sequence of SEQ ID NO:51; (d) HVR-L1 comprising the amino acid sequence of SEQ ID NO:52; (e) HVR-L2 comprising the amino acid sequence of SEQ ID NO:53; and (f) HVR-L3 comprising the amino acid sequence of SEQ ID NO:54;

wherein the antibody is characterized independently by one or more of the following properties: the anti-PD-1 antibody

i) competes for binding to PD-1 with an anti-PD-1 antibody comprising the VH and VL of PD1-0103, and/ or
ii) binds to a human and cynomolguoes PD-1; and/ or
iii) enhances the interferon-gamma (IFN-gamma) secretion by allogenic stimulated T cells by 85% or more (in one preferred embodiment by 90% or more, in one preferred embodiment by 95% or more) at an antibody concentration of 10μg/ml (wherein the secretion without antibody is set as 0% (basal level of IFN gamma) and the secretion with 20 EU/ ml recombinant human IL-2 is set as 100% (in a (allogenic) Mixed lymphocyte reaction (MLR) assay according to Example 3) ; and/ or
iv) enhances the tumor necrosis factor alpha (TNF alpha) secretion by allogenic stimulated T cells by 200% or more (in one preferred embodiment by 250% or more) at an antibody concentration of 10μg/ml (wherein the secretion without antibody is set as 0% (basal level of IFN gamma) and the secretion with 20 EU/ ml recombinant human IL-2 is is set as 100% (in a (allogenic) Mixed lymphocyte reaction (MLR) assay according to Example 3).

In another aspect the disclosure provides an anti-PD1 antibody (e.g. an antibody that binds to human PD1) comprising

A) (a) a VH domain comprising (i) HVR-H1 comprising the amino acid sequence of SEQ ID NO:1, (ii) HVR-H2 comprising the amino acid sequence of SEQ ID NO:2, and (iii) HVR-H3 comprising an amino acid sequence selected from SEQ ID NO:3; and (b) a VL domain comprising (i) HVR-L1 comprising the amino acid sequence of SEQ ID NO:4; (ii) HVR-L2 comprising the amino acid sequence of SEQ ID NO:5 and (iii) HVR-L3 comprising the amino acid sequence of SEQ ID NO:6; or

B) (a) a VH domain comprising (i) HVR-H1 comprising the amino acid sequence of SEQ ID NO:9, (ii) HVR-H2 comprising the amino acid sequence of SEQ ID NO:10, and (iii) HVR-H3 comprising an amino acid sequence selected from SEQ ID NO:11; and (b) a VL domain comprising (i) HVR-L1 comprising the amino acid sequence of SEQ ID NO:12; (ii) HVR-L2 comprising the amino acid sequence of SEQ ID NO:13 and (iii) HVR-L3 comprising the amino acid sequence of SEQ ID NO:14; or

C) (a) a VH domain comprising (i) HVR-H1 comprising the amino acid sequence of SEQ ID NO:17, (ii) HVR-H2 comprising the amino acid sequence of SEQ ID NO:18, and (iii) HVR-H3 comprising an amino acid sequence selected from SEQ ID NO:19; and (b) a VL domain comprising (i) HVR-L1 comprising the amino acid sequence of SEQ ID NO:20; (ii) HVR-L2 comprising the amino acid sequence of SEQ ID NO:21 and (iii) HVR-L3 comprising the amino acid sequence of SEQ ID NO:22; or.

D) (a) a VH domain comprising (i) HVR-H1 comprising the amino acid sequence of SEQ ID NO:25, (ii) HVR-H2 comprising the amino acid sequence of SEQ ID NO:26, and (iii) HVR-H3 comprising an amino acid sequence selected from SEQ ID NO:27; and (b) a VL domain comprising (i) HVR-L1 comprising the amino acid sequence of SEQ ID NO:28; (ii) HVR-L2 comprising the amino acid sequence of SEQ ID NO:29 and (iii) HVR-L3 comprising the amino acid sequence of SEQ ID NO:30; or

E) (a) a VH domain comprising (i) HVR-H1 comprising the amino acid sequence of SEQ ID NO:33, (ii) HVR-H2 comprising the amino acid sequence of SEQ ID NO:34, and (iii) HVR-H3 comprising an amino acid sequence selected from SEQ ID NO:35; and (b) a VL domain comprising (i) HVR-L1 comprising the amino acid sequence of SEQ ID NO:36; (ii) HVR-L2 comprising the amino acid sequence of SEQ ID NO:37 and (iii) HVR-L3 comprising the amino acid sequence of SEQ ID NO:38; or

F) (a) a VH domain comprising (i) HVR-H1 comprising the amino acid sequence of SEQ ID NO:41, (ii) HVR-H2 comprising the amino acid sequence of SEQ ID NO:42, and (iii) HVR-H3 comprising an amino acid sequence selected from SEQ ID NO:43; and (b) a VL domain comprising (i) HVR-L1 comprising the amino acid sequence of SEQ ID NO:44; (ii) HVR-L2 comprising the amino acid sequence of SEQ ID NO:45 and (iii) HVR-L3 comprising the amino acid sequence of SEQ ID NO:46; or

G) (a) a VH domain comprising (i) HVR-H1 comprising the amino acid sequence of SEQ ID NO:49, (ii) HVR-H2 comprising the amino acid sequence of SEQ ID NO:50, and (iii) HVR-H3 comprising an amino acid sequence selected from SEQ ID NO:51; and (b) a VL domain comprising (i) HVR-L1 comprising the amino acid sequence of SEQ ID NO:52; (ii) HVR-L2 comprising the amino acid sequence of SEQ ID NO:53 and (iii) HVR-L3 comprising the amino acid sequence of SEQ ID NO:54;

wherein the antibody is characterized indenpendently by one or more of the following properties: the anti-PD-1 antibody

i) competes for binding to PD-1 with an anti-PD-1 antibody comprising the VH and VL of PD1-0103, and/ or
ii) binds to a human and cynomolguoes PD-1; and/ or
iii) enhances the interferon-gamma (IFN-gamma) secretion by allogenic stimulated T cells by 85% or more (in one preferred embodiment by 90% or more, in one preferred embodiment by 95% or more) at an antibody concentration of 10μg/ml (wherein the secretion without antibody is set as 0% (basal level of IFN gamma) and the secretion with 20 EU/ ml recombinant human IL-2 is set as 100% (in a (allogenic) Mixed lymphocyte reaction (MLR) assay according to Example 3) ; and/ or
iv) enhances the tumor necrosis factor alpha (TNF alpha) secretion by allogenic stimulated T cells by 200% or more (in one preferred embodiment by 250% or more) at an antibody concentration of 10μg/ml (wherein the secretion without antibody is set as 0% (basal level of IFN gamma) and the secretion with 20 EU/ ml recombinant human IL-2 is is set as 100% (in a (allogenic) Mixed lymphocyte reaction (MLR) assay according to Example 3).

In a further aspect of the invention, an anti-PDl antibody according to any of the above embodiments is a monoclonal antibody, including a chimeric, humanized or human antibody. In one embodiment, an anti-PD1 antibody is an antibody fragment, e.g., a Fv, Fab, Fab', scFv, diabody, or $F(ab')_2$ fragment. In another embodiment, the antibody is a full length antibody, e.g., an intact IgG1 or IgG4 antibody or other antibody class or isotype as defined herein.

In a further aspect, an anti-PDl antibody according to disclosure may incorporate any of the features, singly or in combination, as described in Sections 1-7 below:

**1. Antibody Affinity**

In certain aspects, an antibody provided herein has a dissociation constant KD of ≤ 1 μM, ≤ 100 nM, ≤ 10 nM, ≤ 1 nM, ≤ 0.1 nM, ≤ 0.01 nM, or ≤ 0.001 nM (e.g. $10^{-8}$ M or less, e.g. from $10^{-8}$ M to $10^{-13}$ M, e.g., from $10^{-9}$ M to $10^{-13}$ M).

In one aspect, KD is measured using surface plasmon resonance assays using a BIACORE®) at 25°C with immobilized antigen CM5 chips at ~10 response units (RU). Briefly, carboxymethylated dextran biosensor chips (CM5, BIACORE, Inc.) are activated with *N*-ethyl-*N*'- (3-dimethylaminopropyl)-carbodiimide hydrochloride (EDC) and *N*-hydroxysuccinimide (NHS) according to the supplier's instructions. Antigen is diluted with 10 mM sodium acetate, pH 4.8, to 5 μg/ml (~0.2 μM) before injection at a flow rate of 5 ul/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20™) surfactant (PBST) at 25°C at a flow rate of approximately 25 μl/min. Association rates ($k_{on}$ or ka) and dissociation rates ($k_{off}$ or kd) are calculated using a simple one-to-one Langmuir binding model (BIACORE ® Evaluation Software version 3.2) by simultaneously fitting the association and dissociation sensorgrams. The equilibrium dissociation constant KD is calculated as the ratio kd/ka ($k_{off}/k_{on}$.) See, e.g., Chen, Y. et al., J. Mol. Biol. 293 (1999) 865-881. If the on-rate exceeds $10^6$ $M^{-1}$ $s^{-1}$ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25°C of a 20 nM anti-antigen antibody (Fab form) in PBS, pH 7.2, in the presence of increasing concentrations of antigen as measured in a spectrometer, such as a stop-flow equipped spectrophotometer (Aviv Instruments) or a 8000-series SLM-AMINCO™ spectrophotometer (ThermoSpectronic) with a stirred cuvette.

**2. Antibody Fragments**

In certain aspects, an antibody provided herein is an antibody fragment. Antibody fragments include, but are not limited to, Fab, Fab', Fab'-SH, $F(ab')_2$, Fv, and scFv fragments, and other fragments described below. For a review of certain antibody fragments, see Hudson, P.J. et al., Nat. Med. 9 (2003) 129-134. For a review of scFv fragments, see, e.g., Plueckthun, A., In; The Pharmacology of Monoclonal Antibodies, Vol. 113, Rosenburg and Moore (eds.), Springer-Verlag, New York (1994), pp. 269-315; see also WO 93/16185; and U.S. Patent Nos. 5,571,894 and 5,587,458. For discussion of Fab and $F(ab')_2$ fragments comprising salvage receptor binding epitope residues and having increased in vivo half-life, see U.S. Patent No. 5,869,046.

Diabodies are antibody fragments with two antigen-binding sites that may be bivalent or bispecific. See, for example, EP 0 404 097; WO 1993/01161; Hudson, P.J. et al., Nat. Med. 9 (2003) 129-134; and Holliger, P. et al., Proc. Natl. Acad. Sci. USA 90 (1993) 6444-6448. Triabodies and tetrabodies are also described in Hudson, P.J. et al., Nat. Med. 9 (20039 129-134).

Single-domain antibodies are antibody fragments comprising all or a portion of the heavy chain variable domain or all or a portion of the light chain variable domain of an antibody. In certain aspects, a single-domain antibody is a human single-domain antibody (Domantis, Inc., Waltham, MA; *see,* e.g., U.S. Patent No. 6,248,516 B1).

Antibody fragments can be made by various techniques, including but not limited to proteolytic digestion of an intact antibody as well as production by recombinant host cells (e.g. *E. coli* or phage), as described herein.

**3. Chimeric and Humanized Antibodies**

In certain aspects, an antibody provided herein is a chimeric antibody. Certain chimeric antibodies are described, e.g., in U.S. Patent No. 4,816,567; and Morrison, S.L. et al., Proc. Natl. Acad. Sci. USA 81 (1984) 6851-6855). In one example, a chimeric antibody comprises a non-human variable region (e.g., a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain aspects, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody (e.g., the antibody from which the HVR residues are derived), e.g., to restore or improve antibody specificity or affinity.

Humanized antibodies and methods of making them are reviewed, e.g., in Almagro, J.C. and Fransson, J., Front. Biosci. 13 (2008) 1619-1633, and are further described, e.g., in Riechmann, I. et al., Nature 332 (1988) 323-329; Queen, C. et al., Proc. Natl. Acad. Sci. USA 86 (1989) 10029-10033; US Patent Nos. 5, 821,337, 7,527,791, 6,982,321, and 7,087,409; Kashmiri, S.V. et al., Methods 36 (2005) 25-34 (describing SDR (a-CDR) grafting); Padlan, E.A., Mol. Immunol. 28 (1991) 489-498 (describing "resurfacing"); Dall'Acqua, W.F. et al., Methods 36 (2005) 43-60 (describing "FR shuffling"); and Osbourn, J. et al., Methods 36 (2005) 61-68 and Klimka, A. et al., Br. J. Cancer 83 (2000) 252-260 (describing the "guided selection" approach to FR shuffling).

Human framework regions that may be used for humanization include but are not limited to: framework regions selected using the "best-fit" method (see, e.g., Sims, M.J. et al., J. Immunol. 151 (1993) 2296-2308; framework regions derived from the consensus sequence of human antibodies of a particular subgroup of light or heavy chain variable regions (see, e.g., Carter, P. et al., Proc. Natl. Acad. Sci. USA 89 (1992) 4285-4289; and Presta, L.G. et al., J. Immunol. 151 (1993) 2623-2632); human mature (somatically mutated) framework regions or human germline framework regions (see, e.g., Almagro, J.C. and Fransson, J., Front. Biosci. 13 (2008) 1619-1633); and framework regions derived from screening FR libraries (see, e.g., Baca, M. et al., J. Biol. Chem. 272 (1997) 10678-10684 and Rosok, M.J. et al., J. Biol. Chem. 271 (19969 22611-22618).

### 4. Human Antibodies

In certain aspects, an antibody provided herein is a human antibody. Human antibodies can be produced using various techniques known in the art. Human antibodies are described generally in van Dijk, M.A. and van de Winkel, J.G., Curr. Opin. Pharmacol. 5 (2001) 368-374 and Lonberg, N., Curr. Opin. Immunol. 20 (2008) 450-459.

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, N., Nat. Biotech. 23 (2005) 1117-1125. See also, e.g., U.S. Patent Nos. 6,075,181 and 6,150,584 describing XENOMOUSF™ technology; U.S. Patent No. 5,770,429 describing HUMAB® technology; U.S. Patent No. 7,041,870 describing K-M MOUSE® technology, and U.S. Patent Application Publication No. US 2007/0061900, describing VELOCIMOUSE® technology). Human variable regions from intact antibodies generated by such animals may be further modified, e.g., by combining with a different human constant region.

Human antibodies can also be made by hybridoma-based methods. Human myeloma and mouse-human heteromyeloma cell lines for the production of human monoclonal antibodies have been described. (See, e.g., Kozbor, D., J. Immunol. 133 (1984) 3001-3005; Brodeur, B.R. et al., Monoclonal Antibody Production Techniques and Applications, Marcel Dekker, Inc., New York (1987), pp. 51-63; and Boerner, P. et al., J. Immunol. 147 (1991) 86-95) Human antibodies generated via human B-cell hybridoma technology are also described in Li, J. et al., Proc. Natl. Acad. Sci. USA 103 (2006) 3557-3562. Additional methods include those described, for example, in U.S. Patent No. 7,189,826 (describing production of monoclonal human IgM antibodies from hybridoma cell lines) and Ni, J., Xiandai Mianyixue 26 (2006) 265-268 (describing human-human hybridomas). Human hybridoma technology (Trioma technology) is also described in Vollmers, H.P. and Brandlein, S., Histology and Histopathology 20 (2005) 927-937 and Vollmers, H.P. and Brandlein, S., Methods and Findings in Experimental and Clinical Pharmacology 27 (2005) 185-191. Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

### 5. Library-Derived Antibodies

Antibodies may be isolated by screening combinatorial libraries for antibodies with the desired activity or activities. For example, a variety of methods are known in the art for generating phage display libraries and screening such libraries for antibodies possessing the desired binding characteristics. Such methods are reviewed, e.g., in Hoogenboom, H.R. et al., Methods in Molecular Biology 178 (2001) 1-37 and further described, e.g., in the McCafferty, J. et al., Nature 348 (1990) 552-554; Clackson, T. et al., Nature 352 (1991) 624-628; Marks, J.D. et al., J. Mol. Biol. 222 (1992) 581-597; Marks, J.D. and Bradbury, A., Methods in Molecular Biology 248 (2003) 161-175; Sidhu, S.S. et al., J. Mol. Biol. 338 (2004) 299-310; Lee, C.V. et al., J. Mol. Biol. 340 (2004) 1073-1093; Fellouse, F.A., Proc. Natl. Acad. Sci. USA 101 (2004) 12467-12472; and Lee, C.V. et al., J. Immunol. Methods 284 (2004) 119-132.

In certain phage display methods, repertoires of VH and VL genes are separately cloned by polymerase chain reaction (PCR) and recombined randomly in phage libraries, which can then be screened for antigen-binding phage as described in Winter, G. et al., Ann. Rev. Immunol. 12 (1994) 433-455. Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned (e.g., from human) to provide a single source of antibodies to a wide range of non-self and also self antigens without any immunization as described by Griffiths, A.D. et al., EMBO J. 12 (1993) 725-734. Finally, naive libraries can also be made synthetically by cloning non-rearranged V-gene segments from stem cells, and using PCR primers containing random sequence to encode the highly variable CDR3 regions and to accomplish rearrangement *in vitro,* as described by Hoogenboom, H.R. and Winter, G., J. Mol. Biol. 227 (1992) 381-388. Patent publications describing human antibody phage libraries include, for example: US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

Antibodies or antibody fragments isolated from human antibody libraries are considered human antibodies or human antibody fragments herein.

## 6. Multispecific Antibodies

In certain aspects, an antibody provided herein is a multispecific antibody, e.g. a bispecific antibody. Multispecific antibodies are monoclonal antibodies that have binding specificities for at least two different sites. In certain aspects, one of the binding specificities is for PD1 and the other is for any other antigen. In certain embodiments, bispecific antibodies may bind to two different epitopes of PD1. Bispecific antibodies may also be used to localize cytotoxic agents to cells which express PD1. Bispecific antibodies can be prepared as full length antibodies or antibody fragments.

Techniques for making multispecific antibodies include, but are not limited to, recombinant co-expression of two immunoglobulin heavy chain-light chain pairs having different specificities (see Milstein, C. and Cuello, A.C., Nature 305 (1983) 537-540, WO 93/08829, and Traunecker, A. et al., EMBO J. 10 (1991) 3655-3659), and "knob-in-hole" engineering (see, e.g., U.S. Patent No. 5,731,168). Multi-specific antibodies may also be made by engineering electrostatic steering effects for making antibody Fc-heterodimeric molecules (WO 2009/089004); cross-linking two or more antibodies or fragments (see, e.g., US Patent No. 4,676,980, and Brennan, M. et al., Science 229 (1985) 81-83); using leucine zippers to produce bi-specific antibodies (see, e.g., Kostelny, S.A. et al., J. Immunol. 148 (1992) 1547-1553; using "diabody" technology for making bispecific antibody fragments (see, e.g., Holliger, P. et al., Proc. Natl. Acad. Sci. USA 90 (1993) 6444-6448); and using single-chain Fv (sFv) dimers (see, e.g. Gruber, M et al., J. Immunol. 152 (1994) 5368-5374); and preparing trispecific antibodies as described, e.g., in Tutt, A. et al., J. Immunol. 147 (1991) 60-69).

Engineered antibodies with three or more functional antigen binding sites, including "Octopus antibodies," are also included herein (see, e.g. US 2006/0025576).

The antibody or fragment herein also includes a "Dual Acting Fab" or "DAF" comprising an antigen binding site that binds to PD1 as well as another, different antigen (see, US 2008/0069820, for example).

The antibody or fragment herein also includes multispecific antibodies described in WO 2009/080251, WO 2009/080252, WO 2009/080253, WO 2009/080254, WO 2010/112193, WO 2010/115589, WO 2010/136172, WO 2010/145792, and WO 2010/145793, WO2011/117330, WO2012/025525, WO2012/025530, WO2013/026835, WO2013/026831, WO2013/164325, or WO 2013/174873.

## 7. Antibody Variants

In certain aspects, amino acid sequence variants of the antibodies provided herein are contemplated. For example, it may be desirable to improve the binding affinity and/or other biological properties of the antibody. Amino acid sequence variants of an antibody may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, e.g., antigen-binding.

### a) Substitution, Insertion, and Deletion Variants

In certain aspects, antibody variants having one or more amino acid substitutions are provided. Sites of interest for substitutional mutagenesis include the HVRs and FRs. Exemplary changes are provided in Table 1 under the heading of "exemplary substitutions", and as further described below in reference to amino acid side chain classes. Conservative substitutions are shown in Table 1 under the heading of "preferred substitutions". Amino acid substitutions may be introduced into an antibody of interest and the products screened for a desired activity, e.g., retained/improved antigen binding, decreased immunogenicity, or improved ADCC or CDC.

**Table 1**

| Original Residue | Exemplary Substitutions | Preferred Substitutions |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |

(continued)

| Original Residue | Exemplary Substitutions | Preferred Substitutions |
|---|---|---|
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:

(1) hydrophobic: Norleucine, Met, Ala, Val, Leu, Ile;

(2) neutral hydrophilic: Cys, Ser, Thr, Asn, Gln;

(3) acidic: Asp, Glu;

(4) basic: His, Lys, Arg;

(5) residues that influence chain orientation: Gly, Pro;

(6) aromatic: Trp, Tyr, Phe.

Non-conservative substitutions will entail exchanging a member of one of these classes for another class.

One type of substitutional variant involves substituting one or more hypervariable region residues of a parent antibody (e.g. a humanized or human antibody). Generally, the resulting variant(s) selected for further study will have modifications (e.g., improvements) in certain biological properties (e.g., increased affinity, reduced immunogenicity) relative to the parent antibody and/or will have substantially retained certain biological properties of the parent antibody. An exemplary substitutional variant is an affinity matured antibody, which may be conveniently generated, e.g., using phage display-based affinity maturation techniques such as those described herein. Briefly, one or more HVR residues are mutated and the variant antibodies displayed on phage and screened for a particular biological activity (e.g. binding affinity).

Alterations (e.g., substitutions) may be made in HVRs, e.g., to improve antibody affinity. Such alterations may be made in HVR "hotspots," i.e., residues encoded by codons that undergo mutation at high frequency during the somatic maturation process (see, e.g., Chowdhury, P.S., Methods Mol. Biol. 207 (2008) 179-196), and/or SDRs (a-CDRs), with the resulting variant VH or VL being tested for binding affinity. Affinity maturation by constructing and reselecting from secondary libraries has been described, e.g., in Hoogenboom, H.R. et al. in Methods in Molecular Biology 178 (2002) 1-37. In some examples of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (e.g., 4-6 residues at a time) are randomized. HVR residues involved in antigen binding may be specifically identified, e.g., using alanine scanning mutagenesis or modeling. CDR-H3 and CDR-L3 in particular are often targeted.

In certain aspects, substitutions, insertions, or deletions may occur within one or more HVRs so long as such alterations do not substantially reduce the ability of the antibody to bind antigen. For example, conservative alterations (e.g., conservative substitutions as provided herein) that do not substantially reduce binding affinity may be made in HVRs. Such alterations may be outside of HVR "hotspots" or SDRs. In certain examples of the variant VH and VL sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham, B.C. and Wells, J.A., Science 244 (1989) 1081-1085. In this method, a residue or group of target residues (e.g., charged residues such as arg, asp, his, lys, and glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex to identify contact points between the antibody and antigen. Such contact residues and neighboring residues may be targeted or eliminated as candidates for substitution. Variants may be screened to determine whether they contain the desired properties. Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme (e.g. for ADEPT) or a polypeptide which increases the serum half-life of the antibody.

**b) Fc region variants**

In certain aspects, one or more amino acid modifications may be introduced into the Fc region of an antibody provided herein, thereby generating an Fc region variant. The Fc region variant may comprise a human Fc region sequence (*e.g.,* a human IgG1, IgG2, IgG3 or IgG4 Fc region) comprising an amino acid modification (*e.g.* a substitution) at one or more amino acid positions.

Antibodies with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Certain antibody variants with improved or diminished binding to FcRs are described. (See, e.g., U.S. Patent No. 6,737,056; WO 2004/056312, and Shields, R.L. et al., J. Biol. Chem. 276 (2001) 6591-6604).

In one aspect, such antibody is a IgG1 with mutations L234A and L235A or with mutations L234A, L235A and P329G. In another embodiment or IgG4 with mutations S228P and L235E or S228P, L235E or and P329G (numbering according to EU index of Kabat et al , Kabat et al., Sequences of Proteins of Immunological Interest, 5th Ed. Public Health Service, National Institutes of Health, Bethesda, MD, 1991).

Antibodies with increased half lives and improved binding to the neonatal Fc receptor (FcRn), which is responsible for the transfer of maternal IgGs to the fetus (Guyer, R.L. et al., J. Immunol. 117 (1976) 587-593, and Kim, J.K. et al., J. Immunol. 24 (1994) 2429-2434), are described in US 2005/0014934. Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, e.g., substitution of Fc region residue 434 (US Patent No. 7,371,826).

See also Duncan, A.R. and Winter, G., Nature 322 (1988) 738-740; US 5,648,260; US 5,624,821; and WO 94/29351 concerning other examples of Fc region variants.

**c) Cysteine engineered antibody variants**

In certain aspects, it may be desirable to create cysteine engineered antibodies, e.g., "thioMAbs," in which one or more residues of an antibody are substituted with cysteine residues. In particular aspects, the substituted residues occur at accessible sites of the antibody. By substituting those residues with cysteine, reactive thiol groups are thereby positioned at accessible sites of the antibody and may be used to conjugate the antibody to other moieties, such as drug moieties or linkerdrug moieties, to create an immunoconjugate, as described further herein. In certain aspects, any one or more of the following residues may be substituted with cysteine: V205 (Kabat numbering) of the light chain; A118 (EU numbering) of the heavy chain; and S400 (EU numbering) of the heavy chain Fc region. Cysteine engineered antibodies may be generated as described, e.g., in U.S. Patent No. 7,521,541.

### d) Antibody Derivatives

In certain aspects, an antibody provided herein may be further modified to contain additional non-proteinaceous moieties that are known in the art and readily available. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide copolymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer is attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another aspect, conjugates of an antibody and non-proteinaceous moiety that may be selectively heated by exposure to radiation are provided. In one embodiment, the non-proteinaceous moiety is a carbon nanotube (Kam, N.W. et al., Proc. Natl. Acad. Sci. USA 102 (2005) 11600-11605). The radiation may be of any wavelength, and includes, but is not limited to, wavelengths that do not harm ordinary cells, but which heat the non-proteinaceous moiety to a temperature at which cells proximal to the antibody-non-proteinaceous moiety are killed.

### B. Recombinant Methods and Compositions

Antibodies may be produced using recombinant methods and compositions, e.g., as described in U.S. Patent No. 4,816,567. In one aspect, isolated nucleic acid encoding an anti-PDI antibody described herein is provided. Such nucleic acid may encode an amino acid sequence comprising the VL and/or an amino acid sequence comprising the VH of the antibody (e.g., the light and/or heavy chains of the antibody). In a further aspect, one or more vectors (e.g., expression vectors) comprising such nucleic acid are provided. In a further aspect, a host cell comprising such nucleic acid is provided. In one such aspect, a host cell comprises (e.g., has been transformed with): (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody. In one aspect, the host cell is eukaryotic, e.g. a Chinese Hamster Ovary (CHO) cell or lymphoid cell (e.g., Y0, NS0, Sp20 cell). In one embodiment, a method of making an anti-PDI antibody is provided, wherein the method comprises culturing a host cell comprising a nucleic acid encoding the antibody, as provided above, under conditions suitable for expression of the antibody, and optionally recovering the antibody from the host cell (or host cell culture medium).

For recombinant production of an anti-PDI antibody, nucleic acid encoding an antibody, e.g., as described above, is isolated and inserted into one or more vectors for further cloning and/or expression in a host cell. Such nucleic acid may be readily isolated and sequenced using conventional procedures (e.g., by using oligonucleotide probes that are capable of binding specifically to genes encoding the heavy and light chains of the antibody).

Suitable host cells for cloning or expression of antibody-encoding vectors include prokaryotic or eukaryotic cells described herein. For example, antibodies may be produced in bacteria, in particular when glycosylation and Fc effector function are not needed. For expression of antibody fragments and polypeptides in bacteria, see, e.g., US 5,648,237, US 5,789,199, and US 5,840,523. (See also Charlton, K.A., In: Methods in Molecular Biology, Vol. 248, Lo, B.K.C. (ed.), Humana Press, Totowa, NJ (2003), pp. 245-254, describing expression of antibody fragments in *E. coli.*) After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. See Gerngross, T.U., Nat. Biotech. 22 (2004) 1409-1414; and Li, H. et al., Nat. Biotech. 24 (2006) 210-215.

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells.

Plant cell cultures can also be utilized as hosts. *See,* e.g., US Patent Nos. 5,959,177, 6,040,498, 6,420,548, 7,125,978, and 6,417,429 (describing PLANTIBODIES™ technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham, F.L. et al., J. Gen Virol. 36 (1977) 59-74); baby hamster kidney cells (BHK); mouse sertoli cells (TM4 cells as described, e.g., in Mather, J.P., Biol. Reprod. 23 (1980) 243-252); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, e.g., in Mather, J.P. et al., Annals N.Y. Acad. Sci. 383 (1982) 44-68; MRC 5 cells; and FS4 cells. Other useful mammalian host cell lines include Chinese hamster ovary (CHO) cells, including $DHFR^-$ CHO cells (Urlaub, G. et al., Proc. Natl. Acad. Sci. USA 77 (1980) 4216-4220); and myeloma cell lines such as Y0, NS0 and Sp2/0. For a review of certain mammalian host cell lines suitable for antibody production, see, e.g., Yazaki, P. and Wu, A.M., Methods in Molecular Biology, Vol. 248, Lo, B.K.C. (ed.), Humana Press, Totowa, NJ (2004), pp. 255-268.

**C. Assays**

Anti-PDl antibodies provided herein may be identified, screened for, or characterized for their physical/chemical properties and/or biological activities by various assays known in the art.

**1. Binding assays and other assays**

In one aspect, an antibody of the invention is tested for its antigen binding activity, e.g., by known methods such as ELISA, Western blot, etc.

In another aspect, competition assays may be used to identify an antibody that competes with PD1-0103 (comprising a VH sequence of SEQ ID NO:7 and a VL sequence of SEQ ID NO:8) for binding to PD1 (or alternatively with the humanized PD1-0103 variants antibodies PD1-0103-0312, PD1-0103-0313, PD1-0103-0314, PD1-0103-0315, with the identical 5 to 6 HVRs) . One embodiment of the invention is antibody which competes for binding to human PD1 with an anti-PDl antibody comprising all 3 HVRs of VH sequence of SEQ ID NO:7 and all 3 HVRs of VL sequence of SEQ ID NO:8. One embodiment of the invention is antibody which competes for binding to human PD1 with an anti-PDl antibody comprising all 3 HVRs of VH sequence of SEQ ID NO:57 and all 3 HVRs of VL sequence of SEQ ID NO:58. In certain embodiments, such a competing antibody binds to the same epitope (e.g., a linear or a conformational epitope) that is bound by anti-PDl antibody PD1-0103. Detailed exemplary methods for mapping an epitope to which an antibody binds are provided in Morris, G.E. (ed.), Epitope Mapping Protocols, In: Methods in Molecular Biology, Vol. 66, Humana Press, Totowa, NJ (1996).

In an exemplary competition assay, immobilized PD1(-ECD) is incubated in a solution comprising a first labeled antibody that binds to PD1 (e.g., anti-PDl antibody PD1-0103 or humanized antibody PD1-0103-0312) and a second unlabeled antibody that is being tested for its ability to compete with the first antibody for binding to PD1. The second antibody may be present in a hybridoma supernatant. As a control, immobilized PD1 is incubated in a solution comprising the first labeled antibody but not the second unlabeled antibody. After incubation under conditions permissive for binding of the first antibody to PD1, excess unbound antibody is removed, and the amount of label associated with immobilized PD1 is measured. If the amount of label associated with immobilized PD1 is substantially reduced in the test sample relative to the control sample, then that indicates that the second antibody is competing with the first antibody for binding to PD1. See Harlow, E. and Lane, D., Antibodies: A Laboratory Manual, Chapter 14, Cold Spring Harbor Laboratory, Cold Spring Harbor, NY (1988). For another exemplary competition assay see Example 2 (Epitope mapping ELISA/ Binding competition assay).

**2. Activity assays**

In one aspect, assays are provided for identifying anti-PDl antibodies thereof having biological activity. Biological activity may include, e.g., the ability to enhance the activation and/or proliferation of different immune cells especially T-cells. E.g. they enhance secretion of immunemodulating cytokines (e.g. interferon-gamma (IFN-gamma) and/or tumor necrosis factor alpha (TNF alpha)). Other immunemodulating cytokines which are or can be enahnce are e.g IL12, Granzyme B etc. Biological activity may also include, cynomolgous binding crossreactivity, as well as binding to different cell types. Antibodies having such biological activity in vivo and/or in vitro are also provided.

In certain embodiments, an antibody of the invention is tested for such biological activity as decribed e.g. in Examples below.

**D. Immunoconjugates (Cancer only or modify for target)**

The invention also provides immunoconjugates comprising an anti-PDl antibody herein conjugated to one or more cytotoxic agents, such as chemotherapeutic agents or drugs, growth inhibitory agents, toxins (e.g., protein toxins, enzymatically active toxins of bacterial, fungal, plant, or animal origin, or fragments thereof), or radioactive isotopes.

In one embodiment, an immunoconjugate is an antibody-drug conjugate (ADC) in which an antibody is conjugated to one or more drugs, including but not limited to a maytansinoid (see US 5,208,020, US 5,416,064 and EP 0 425 235 B1); an auristatin such as monomethyl auristatin drug moieties DE and DF (MMAE and MMAF) (see US 5,635,483, US 5,780,588, and US 7,498,298); a dolastatin; a calicheamicin or derivative thereof (see US 5,712,374, US 5,714,586, US 5,739,116, US 5,767,285, US 5,770,701, US 5,770,710, US 5,773,001, and US 5,877,296; Hinman, L.M. et al., Cancer Res. 53 (1993) 3336-3342; and Lode, H.N. et al., Cancer Res. 58 (1998) 2925-2928); an anthracycline such as daunomycin or doxorubicin (see Kratz, F. et al., Curr. Med. Chem. 13 (2006) 477-523; Jeffrey, S.C. et al., Bioorg. Med. Chem. Lett. 16 (2006) 358-362; Torgov, M.Y. et al., Bioconjug. Chem. 16 (2005) 717-721; Nagy, A. et al., Proc. Natl. Acad. Sci. USA 97 (2000) 829-834; Dubowchik, G.M. et al., Bioorg. & Med. Chem. Letters 12 (2002) 1529-1532; King, H.D. et al., J. Med. Chem. 45 (20029 4336-4343; and U.S. Patent No. 6,630,579); methotrexate; vindesine; a taxane such as docetaxel, paclitaxel, larotaxel, tesetaxel, and ortataxel; a trichothecene; and CC1065.

In another embodiment, an immunoconjugate comprises an antibody as described herein conjugated to an enzymatically active toxin or fragment thereof, including but not limited to diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes.

In another embodiment, an immunoconjugate comprises an antibody as described herein conjugated to a radioactive atom to form a radioconjugate. A variety of radioactive isotopes are available for the production of radioconjugates. Examples include $At^{211}$, $I^{131}$ , $I^{125}$ , $Y^{90}$, $Re^{186}$, $Re^{188}$ , $Sm^{153}$, $Bi^{212}$, $P^{32}$, $Pb^{212}$ and radioactive isotopes of Lu. When the radioconjugate is used for detection, it may comprise a radioactive atom for scintigraphic studies, for example $TC^{99m}$ or $I^{123}$, or a spin label for nuclear magnetic resonance (NMR) imaging (also known as magnetic resonance imaging, MRI), such as iodine-123 again, iodine-131, indium-111, fluorine-19, carbon-13, nitrogen-15, oxygen-17, gadolinium, manganese or iron.

Conjugates of an antibody and cytotoxic agent may be made using a variety of bifunctional protein coupling agents such as N-succinimidyl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta, E.S. et al., Science 238 (1987) 1098-1104. Carbon-14-labeled 1-isothiocyanatobenzyl-3-methyldiethylene triamine pentaacetic acid (MX-DTPA) is an exemplary chelating agent for conjugation of radionucleotide to the antibody. See WO 94/11026. The linker may be a "cleavable linker" facilitating release of a cytotoxic drug in the cell. For example, an acid-labile linker, peptidase-sensitive linker, photolabile linker, dimethyl linker or disulfide-containing linker (Chari, R.V. et al., Cancer Res. 52 (1992) 127-131; U.S. Patent No. 5,208,020) may be used. The immunuoconjugates or ADCs herein expressly contemplate, but are not limited to such conjugates prepared with cross-linker reagents including, but not limited to, BMPS, EMCS, GMBS, HBVS, LC-SMCC, MBS, MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, and sulfo-SMPB, and SVSB (succinimidyl-(4-vinylsulfone)benzoate) which are commercially available (e.g., from Pierce Biotechnology, Inc., Rockford, IL., U.S.A).

### E. Methods and Compositions for Diagnostics and Detection

In certain aspects, any of the anti-PDl antibodies provided herein is useful for detecting the presence of PD1 in a biological sample. The term "detecting" as used herein encompasses quantitative or qualitative detection. In certain aspects, a biological sample comprises a cell or tissue, such as immune cell or T cell infiltrates.

In one aspect, an anti-PDl antibody for use in a method of diagnosis or detection is provided. In a further aspect, a method of detecting the presence of PD1 in a biological sample is provided. In certain embodiments, the method comprises contacting the biological sample with an anti-PDl antibody as described herein under conditions permissive for binding of the anti-PDl antibody to PD1, and detecting whether a complex is formed between the anti-PDl antibody and PD1. Such method may be an *in vitro* or *in vivo* method. In one aspects, an anti-PDl antibody is used to select subjects eligible for therapy with an anti-PDl antibody, e.g. where PD1 is a biomarker for selection of patients.

In certain embodiments, labeled anti-PDl antibodies are provided. Labels include, but are not limited to, labels or moieties that are detected directly (such as fluorescent, chromophoric, electron-dense, chemiluminescent, and radioactive labels), as well as moieties, such as enzymes or ligands, that are detected indirectly, e.g., through an enzymatic reaction or molecular interaction. Exemplary labels include, but are not limited to, the radioisotopes $^{32}P$, $^{14}C$, $^{125}I$, $^{3}H$,

and $^{131}I$, fluorophores such as rare earth chelates or fluorescein and its derivatives, rhodamine and its derivatives, dansyl, umbelliferone, luceriferases, e.g., firefly luciferase and bacterial luciferase (U.S. Patent No. 4,737,456), luciferin, 2,3-dihydrophthalazinediones, horseradish peroxidase (HRP), alkaline phosphatase, β-galactosidase, glucoamylase, lysozyme, saccharide oxidases, e.g., glucose oxidase, galactose oxidase, and glucose-6-phosphate dehydrogenase, heterocyclic oxidases such as uricase and xanthine oxidase, coupled with an enzyme that employs hydrogen peroxide to oxidize a dye precursor such as HRP, lactoperoxidase, or microperoxidase, biotin/avidin, spin labels, bacteriophage labels, stable free radicals, and the like.

**F. Pharmaceutical Formulations**

Pharmaceutical formulations of an anti-PDl antibody as described herein are prepared by mixing such antibody having the desired degree of purity with one or more optional pharmaceutically acceptable carriers (Remington's Pharmaceutical Sciences, 16th edition, Osol, A. (ed.) (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyl dimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as poly(vinylpyrrolidone); amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counterions such as sodium; metal complexes (e.g. Zn-protein complexes); and/or non-ionic surfactants such as polyethylene glycol (PEG). Exemplary pharmaceutically acceptable carriers herein further include interstitial drug dispersion agents such as soluble neutral-active hyaluronidase glycoproteins (sHASEGP), for example, human soluble PH-20 hyaluronidase glycoproteins, such as rhuPH20 (HYLENEX®, Baxter International, Inc.). Certain exemplary sHASEGPs and methods of use, including rhuPH20, are described in US Patent Publication Nos. 2005/0260186 and 2006/0104968. In one aspect, a sHASEGP is combined with one or more additional glycosaminoglycanases such as chondroitinases.

Exemplary lyophilized antibody formulations are described in US Patent No. 6,267,958. Aqueous antibody formulations include those described in US Patent No. 6,171,586 and WO 2006/044908, the latter formulations including a histidine-acetate buffer.

The formulation herein may also contain more than one active ingredients as necessary for the particular indication being treated, preferably those with complementary activities that do not adversely affect each other. For example, it may be desirable to further provide. Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methyl methacrylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences, 16th edition, Osol, A. (ed.) (1980).

Sustained-release preparations may be prepared. Suitable examples of sustained-release preparations include semi-permeable matrices of solid hydrophobic polymers containing the antibody, which matrices are in the form of shaped articles, e.g. films, or microcapsules.

The formulations to be used for *in vivo* administration are generally sterile. Sterility may be readily accomplished, e.g., by filtration through sterile filtration membranes.

**G. Therapeutic Methods and Compositions**

Any of the anti-PDl antibodies (or antigen binding proteins) provided herein may be used in therapeutic methods.

In one aspect, an anti-PD1 antibody for use as a medicament is provided. In further aspects, an anti-PDl antibody or use in treating cancer is provided. In certain embodiments, an anti-PDl antibody for use in a method of treatment is provided. In certain embodiments, the invention provides an anti-PDl antibody for use in a method of treating an individual having cancer comprising administering to the individual an effective amount of the anti-PDl antibody.

In further embodiments, the invention provides an anti-PDl antibody for use as immunostimmulatory agent/ or stimulating interferon-gamma (IFN-gamma) secretion. In certain embodiments, the invention provides an anti-PDl antibody for use in a method of immunostimmulation/ or stimulating interferon-gamma (IFN-gamma) secretion in an individual comprising administering to the individual an effective of the the anti-PDl antibody for immunostimmulation/ or stimulating interferon-gamma (IFN-gamma) secretion.

In further aspects, the invention provides an anti-PDl antibody for use as immunostimmulatory agent/ or stimulating tumor necrosis factor alpha (TNF alpha) secretion. In certain embodiments, the invention provides an anti-PDl antibody for use in a method of immunostimmulation/ or stimulating tumor necrosis factor alpha (TNF alpha) secretion in an individual comprising administering to the individual an effective of the the anti-PD 1 antibody for immunostimmulation/ or stimulating tumor necrosis factor alpha (TNF alpha) secretion.

An "individual" according to any of the above aspects, is preferably a human. In a further aspect, the invention provides for the use of an anti-PD1 antibody in the manufacture or preparation of a medicament. In one embodiment, the medicament is for treatment of cancer. In a further aspects, the medicament is for use in a method of treating cancer comprising administering to an individual having cancer an effective amount of the medicament. In a further aspect, the medicament is for inducing cell mediated lysis of cancer cells In a further embodiment, the medicament is for use in a method of inducing cell mediated lysis of cancer cells in an individual suffering from cancer comprising administering to the individual an amount effective of the medicament to induce apoptosis in a cancer cell/ or to inhibit cancer cell proliferation. An "individual" according to any of the above aspects or embodiments, may be a human.

In a further aspect, the invention provides a method for treating cancer. In one embodiment, the method comprises administering to an individual having cancer an effective amount of an anti-PD1. An "individual" according to any of the above embodiments may be a human.

In a further aspect, the disclosure provides a method for inducing cell mediated lysis of cancer cells in an individual suffering from cancer. In one embodiment, the method comprises administering to the individual an effective amount of an anti-PD1 to induce cell mediated lysis of cancer cells in the individual suffering from cancer. In one embodiment, an "individual" is a human.

In a further aspect, the invention provides pharmaceutical formulations comprising any of the anti-PDl antibodies provided herein, e.g., for use in any of the above therapeutic methods. In one embodiment, a pharmaceutical formulation comprises any of the anti-PDl antibodies provided herein and a pharmaceutically acceptable carrier.

An antibody (and any additional therapeutic agent) can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein.

Antibodies would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The antibody need not be, but is optionally formulated with one or more agents currently used to prevent or treat the disorder in question. The effective amount of such other agents depends on the amount of antibody present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of disease, the appropriate dosage of an antibody of the invention (when used alone or in combination with one or more other additional therapeutic agents) will depend on the type of disease to be treated, the type of antibody, the severity and course of the disease, whether the antibody is administered for preventive or therapeutic purposes, previous therapy, the patient's clinical history and response to the antibody, and the discretion of the attending physician. The antibody is suitably administered to the patient at one time or over a series of treatments. Depending on the type and severity of the disease, about 1 $\mu$g/kg to 15 mg/kg (e.g. 0.5mg/kg - 10 mg/kg) of antibody can be an initial candidate dosage for administration to the patient, whether, for example, by one or more separate administrations, or by continuous infusion. One typical daily dosage might range from about 1 $\mu$g/kg to 100 mg/kg or more, depending on the factors mentioned above. For repeated administrations over several days or longer, depending on the condition, the treatment would generally be sustained until a desired suppression of disease symptoms occurs. One exemplary dosage of the antibody would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the patient. Such doses may be administered intermittently, e.g. every week or every three weeks (e.g. such that the patient receives from about two to about twenty, or *e.g.* about six doses of the antibody). An initial higher loading dose, followed by one or more lower doses may be administered. An exemplary dosing regimen comprises administering an initial loading dose of about 4 mg/kg, followed by a weekly maintenance dose of about 2 mg/kg of the antibody. However, other dosage regimens may be useful. The progress of this therapy is easily monitored by conventional techniques and assays.

It is understood that any of the above formulations or therapeutic methods may be carried out using an immunoconjugate of the invention in place of or in addition to an anti-PDl antibody.

## II. Articles of Manufacture

In another aspect, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active agent in the composition is an antibody of the invention. The label or package insert indicates that the composition is used for treating the condition of choice. Moreover, the article of manufacture may comprise (a) a first container with a composition contained therein, wherein the composition comprises an antibody of the invention; and (b) a second container with a composition contained therein, wherein the composition comprises a further cytotoxic or otherwise therapeutic agent. The article of manufacture may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

It is understood that any of the above articles of manufacture may include an immunoconjugate of the invention in place of or in addition to an anti-PD1 antibody.

**Description of the amino acid sequences**

| | |
|---|---|
| SEQ ID NO: 1 | heavy chain HVR-H1, PD1-0103 |
| SEQ ID NO: 2 | heavy chain HVR-H2, PD1-0103 |
| SEQ ID NO: 3 | heavy chain HVR-H3, PD1-0103 |
| SEQ ID NO: 4 | light chain HVR-L1, PD1-0103 |
| SEQ ID NO: 5 | light chain HVR-L2, PD1-0103 |
| SEQ ID NO: 6 | light chain HVR-L3, PD1-0103 |
| SEQ ID NO: 7 | heavy chain variable domain VH, PD1-0103 |
| SEQ ID NO: 8 | light chain variable domain VL, PD1-0103 |
| SEQ ID NO: 9 | heavy chain HVR-H1, PD1-0098 |
| SEQ ID NO: 10 | heavy chain HVR-H2, PD1-0098 |
| SEQ ID NO: 11 | heavy chain HVR-H3, PD1-0098 |
| SEQ ID NO: 12 | light chain HVR-L1, PD1-0098 |
| SEQ ID NO: 13 | light chain HVR-L2, PD1-0098 |
| SEQ ID NO: 14 | light chain HVR-L3, PD1-0098 |
| SEQ ID NO: 15 | heavy chain variable domain VH, PD1-0098 |
| SEQ ID NO: 16 | light chain variable domain VL, PD1-0098 |
| SEQ ID NO: 17 | heavy chain HVR-H1, PD1-0050 |
| SEQ ID NO: 18 | heavy chain HVR-H2, PD1-0050 |
| SEQ ID NO: 19 | heavy chain HVR-H3, PD1-0050 |
| SEQ ID NO: 20 | light chain HVR-L1, PD1-0050 |
| SEQ ID NO: 21 | light chain HVR-L2, PD1-0050 |
| SEQ ID NO: 22 | light chain HVR-L3, PD1-0050 |
| SEQ ID NO: 23 | heavy chain variable domain VH, PD1-0050 |
| SEQ ID NO: 24 | light chain variable domain VL, PD1-0050 |
| SEQ ID NO: 25 | heavy chain HVR-H1, PD1-0069 |
| SEQ ID NO: 26 | heavy chain HVR-H2, PD1-0069 |
| SEQ ID NO: 27 | heavy chain HVR-H3, PD1-0069 |
| SEQ ID NO: 28 | light chain HVR-L1, PD1-0069 |
| SEQ ID NO: 29 | light chain HVR-L2, PD1-0069 |

(continued)

| | |
|---|---|
| SEQ ID NO: 30 | light chain HVR-L3, PD1-0069 |
| SEQ ID NO: 31 | heavy chain variable domain VH, PD1-0069 |
| SEQ ID NO: 32 | light chain variable domain VL, PD1-0069 |
| SEQ ID NO: 33 | heavy chain HVR-H1, PD1-0073 |
| SEQ ID NO: 34 | heavy chain HVR-H2, PD1-0073 |
| SEQ ID NO: 35 | heavy chain HVR-H3, PD1-0073 |
| SEQ ID NO: 36 | light chain HVR-L1, PD1-0073 |
| SEQ ID NO: 37 | light chain HVR-L2, PD1-0073 |
| SEQ ID NO: 38 | light chain HVR-L3, PD1-0073 |
| SEQ ID NO: 39 | heavy chain variable domain VH, PD1-0073 |
| SEQ ID NO: 40 | light chain variable domain VL, PD1-0073 |
| SEQ ID NO: 41 | heavy chain HVR-H1, PD1-0078 |
| SEQ ID NO: 42 | heavy chain HVR-H2, PD1-0078 |
| SEQ ID NO: 43 | heavy chain HVR-H3, PD1-0078 |
| SEQ ID NO: 44 | light chain HVR-L1, PD1-0078 |
| SEQ ID NO: 45 | light chain HVR-L2, PD1-0078 |
| SEQ ID NO: 46 | light chain HVR-L3, PD1-0078 |
| SEQ ID NO: 47 | heavy chain variable domain VH, PD1-0078 |
| SEQ ID NO: 48 | light chain variable domain VL, PD1-0078 |
| SEQ ID NO: 49 | heavy chain HVR-H1, PD1-0102 |
| SEQ ID NO: 50 | heavy chain HVR-H2, PD1-0102 |
| SEQ ID NO: 51 | heavy chain HVR-H3, PD1-0102 |
| SEQ ID NO: 52 | light chain HVR-L1, PD1-0102 |
| SEQ ID NO: 53 | light chain HVR-L2, PD1-0102 |
| SEQ ID NO: 54 | light chain HVR-L3, PD1-0102 |
| SEQ ID NO: 55 | heavy chain variable domain VH, PD1-0102 |
| SEQ ID NO: 56 | light chain variable domain VL, PD1-0102 |
| SEQ ID NO: 57 | humanized variant -heavy chain variable domain VH of PD1-0103_01 |
| SEQ ID NO: 58 | humanized variant -light chain variable domain VL of PD1-0103_01 |
| SEQ ID NO: 59 | humanized variant -light chain variable domain VL of PD1-0103_02 |
| SEQ ID NO: 60 | humanized variant -light chain variable domain VL of PD1-0103_03 |
| SEQ ID NO: 61 | humanized variant -light chain variable domain VL of PD1-0103_04 |
| SEQ ID NO: 62 | human kappa light chain constant region |
| SEQ ID NO: 63 | human lambda light chain constant region |
| SEQ ID NO: 64 | human heavy chain constant region derived from IgG1 |
| SEQ ID NO: 65 | human heavy chain constant region derived from IgG1 with mutations L234A and L235A |
| SEQ ID NO: 66 | human heavy chain constant region derived from IgG1 with mutations L234A, L235A and P329G |
| SEQ ID NO: 67 | human heavy chain constant region derived from IgG4 |
| SEQ ID NO: 68 | exemplary human PD1 sequence (without signal sequence) |
| SEQ ID NO: 69 | human PD1 Extracellular Domain (ECD) |
| SEQ ID NO: 70 | exemplary human PD1 sequence (including signal sequence) |
| SEQ ID NO: 71: | Minimal HVR1 of PD1-0103 and PD1-0103 humanized variant PD1-0103-0312, PD1-0103-0313, PD1-0103-0314 , and PD1-0103-0315 |
| SEQ ID NO: 72: | Minimal HVR2 of PD1-0103 and PD1-0103 humanized variant PD1-0103-0312, PD1-0103-0313, PD1-0103-0314 , and PD1-0103-0315 |

(continued)

| | |
|---|---|
| SEQ ID NO: 73: | Minimal HVR3 of PD1-0103 and PD1-0103 humanized variant PD1-0103-0312, PD1-0103-0313, PD1-0103-0314 , and PD1-0103-0315 |
| SEQ ID NO: 74: | Minimal LVR1 of PD1-0103 and PD1-0103 humanized variant PD1-0103-0312, PD1-0103-0313, PD1-0103-0314 , and PD1-0103-0315 |
| SEQ ID NO: 75: | Minimal LVR2 of PD1-0103 and PD1-0103 humanized variant PD1-0103-0312, PD1-0103-0313, PD1-0103-0314 , and PD1-0103-0315 |
| SEQ ID NO: 76: | Minimal LVR3 of PD1-0103 and PD1-0103 humanized variant PD1-0103-0312, PD1-0103-0313, PD1-0103-0314 , and PD1-0103-0315 |
| SEQ ID NO: 77: | fragment of FR-H3 comprising the amino acid sequence RDN at positions of 71, 72, 73 according to Kabat numbering |

In the following the amino acid sequences of the VH und VL domains including marked HVRs (HVRs in bold, underlined letters) of anti-PDI antibodies PD1-0016 (and its humanized versions PD1-0103-0312, PD 1-0103-0313, PD1-0103-0314 and PD1-0103-0315), PD1-0098, PD1-0050, PD1-0069, PD1-0073, PD1-0078 and PD1-0102 are listed:

anti-PD1 PD1-0103:

VH PD1-0103:

EVILVESGGGLVKPGGSLKLSCAAS**GFSFSSY**TMSWVRQTPEKRLDWVATISG**GGR**DIYYPDSVKGR
FTISRDNAKNTLYLEMSSLMSEDTALYYCVLL**TGRVYFALD**SWGQGTSVTVSS

VL PD1-0103:

KIVLTQSPASLPVSLGQRATISCRA**SESVDTSDNSF**IHWYQQRPGQSPKLLIY**RSS**TLESGVPARFS
GSGSRTDFTLTIDPVEADDVATYYCQQ**NYDVPW**TFGGGTKLEIK

**Humanized anti-PD1 PD1-0103 versions PD1-0103-0312, PD1-0103-0313, PD1-0103-0314 and PD1-0103-0315:**

**VH PD1-0103-0312= VH PD1-0103-0313= VH PD1-0103-0314= VH PD1-0103-0315:**

EVQLLESGGGLVQPGGSLRLSCAAS**GFSFSSY**TMSWVRQAPGKGLEWVATISG**GGR**DIYYPDSVKGR
FTISRDNSKNTLYLQMNSLRAEDTAVYYCVLL**TGRVYFALD**SWGQGTLVTVSS

**VL PD1-0103-0312:**

DIVMTQSPDSLAVSLGERATINCKA**SESVDTSDNSF**IHWYQQKPGQSPKLLIY**RSS**TLESGVPDRFS
GSGSGTDFTLTISSLQAEDVAVYYCQQ**NYDVPW**TFGQGTKVEIK

**VL PD1-0103-0313:**

DVVMTQSPLSLPVTLGQPASISCRA**SESVDTSDNSF**IHWYQQRPGQSPRLLIY**RSS**TLESGVPDRFS
GSGSGTDFTLKISRVEAEDVGVYYCQQ**NYDVPW**TFGQGTKVEIK

**VL PD1-0103-0314:**

EIVLTQSPATLSLSPGERATLSCRA**SESVDTSDNSF**IHWYQQKPGQSPRLLIY**RSS**TLESGIPARFS
GSGSGTDFTLTISSLEPEDFAVYYCQQ**NYDVPW**TFGQGTKVEIK

**VL PD1-0103-0315:**

EIVLTQSPATLSLSPGERATLSCRA**SESVDTSDNSF**IHWYQQKPGQSPRLLIY**RSS**TLESGIPARFS GSGSGTDFTLTISSLEPEDFAVYYCQQ**NYDVPW**TFGQGTKVEIK

**anti-PD1 PD1-0098:**

**VH PD1-0098:**

DVQLQESGPGLVKPSQSLSLTCTVT**GYSITSDY**AWNWIRQFPGDKLEWLGYIT**YSG**FTNYNPSLKSR ISISRDTSKNQFFLQLNSVATEDTATYYCARW**HGSAPWYFD**YWGRGTTLTVSS

**VL PD1-0098:**

DVLMTQTPLSLPVSLGDQASISCRS**SQNIVHSDGNTY**LEWYLQKPGQSPNLLIY**KVS**RRFSGVPDRF SGSGSGTDFTLKISRVEAEDLGVYYCFQ**GSHFPL**TFGAGTKLELK

**VH: 0050**

DVQLQESGPGLVKPSQSLSLTCTVT**GYSITSDY**AWNWIRQFPGNKLEWMGYIT**YTG**RTSYNPSLKSR ISITRDTSKNQFFLQLNSVTTEDTATYYCARE**MDYYGSTLD**YWGQGTTLTVSS

**VL: 0050**

KIVLTQSPASLAVSLRQRATISCRA**SESVDRYGNSF**IHWYQQKPGQPPKVLIY**RAS**NLESGFPARFS GSGSRTDFTLTIDPVEADDAATYYCQQ**NNEDPY**TFGSGTKLEIK

**VH: 0069**

QVQLQQSGPELVRPGVSVKISCKGS**GYTFTDY**AMHWVKQSHARTLEWIGVIST**YSG**DTNYNQKFKDK ATMTVDKSSSTAYLELARMTSEDSAIYYCARL**GITTGFA**YWGQGTLVTVSA

**VL: 0069**

DIVLTQSPASLAVSLGQRATISCRA**SKGVSTSSYSF**MHWYQQKPRQPPKLLIK**YAS**YLESGVPARFS GSGSGTDFTLNIHPVEEEDAATYYCHH**SREFPW**TFGGGTKLEIK

**VH: 0073**

EVKLVESGGGLVKPGGSLKLSCAAS**GFTFSNY**GMSWIRQTPEKGLEWVATISG**GGR**DTYYPDSVKGR FTISRDNVKNNLYLQMSSLRSEDTAFYYCASY**YYGID**YWGQGTSVTVSS

**VL: 0073**

DIVMTQPHKFMSTSVGDRVRITCKA**SQDVTTA**VAWYQQKPGQSPKLLIY**WAS**TRHTGVPDRFTGSGS GTEFTLTISSVQAEDLALYYCQQ**HYSIPW**TFGGGTKLEIK

**VH: 0078**

QVQLQQPGAELVKPGASVKMSCKAS**GYTFTST**WMHWVKQRPGQGLEWIGAIDP**SDS**YTTYNQKFKGK ATLTVDTSSTTAYMQLSSLTSEDSAVYYCTRS**PFD**YWGQGTTLTVSS

**VL: 0078**

DIVMTQSHKFMSTSVGDRVSITCKA**SQDVSTA**VAWYQQKPGQSPKLLIY**SAS**YRYTGVPDRFTGSGS GTDFTFAISSVQAEDLAVYYCQQ**HYSHPF**TFGSGTKLEIK

**VH: 0102**

DVQLQESGPDLVKPSQSLSLTCTVT**GYSITSGY**SWHWIRQFPGNKLEWMGFIH**SSG**DTNYNPSLKSR ISFTRDTSKNQFFLQLSSLTDEDTATYYCATY**RNWYFD**VWGAGTTVTVSS

**VL: 0102**

DIVMTQSPSSLTVTAGEKVTMRCKS**SQSLLNSGTQKNY**LTWYQQKPGQPPKLLIY**WAS**TRESGVPNR FTGSGSGTDFTLTISSVQAEDLSVYYCQS**DYTFPL**TFGGGTKLELK

## III. EXAMPLES

The following are examples of methods and compositions of the invention.

### Example 1:

### Generation of anti-PD-1 antibodies

### Immunization of mice

NMRI mice were immunized genetically, using a plasmid expression vector coding for full-length human PD-1 by intradermal application of 100 ug vector DNA (plasmidl5300_hPD1-fl), followed by Electroporation (2 square pulses of 1000 V/cm, duration 0.1 ms, interval 0.125 s; followed by 4 square pulses of 287.5 V/cm, duration 10 ms, interval 0.125 s. Mice received either 6 consecutive immunizations at days 0, 14, 28, 42, 56, 70, and 84. Blood was taken at days 36, 78 and 92 and serum prepared, which was used for titer determination by ELISA (see below). Animals with highest titers were selected for boosting at day 96, by intravenous injection of 50 ug of recombinant human PD1 human Fc chimera, and monoclonal antibodies were isolated by hybridoma technology, by fusion of splenocytes to myeloma cell line 3 days after boost.Determination of serum titers (ELISA).

Human recombinant PD1 human Fc chimera was immobilized on a 96-well NUNC Maxisorp plate at 0.3 ug/ml, 100 ul/well, in PBS, followed by: blocking of the plate with 2% Crotein C in PBS, 200 ul/well; application of serial dilutions of antisera, in duplicates, in 0.5% Crotein C in PBS, 100 ul/well; detection with HRP-conjugated goat anti-mouse antibody (Jackson Immunoresearch/Dianova 115-036-071; 1/16 000). For all steps, plates were incubated for 1 h at 37° C. Between all steps, plates were washed 3 times with 0.05% Tween 20 in PBS. Signal was developed by addition of BM Blue POD Substrate soluble (Roche), 100 ul/well; and stopped by addition of 1 M HCl, 100 ul/well. Absorbance was read out at 450 nm, against 690 nm as reference. Titer was defined as dilution of antisera resulting in half-maximal signal.

### Example 2:

### Characterization anti-PDI antibodies

### Binding of anti-PDI antibodies to human PD1

### ELISA for hu PD1

Nunc maxisorp streptavidin coated plates (MicroCoat #11974998001) were coated with 25 μl/well biotinylated PD1-ECD-AviHis and incubated at 4°C over night. After washing (3x90 μl/well with PBST-buffer) 25 μl anti PD1 samples

or reference antibodies (human anti PD1; Roche/mouse anti PD1; Biolegend; cat.:329912) were added and incubated 1h at RT. After washing (3x90 µl/well with PBST-buffer) 25µl/well goat-anti-human H+L-POD (JIR, JIR109-036-088)/ Sheep-anti-mouse-POD (GE Healthcare; NA9310) was added in 1:2000/1: 1000 dilution and incubated at RT for 1 h on shaker. After washing (3x90 µl/well with PBST-buffer) 25 µl/well TMB substrate (Roche Catalogue No. 11835033001) was added and incubated until OD 2 - 3. Measurement took place at 370/492 nm.

ELISA results are listed as EC50-values [ng/ml] in summary Tables 2 and 3 below.

**Cell ELISA for PD1**

Adherent CHO-K1 cell line stably transfected with plasmid 15311_hPD1-fl_pUC_Neo coding for full-length human PD1 and selection with G418 (Neomycin restistance marker on plasmid) were seeded at a concentration of 0.01x10E6 cells/well in 384-well flat bottom plates and grown over night.

The next day 25 µl/well PD1 sample or human anti PD1 (Roche)/mouse anti PD1(Biolegend; cat.:329912) reference antibody were added and incubated for 2h at 4°C (to avoid internalization). After washing carefully (1x90µl/well PBST) cells were fixed by adding 30µl/well 0,05% Glutaraldehyde (Sigma, Cat.No: G5882, 25%)diluted in 1xPBS-buffer and incubated for 10min at RT. After washing (3x90µl/well PBST) 25 µl/well secondary antibody was added for detection: goat-anti-human H+L-POD (JIR, JIR109-036-088)/Sheep-anti-mouse-POD (GE NA9310) followed by 1h incubation at RT on shaker. After washing (3x90µl/well PBST) 25 µl/well TMB substrate solution (Roche 11835033001) was added and incubated until OD 1.0 - 2.0. Plates were measured at 370/492 nm.

Cell ELISA results are listed as "EC50 CHO-PD1"-values [ng/ml] in summary table Table 3 below.

**ELISA for cyno PD1**

Nunc maxisorp streptavidin coated plates (MicroCoat #11974998001) were coated with 25 µl/well biotinylated cynoPD1-ECD-Biotin and incubated at 4°C over night. After washing (3x90 µl/well with PBST-buffer) 25 µl anti PD1 samples or reference antibodies (human anti PD1; Roche) were added and incubated 1h at RT on shaker. After washing (3x90 µl/well with PBST-buffer) 25µl/well goat-anti-human H+L-POD (JIR, JIR109-036-088) was added in 1:1000 dilution and incubated at RT for 1 h on shaker. After washing (3x90 µ|/well with PBST-buffer) 25 µ|/well TMB substrate (Roche, 11835033001) was added and incubated until OD 2 - 3. Measurement took place at 370/492 nm.

ELISA results are listed as EC50-values [ng/ml] in summary Table 2 and 3 below.

**PD Ligand 1 replacing assay**

Nunc maxisorp streptavidin coated plates (MicroCoat #11974998001) were coated with 25 µl/well biotinylated PD1-ECD-AviHis and incubated at 4°C over night. After washing (3x90 µl/well with PBST-buffer) 25 µl anti PD1 samples or reference antibodies (mouse anti PD1; Biolegend; cat.:329912) were added and incubated 1h at RT on shaker. After washing (3x90 µl/well with PBST-buffer) 25µl/well PD-L1 (Recombinant human B7-H1/PD-L1 Fc Chimera; 156-B7, R&D) was added and incubated 1h at RT on shaker. After washing (3x90 µl/well with PBST-buffer) 25µl/well goat-anti-human H+L-POD (JIR, 109-036-088) was added in 1:1000 dilution and incubated at RT for 1 h on shaker. After washing (3x90 µl/well with PBST-buffer) 25 µl/well TMB substrate (Roche, 11835033001) was added and incubated until OD 2 - 3. Measurement took place at 370/492 nm.

ELISA results are listed as IC50-values [ng/ml] in summary Table 2 below.

**PD Ligand 2 replacing assay**

Nunc maxisorp streptavidin coated plates (MicroCoat #11974998001) were coated with 25 µl/well biotinylated PD1-ECD-AviHis and incubated at 4°C over night. After washing (3x90 µl/well with PBST-buffer) 25 µl anti PD1 samples or reference antibodies (mouse anti huPD1; Roche) were added and incubated 1h atRT on shaker. After washing (3x90 µl/well with PBST-buffer) 25µ|/well PD-L2 (Recombinant human B7-DC/PD-L2 Fc Chimera; 1224-PL-100, R&D) was added and incubated 1h at RT on shaker. After washing (3x90 µl/well with PBST-buffer) 25µl/well goat-anti-human H+L-POD (JIR, 109-036-088) was added in 1:2000 dilution and incubated at RT for 1 h on shaker. After washing (3x90 µl/well with PBST-buffer) 25 µl/well TMB substrate (Roche, 11835033001) was added and incubated until OD 2 - 3. Measurement took place at 370/492 nm.

ELISA results are listed as IC50-values [ng/ml] in summary Table 2 below.

**Epitope mapping ELISA/ Binding competition assay**

Nunc maxisorp plates (Nunc #464718) were coated with 25µl/well capture antibody (goat anti mouse IgG; JIR;

115-006-071) and incubated for 1h at RT on shaker. After washing (3x90μl/well with PBST-buffer) plates were blocked for 1h with 2% BSA containing PBS buffer at RT on shaker. After washing (3x90μl/well with PBST-buffer) 25μl mouse anti PD1 samples were added and incubated 1h at RT on shaker. After washing (3x90μl/well with PBST-buffer) capture antibody was blocked by 30μl/well mouse IgG (JIR; 015-000-003) for 1h at RT on shaker. At the same time biotinylated PD1-ECD-AviHis was preincubated with second sample antibody for 1h at RT on shaker. After washing assay plate (3x90 μl/well with PBST-buffer) the PD1 antibody mix was transferred to assay plate and incubated at RT for 1h on shaker. After washing (3x90 μl/well with PBST-buffer) 25μl/well streptavidin POD (Roche, #11089153001) was added in 1:4000 dilution and incubated at RT for 1 h on shaker. After washing (3x90 μl/well with PBST-buffer) 25 μl/well TMB substrate (Roche, #11089153001) was added and incubated until OD 1.5 - 2.5. Measurement took place at 370/492 nm. Epitope groups were defined by hierarchical clustering against reference antibodies.

**Table 2: Binding, PD-L1 inhibition and epitope region groups of exemplary antibodies (ELISA)**

| Antibody | ELISA huPD1 EC50 [ng/ml] | ELISA cyPD1 EC50 [ng/ml] | ELISA PD-L1 inhibition IC50 [ng/ml] | ELISA PD-L2 inhibition IC50 [ng/ml] | Epitope region group By competion assay) |
|---|---|---|---|---|---|
| PD1- 0050 | 17.9 | 9.8 | 128 | 34 | 1 |
| PD1- 0069 | 45.7 | 22.7 | 225 | 89 | 6 |
| PD1- 0073 | 15.1 | 8.3 | 124 | 65 | 5 |
| PD1- 0078 | 26.3 | 22.4 | x | 86 | 2 |
| PD1- 0098 | 50.8 | 54.6 | 174 | 45 | 5 |
| PD1- 0102 | 34.2 | 52.7 | >35.5 μg/ml | 140 | 4 |
| PD1-0103 | 33.7 | 36.9 | 182 | 51 | 5 |

**Table 3: Biochemial- and Cell-binding of humanized PD1 antibodies derived from parental mouse antibody PD1-0103 (ELISA).**

| Humanize d antibody | ELISA huPD1 EC50 [ng/ml] | ELISA cyPD1 EC50 [ng/ml] | ELISA CHO-PD1 EC50 [ng/ml] |
|---|---|---|---|
| PD1-0103-0312 | 11 | 8.3 | 10.1 |
| PD1-0103-0313 | 15 | 11 | 10.8 |
| PD1-0103-0314 | 11 | 8.3 | 7.7 |
| PD1-0103-0315 | 10 | 7.9 | 7.3 |

**Biacore characterization of the humanized anti-PD-1 antibodies**

A surface plasmon resonance (SPR) based assay has been used to determine the kinetic parameters of the binding between several murine PD1 binders as well as commercial human PD1 binding references. Therefore, an anti-human IgG was immobilized by amine coupling to the surface of a (Biacore) CM5 sensor chip. The samples were then captured and hu PD1-ECD was bound to them. The sensor chip surface was regenerated after each analysis cycle. The equilibrium constant and kinetic rate constants were finally gained by fitting the data to a 1:1 langmuir interaction model.

About 2000 response units (RU) of 20 μg/ml anti-human IgG (GE Healthcare #BR-1008-39) were coupled onto the flow cells 1 and 2 (alternatively: 3 and 4) of a CM5 sensor chip in a Biacore T200 at pH 5.0 by using an amine coupling kit supplied by GE Healthcare.

The sample and running buffer was HBS-EP+ (0.01 M HEPES, 0.15 M NaCl, 3 mM EDTA, 0.05 % v/v Surfactant P20, pH 7.4). Flow cell temperature was set to 25 °C and sample compartment temperature to 12 °C. The system was primed with running buffer.

The samples were injected for 20 seconds with a concentration of 10 nM and bound to the second flow cell. Then a complete set of human PD1-ECD concentrations (144 nM, 48 nM, 16 nM, 5.33 nM, 1.78 nM, 0.59 nM, 0.20 nM and 0 nM) was injected over each sample for 120s followed by a dissociation time of 30/300s and two 20s regeneration steps with 3 M $MgCl_2$, of which the last one contained an "extra wash after injection" with running buffer.

Finally the double referenced data was fitted to a 1:1 langmuir interaction model with the Biacore T200 Evaluation Software. Resulting $K_D$, $k_a$ and $k_d$ values are shown in Table 4.

**Table 4: Kinetic rate constants and equilibrium constant for chimeric PD1-0103 and humanized PD1-Abs determined by Biacore (see next page).**

| Ligand | $k_a$[$M^{-1}s^{-1}$] | $k_d$[$s^{-1}$] | $K_D$ [nM] |
|---|---|---|---|
| chimeric PD1-0103 | 3.86E+05 | 3.07E-04 | 0.8 |
| PD1-0103-0312 | 1.95E+05 | 3.45E-04 | 1.8 |
| PD1-0103-0313 | 1.60E+05 | 3.67E-04 | 2.3 |
| PD1-0103-0314 | 1.87E+05 | 2.79E-04 | 1.5 |
| PD1-0103-0315 | 1.89E+05 | 2.91E-04 | 1.5 |

As shown in Table 4, all the humanized versions of chimeric PD1-0103 (generation see Example 6) display kinetic properties similar to the parental antibody (chimeric PD1-0103).

**Kinetics**

A CM5 sensor series S was mounted into the Biacore 4000 System and the detection spots were hydrodynamically addressed according to the manufacturer's instructions.

The polyclonal rabbit IgG antibody <IgGFCγM>R (Jackson ImmunoResearch Laboratories Inc.) was immobilized at 10 000 Ru on the detection spots 1 and 5 in the flow cells 1,2,3 and 4. Coupling was done via EDC/NHS chemistry according to the manufacturer's instructions. The remaining spots in the flow cells served as a reference. The sample buffer was the system buffer supplemented with 1 mg/ml carb oxymethyl dextrane.

In one embodiment the assay was driven at 25 °C. In another embodiment the assay was driven at 37 °C. 50 nM of each murine monoclonal antibody was captured on the sensor surface by a 1 min injection at 10 μl/min. Subsequently the respective antigens were injected in a concentration series of 100 nM, 2x 33 nM, 11 nM, 4 nM, 1 nM and system buffer 0 nM at 30μl/min for 4 min association phase time. The dissociation was monitored for another 4 min. The capture system was regenerated using a 3 min injection of 10 mM glycine pH 1.5 at 30 μl/min. Relevant kinetic data was calculated using the Biacore evaluation software according to the manufacturer's instructions.

**Epitope Mapping**

A Biacore 4000 instrument was mounted with a Biacore CAP sensor and was prepared like recommended by the manufacturer. The instrument buffer was HBS-ET (10 mM HEPES pH 7.4, 150 mM NaCl, 3 mM EDTA, 0.005% w/v Tween 20). The instrument was running at 25 °C.

All samples were diluted in system buffer. A 35kDa biotinylated antigen PD1-ECD-AviHis was captured at 200 RU on the CAP sensor surface by a 1 min injection at 30μl/min in the flow cells 1, 2, 3 and 4 in the spots 1 and 5. Spots 2, 3 and 4 served as a reference. In another embodiment, a 35 kDa biotinylated antigen PD1-ECD-AviHis was captured at 200 RU on the CAP sensor in the same manner.

Subsequently a primary antibody was injected at 100 nM for 3 min at 30 μl/min followed by the injection of a secondary antibody at 100 nM for 3 min at 30 μl/min. The primary antibody was injected until full saturation of the surface presented antigen. At the end of the primary and secondary antibody injection phases report points "Binding Late" (BL) were set to monitor the binding response of the respective antibodies. The Molar Ratio, a quotient between the secondary antibody binding response "BL2" and the primary antibody response "BL1" was calculated. The Molar Ratio was used as an indicator of the antigen accessibility of the secondary antibody, when the antigen was already complexed by the primary antibody.

The complexes were completely removed from the sensor surface by an injection for 2 min at 30μl/min 2M guanidine-HCL 250 mM NaOH regeneration buffer as recommended by the manufacturer, followed by a 1 min injection at 30μl /min of system buffer.

**Example 3:**

**Effect of different anti-PD-1 Antibodies on Cytokine Production in a Mixed Lymphocyte Reaction (MLR)**

3A) The Mixed Lymphocyte Reaction (MLR) is a immune cell assay which measures the activation of lymphocytes from one individual (donor X) to lymphocytes from another individual (donor Y). A mixed lymphocyte reaction was used to demonstrate the effect of blocking the PD1 pathway to lymphocyte effector cells. T cells in the assay were tested for activation and theier IFN-gamma secretion in the presence or absence of an anti-PDl mAbs.

To perform an allogeneic MLR, peripheral blood mononuclear cells (PBMCs) from at least four healthy donors of unknown HLA type were isolated by density gradient centrifugation using Leukosep (Greiner Bio One, 227 288). Briefly, heparinized blood samples were diluted with the three fold volume of PBS and 25 ml aliquots of the diluted blood were layered in 50 ml Leukosep tubes. After centrifugation at 800 x g for 15 min at room temperature (w/o break) the lymphocyte containing fractions were harvested, washed in PBS and used directly in functional assay or resuspended in freezing medium (10% DMSO, 90 %FCS) at 1.0E+07 cells/ml and stored in liquid nitrogen. Individual 2-way MLR reactions were set up by mixing PBMCs from two different donors at a 1:1 stimulator/responder cell ratio and co-cultures were done at least in duplicate in flat-bottomed 96-well plates for 6 days at 37oC, 5% CO2, in the presence or w/o of a different concentration range of purified anti-PDl monoclonal antibodies PD1-0050, PD1-0069, PD1-0073, PD1-0078, PD1-0098, PD1-0102, PD1-0103. As reference anti-PD1 antibodies , antibodies comprising the VH and VL domains of either nivolumab (also known as MDX-5C4 or MDX-1106) or pembrolizumab (also known as MK-3475 or Org 1.09A) were synthesized and cloned with backbones of human IgG1 (with mutations L234A, L235A and P329G (EU index of Kabat)). Either no antibody or an isotype control antibody was used as a negative control and rec hu IL-2 (20 EU/ml) was used as positive control. After day 6 100 μl of medium was taken from each culture for cytokine measurement. The levels of IFN-gamma were measured using OptEIA ELISA kit (BD Biosciences).

The results are shown in Table 5 (IFN-g secretion/release). The anti-PDl monoclonal antibodies promoted T cell activation and IFN-gamma secretion in concentration dependent manner. The value of % increase of IFNg secretion was calculated in relation to IFN-g production of MLR w/o adding of any blocking mAbs (basal allogeneic stimulation induced IFNg value as E-c) and MLR with adding of 20 EU/ml rec hu IL-2 (positive control = 100% IFNg value as E+c) and was calculated according to formula: Rel.Stimulation [%] = ((Esampel - E-c)/(E+c - E-c)*100

**Table 5: Percentage of of IFN gamma secretion after allogenic stimulation andtreatment with anti-PD-1 antibody in comparison to effect of recombinant human IL-2 treatment (20 EU/ml) (= 100% increase) as positive control**

| | Concentration (μg/ml) | 1:12 | 1:120 | 1:1200 | Effect in MLR |
|---|---|---|---|---|---|
| PD1-0050 | 44 | 136 | 96 | 33 | +++ |
| PD1-0069 | 60 | 76 | 71 | 55 | +++ |
| PD1-0073 | 43 | 103 | 63 | 38 | ++ |
| PD1-0078 | 64 | 99 | 72 | 21 | ++ |

Several PD1 blocking antibodies PD1-0050, PD1-0069, PD1-0073, PD1-0078, PD1-0098, PD1-0102, PD1-0103 demonstrated strong immune modulating activity by enhancing secretion of interferon gamma (IFN-g) (data not shown for all antibodies.

3B) In a further experiment chimeric PD1-0103 (human IgG1 isotype with mutations L234A, L235A and P329G (EU index of Kabat)) was evaluated. Blockade of PD1 with chimeric PD1-0103 strongly enhances IFN-gamma secretion by allogenic stimulated primary human T cells. Chimeric PD1-0103 is more potent than reference anti-PDl antibodies (see Figure 1).

For comparison the reference anti-PDl antibodies comprising the VH and VL domains of either nivolumab (also known as MDX5C4 or MDX-1106) and pembrolizumab (also known as MK-3475 or Org 1.09A) were synthesized and cloned with backbones of human IgG1 (with mutations L234A, L235A and P329G (EU index of Kabat)) were used.

3C) In additional experiments the immune modulating activity of the humanized variants of anti-PD-1 antibody PD1-0103 (humanized antibodies PD1-0103-0312, , PD1-0103-0314, in figures 2 and 3, see also Example 6 below) the a) IFNrelease (secretion) b) TNF-alpha release (secretion) was evaluated in MLR as described above. The effect of the chimeric PD1-0103 antibody and its humanized versions were compared to the reference anti-PDl antibodies comprising the VH and VL domains of either nivolumab (also known as MDX5C4 or MDX-1106) and pembrolizumab (also known as MK-3475 or Org 1.09A) with backbones of human IgG1 (with mutations L234A, L235A and P329G (EU index of Kabat)). After 6 days of MLR culture 50 μl of supernatant was taken and multiple cytokines were measured in a single culture using Bio-Plex Pro™ Human Cytokine Th1/Th2 Assay (Bio-Rad Laboratories Inc.). (data not shown for all cytokines).

The chimeric PD1-0103 antibody and its humanized versions (PD1-0103_0312 and PD1-0103_0314) were more potent compared to the reference anti-PDl antibodies in enhancing the T cell activation and IFN-gamma secretion (see Figure 2).

Further the chimeric PD1-0103 antibody and its humanization variants increase tumor necrosis factor alpha (TNF alpha) (see Figure 3) and IL-12 (data not shown) secretion by antigen presenting cells and encance capacity of monocytes /macrophages or antigen presenting cells to stimulate a T cell.

**Example 4:**

**Effect of anti-PD-1 blockade on cytotoxic Granzyme B release and IFN-γ secretion by human CD4 T cells cocultured with allogeneic mature dendritic cells**

To further investigate the effect of anti-PD-1 treatment in an allogeneic setting we developed an assay in which freshly purified CD4 T cells are cocultured for 5 days in presence of monocyte-derived allogeneic mature dendritic cells (mDCs). Monocytes were isolated from fresh PBMCs one week before through plastic adherence followed by the removal of the non-adherent cells. We then generated immature DCs from the monocytes by culturing them for 5 days in media containing GM-CSF (50 ng/ml) and IL-4 (100 ng/ml). To induce iDCs maturation, we added TNF-alpha, IL-1beta and IL-6 (50 ng/ml each) to the culturing media for 2 additional days. We then assessed DCs maturation by measuring their surface expression of Major Histocompatibility Complex Class II (MHCII), CD80, CD83 and CD86 thorugh flow cytometry (LSRFortessa, BD Biosciences).

On the day of the minimal mixed lymphocyte reaction (mMLR), CD4 T cells were enriched via a microbead kit (Miltenyi Biotec) from 108 PBMCs obtained from an unrelated donor. Prior culture, CD4 T cells were labeled with 5μM of Carboxy-Fluorescein-Succinimidyl Esther (CFSE). 105 CD4 T cells were then plated in a 96 well plate together with mature allo-DCs (5:1) in presence or absence of blocking anti-PD1 antibody (either PD1-0103, chimeric PD1-0103, or humanized antibodies PD1-0103-0312, PD1-0103-0313, PD1-0103-0314, PD1-0103-0315, abbreviated as 0312, 0313, 0314, 0315 in figures 4A and 4B), at the concentration of 10 μg/ml if not differentely indicated in the figures.

Five days later we collected the cell-culture supernatants, used later to measure the IFN-gamma levels by ELISA (R&D systems), and left the cells at 37 C degrees for additional 5 hours in presence of Golgi Plug (Brefeldin A) and Golgi Stop (Monensin). The cells were then washed, stained on the surface with anti-human CD4 antibody and the Live/Dead fixable dye Aqua (Invitrogen) before being fixed/permeabilized with Fix/Perm Buffer (BD Bioscience). We performed intracellular staining for Granzyme B (BD Bioscience), IFN-gamma and IL-2 (both from eBioscience). Results are shown in Figures 4A and 4B.

We also tested different concentrations of the humanized variants PD1-0103 (humanized antibodies PD1-0103-0312, PD1-0103-0313, PD1-0103-0314, PD1-0103-0315, abbreviated as 0312, 0313, 0314, 0315 in the figures, see also Example 6 below) and found them to be equally good in enhancing granzyme B and interferon gamma. DP47 is a non binding human IgG with a LALA mutation in the Fc portion to avoid recognition by FcgammaR and was used as negative control.

**Example 5:**

**Chimeric antibodies derivatives**

Chimeric PD1 antibodies were generated by amplifying the variable heavy and light chain regions of the anti-PD1 mouse antibodies PD1-0098, PD1-0103 via PCR and cloning them into heavy chain expression vectors as fusion proteins with human IgG1 backbones / human CH1-Hinge-CH2-CH3 with mutations L234A, L235A and P329G (EU index of Kabat)) (Leucine 234 to Alanine, Leucine 235 to Alanine, Proline 329 to Glycine) abrogating effector functions and light chain expression vectors as fusion proteins to human C-kappa. LC and HC Plasmids were then cotransfected into HEK293 and purified after 7 days from supertnatants by standard methods for antibody purification. The chimeric PD1-antibodies were renamed chimeric chiPD1-0098 (chiPD1-0098) and chimeric PD1-0103 (chiPD1-0103). For comparison the reference anti-PD1 antibodies comprising the VH and VL domains of either nivolumab (also known as MDX-5C4 or MDX-1106) and pembrolizumab (also known as MK-3475 or Org 1.09A) were synthesized and cloned with backbones of human IgG1 (with mutations L234A, L235A and P329G (EU index of Kabat)) were used.

**Example 6:**

**Generation, Expression and Purification of humanized variants of anti-PDI antibody PD-0103 (huMab PD-0103) and characterization**

<u>Humanization of the VH and VL domains of murine anti-PD1 antibody 0103</u>

Based upon the amino acid sequence of the murine VH and VL domains of murine anti-PD1 antibody 0103 (SEQ ID NO: 7 and 8), humanized anti- anti-PD1 antibody variants were generated.

The humanized VH-variant is based on the human germline IMGT_hVH_3_23 in combination with the human J-element germline IGHJ5-01 with several mutations. (resulting in SEQ ID NO: 57).

The humanized variants of VL are based on the human germlines IMGT_hVK_4_1, IMGT_hVK_2_30, IMGT_hVK_3_11 and IMGT_hVK_1_39 in combination with the human J-element germline IGKJ1-01. Different muations resulted in humanized variants of SEQ ID NO: 58 to SEQ ID NO: 61.

The humanized amino acid sequences for heavy and light chain variable regions of PD1-0103 were backtranslated in to DNA and the resulting cNDA were synthesized (GenArt) and then cloned into heavy chain expression vectors as fusion proteins with human IgG1 backbones /human CH1-Hinge-CH2-CH3 with LALA and PG mutations (Leucine 234 to Alanine, Leucine 235 to Alanine, Proline 329 to Glycine) abrogating effector functions or into light chain expression vectors as fusion proteins to human C-kappa. LC and HC Plasmids were then cotransfected into HEK293 and purified after 7 days from supernatants by standard methods for antibody purification. The resulting humanized PD1-antibodies named as follows:

**Table 6: VH and VL sequences of humanized variant antibodies of PD1-0103**

| Humanized antibodies of PD1-0103 | humanized variant of VH/SEQ ID NO: | humanized variant of VL/SEQ ID NO: |
|---|---|---|
| PD1-0103-0312 | SEQ ID NO: 57 | SEQ ID NO: 58 |
| PD1-0103-0313 | SEQ ID NO: 57 | SEQ ID NO: 59 |
| PD1-0103-0314 | SEQ ID NO: 57 | SEQ ID NO: 60 |
| PD1-0103-0315 | SEQ ID NO: 57 | SEQ ID NO: 61 |

**Table 7: HVR sequences of humanized variant antibodies of PD1-0103**

| Humanized antibodies of PD1-0103 | HVR-H1, HVR-H2, and HVR-H3 of humanized variant/SEQ ID NO: | HVR-L1, HVR-L2, and HVR-L3 of humanized variant/SEQ ID NO: |
|---|---|---|
| PD-0103-0312 | SEQ ID NOs: 1, 2 and 3 | SEQ ID NOs: 4, 5 and 6 |
| PD-0103-0313 | SEQ ID NOs: 1, 2 and 3 | SEQ ID NOs: 4, 5 and 6 |
| PD-0103-0314 | SEQ ID NOs: 1, 2 and 3 | SEQ ID NOs: 4, 5 and 6 |
| PD-0103-0315 | SEQ ID NOs: 1, 2 and 3 | SEQ ID NOs: 4, 5 and 6 |

Humanized PD1-0103 antibody variants and parental chimeric PD1-0103 were characterized as descibed above. Results are shown in Table 8.

**Table 8: Summary of results for humanized PD1-0103 antibody variants and parental chimeric PD1-0103**

| Assay | chimeric PD1-0103 | PD-0103-0312 | PD-0103-0313 | PD-0103-0314 | PD-0103-0315 |
|---|---|---|---|---|---|
| Affinity $K_D$ 37°C [nM] *) | 2.0/0.8 | 1.5/1.8 | 1.9/2.3 | 1.6/1.5 | 1.7/1.5 |
| ELISA EC50 [nM] | 0,2 | 0,1 | 0,07 | 0,07 | 0,06 |
| CHO-PD1 EC50 | + | + | + | + | + |
| IC50 PD-L1, 2[nM] | 1.35 | tbd | tbd | tbd | tbd |
| Mixed Lymphocyte Reaction assay | +++ | +++ | +++ | ++++ | ++ |
| cynomolgus crossreactivity (EC50 [nm] | + | 0,08 | 0,06 | 0,05 | 0,04 |

**Example 7:**

**Neutralizing potency PD-1 antibodies**

To test the neutralizing potency of inhouse generated PD-1 antibodies in mimicking a restoration of a suppressed T cell response in vitro a commercially available PD1/PD-L1 reporter assay (Promega) was used. This system consists ofPD1+NFAT Jurkat cells and a PD-L1+ CHO counterpart, which also gives the activation signal. In principle, the reporter system is based on three steps: (1) TCR-mediated NFAT activation, (2) inhibition of NFAT signal upon activation by the PD-1/PD-L1 axis and (3) recovery of the NFAT signal by PD-1 blocking antibodies.

Material and Methods

- PD-L1 Medium: PAN Biotech (#P04-03609); FBS (10%) and L-Gln (4mM)
- Assay Medium: RPMI 1640 (#31870; Invitrogen), 25mM HEPES, 2mM L-Gln, FBS (2%)
- Cells used for this assay (both cell types purchased by Promega):

  PD-L1+ CHO cells (batch no. #139147): 2-3x104 cells/96well

  PD-1+ NFAT Jurkat cells (batch no. #133024: 3.5x104 cells/well

On day 1, PD-L1+ cells were thawed, seeded at the indicated cell concentration in the above mentioned medium and cultured over night at 37°C and 5% CO2. On the next day, medium was removed and PD-L1+ cells were incubated with the prepared antibodies at indicated concentrations (in Assay Medium). In parallel, PD-1+ NFAT Jurkat cells were thawed and above mentioned cell numbers were transferred to and co-cultured with the PD-L1+ cells. After an incubation of 6 hrs at 37°C and 5% CO2, Bio-Glo substrate was warmed to room temperature (1-2 hrs prior addition). The cell culture plate was removed from the incubator and adjusted to room temperature (10min) before 80μl Bio-Glo solution was added per well, incubated for 5-10 min before the luminescence was measured at a Tecan Infinite reader according to the kit's manufacturer's recommendation. Results can be seen in the Figures 5A and 5 B where the restoration of a PD-1/PD-L1 mediated suppression of the NFAT signal by different PD-1 antibodies upon TCR stimulation is shown: Figure 5 A: Chimeric PD1_0103 showed a reproducibly superior effect when compared to a reference antibody. As reference an anti-PD1 antibody comprising the VH and VL domains nivolumab (also known as MDX-5C4 or MDX-1106) was synthesized and cloned with backbones of human IgG1 (with mutations L234A, L235A and P329G (EU index of Kabat)). Figure 5B: The four humanized variants of PD1_0103 demonstrated a similar in vitro potency to the lead antibody and were also slightly superior to the reference antibody.

**Example 8:**

**Crystallization of Fab PD1-0103 with PD-1 ectodomain:**

For complex formation Fab PD1-0103 was mixed in a 1.1 molar excess with the PD-1 ectodomain. After incubation on ice for 1 hour the complex was deglycosylated by a PNGase step to remove glycans which are not involved in complex formation. Crystallization screening for complex crystals of Fab fragment PD1-0103 (with human CH1 and CL) with the PD-1 ECD was performed at a concentration of 15mg/ml. Crystallization droplets were set up at 21 °C by mixing 0.1 μl of protein solution with 0.1 μl reservoir solution in vapor diffusion sitting drop experiments. Crystals appeared out of various conditions containing PEG as precipitating agent. Crystals used to determine the structure appeared within 4 days out of 30% PEG1500 and grew to final size of 0.03x0.06x0.02 μm within 7 days.

Crystals were transferred into reservoir solution supplemented with 20% Glycerol as cryoprotectant and then flash-cooled in liquid $N_2$. Diffraction images were collected with a Pilatus 6M detector at a temperature of 100K at the beam line X10SA of the Swiss Light Source and processed with the XDS package *[Kabsch, W. Automatic processing of rotation diffraction data from crystals of initially unknown symmetry and cell constants. J. Appl. Cryst. 26, 795-800 (1993)].* Data from one crystal were merged to yield a 1.9 Å resolution data set in space group P1 with two complex molecules per crystallographic asymmetric unit (see Table 1).

The structure was determined by molecular replacement using the coordinates of a Fab fragment from PDB-ID 3UTZ as search model. As search coordinates for the PD-1 ECD the PDB-ID 3RRQ was used. The Fab was split into constant and variable domains and with both separate searches in the CCP4 program PHASER CCP4 were performed *[CCP4 (*Collaborative Computational Project, N. The CCP4 suite: programs for protein crystallography. Acta Crystallogr. D, 760-763 (1994*)]* in order to account for possible changes in the elbow angle. The model was rebuilt in COOT *(*Emsley, P., Lohkamp, B., Scott, WG. & Cowtan, K. Features and development of COOT. Acta Crystallogr. D Biol. Crystallogr. 60, 486-501 (2010*))* and refined with the CCP4 program REFMAC. The final refinement steps were performed with the program BUSTER *(*Bricogne G., Blanc E., Brandl M., Flensburg C., Keller P., Paciorek W.,Roversi P, Sharff A., Smart O.S., Vonrhein C., Womack T.O. (2016). BUSTER version 2.11.6. Cambridge, United Kingdom: Global Phasing Ltd.*).*

**Table 9: Data collection and structure refinement statistics for Fab PD1-0103-PD-1 ECD crystal**

| **Data Collection** | |
|---|---|
| Wavelength (Å) | 1.0 |
| Resolution[1] (Å) | 48.27 - 1.90 (1.99 - 1.90) |
| Space group | P1 |
| Unit cell (Å, °) | 66.37 69.82 86.09 99.17 98.01 119.40 |
| Total reflections | 170515 (20750) |
| Unique reflections | 97997 (12250) |
| Multiplicity | 1.72 (1.66) |
| Completeness (%) | 0.97 (0.96) |
| Mean $I/\sigma(I)$ | 8.02 (0.86) |
| Wilson B-factor | 30.30 |
| R-meas | 0.093 (0.610) |
| CC1/2 | 0.999 (0.290) |
| **Refinement** | |
| Reflections used in refinement | 97986 (6792) |
| Reflections used for R-free | 4754 (355) |
| R-work [3] | 0.1899 (0.2290) |
| R-free [4] | 0.2291 (0.2628) |
| Number of non-hydrogen atoms | 9235 |
| macromolecules | 8199 |
| Carbohydrate | 162 |
| Protein residues | 1068 |
| RMS bonds (Å) | 0.013 |
| RMS angles (°) | 1.81 |
| Ramachandran favored (%) | 97 |
| Ramachandran allowed (%) | 2.9 |
| Ramachandran outliers (%) | 0.38 |
| Rotamer outliers (%) | 2.1 |
| Clashscore | 2.60 |
| Average B-factor ($Å^2$) | 36.98 |
| macromolecules | 36.01 |
| Carbohydrate | 49.62 |

(continued)

| Refinement | |
|---|---|
| solvent | 38.12 |
| [1] Values in parentheses refer to the highest resolution bins.<br>[2] $R_{merge}=\sum|I-<I>|/\sum I$ where I is intensity.<br>[3] $R_{work}=\sum|F_o-<F_c>|/\sum F_o$ where $F_o$ is the observed and $F_c$ is the calculated structure factor amplitude.<br>[4] $R_{free}$ was calculated based on 5% of the total data omitted during refinement. | |

**Structure determination of Fab PD1-0103 in complex with the PD-1 ectodomain**

In order to characterize the epitope and paratope in detail we determined the crystal structure of the PD-1 ectodomain in complex with Fab PD1-0103 to a resolution of 1.9Å. The structure reveals Fab PD1-0103 to recognize an epitope formed by the BC and FG loop regions and by residues of β-strands CC'FG of the front β-sheet of the PD-1 V-type Ig domain. In addition the epitope includes the N-linked glycosylation tree at the position Asn58 which is part of the BC loop of PD-1. All CDRs except CDR2 of the light chain of Fab PD1-0103 contribute to the paratope.

A surface area of 1063Å$^2$ of PD-1 is covered by Fab PD1-0103 with 743 Å$^2$ contributed by the heavy chain and 320 Å$^2$ by the light chain. Analysis of the binding interface with the program PISA reveals an interaction pattern of Fab PD1-0103 with the PD-1 ECD via 6 hydrogen bonds and Van der Waals forces. Side chain hydrogen bonds are formed between residues of heavy chain CDR1 (Thr33) and CDR2 (Ser52, Arg56, Asp57) with Glu61 and Ser62 of the BC loop of PD-1. Van der Waals contacts are mainly driven by CDR3 of the light and heavy chain, in particular Phe105 of HCDR3, and by Tyr32 of HCDR1 which are in close distance to residues Val64 of the BC loop, Pro83 and to Ile126 and Leu128 of the FG loop. Further Van der Waals contacts are observed between FG loop residues Pro130, Ala132, Ile134 with the CDR2 of heavy chain and CDR3 of the light chain of Fab PD1-0103. The light chain of Fab PD1-0103 exclusively contacts the FG loop of PD-1. No contacts are provided by the CDR2 of the light chain for formation of the complex.

The N-linked glycosylation tree at position Asn58 of PD-1 is part of the epitope and interacts solely with residues of the heavy chain of Fab PD1-0103.

The core sugar chain (N-linked glycosylation) tree at position Asn58 of PD-1 has the following structure with respect to the monoscharides

Asn58-N-GlcNAc(FUC) - GlcNAc- - BMA - MAN (see Figure 9) wherein the following abbreviations are used.

[GlcNAc]= NGA = N-acetyl-beta-D-galactosamine = 2-(acetylamino)-2-deoxy-beta-D-galactopyranose

[FUC] = alpha-L-fucose

[BMA] = beta-D-mannopyranose

[MAN] = alpha-D-mannopyranose

The first GlcNAC in the sugar chain is fucosylated which abbreviated as GlcNAc(FUC).

In the structure the core glycans are well defined in the electron density except one mannose unit. The fucose moiety points into a hydrophilic pocket formed by PD-1 with CDR1 and CDR2. Binding of the fucose is coordinated by a hydrogen bonding network with Ser30 and Ser31 of CDR1 together with Glu61 and Gln99 of PD-1. Further contacts are provided by hydrogen bonding of the first GlcNac to Arg56 and framework residues Arg72, Asp73, Asn74 to Man.

**Table 10: List of contacts PD1 - Fab PD1-0103 Heavy chain**

| Contacts identified by distance cutoff of 5Å | |
|---|---|
| **PD1** | **HC of PD-103** |
| Ser60 | Asp57, Tyr59 |
| Glu61 | Thr33, Ser52, Gly53, Gly54, Arg56, Asp57 |
| Ser62 | Thr33, Ser52, Asp57, Phe105 |
| Phe63 | Phe105 |
| Val64 | Gly101, Arg102, Phe105 |

(continued)

| Contacts identified by distance cutoff of 5Å | |
|---|---|
| **PD1** | **HC of PD-103** |
| Tyr68 | Tyr104 |
| Lys78 | Arg 102 |
| Phe82 | Ser31 |
| Pro83 | Ser31, Tyr32 |
| Glu84 | Tyr32 |
| Ile126 | Gly101, Tyr104, Phe105 |
| Ser127 | Phe105 |
| Leu128 | Tyr59, Leu99, Phe105 |
| Pro130 | Tyr59 |
| Ile134 | Tyr104 |

**Table 11: List of contacts PD1 - Fab PD1-0103 light chain**

| Contacts identified by distance cutoff of 5Å | |
|---|---|
| | **of PD-103** |
| Ile126 | Phe36 |
| Leu128 | Asn95, Trp100 |
| Pro130 | Asn95, Tyr96, Asp97, Val98 |
| Lys131 | Tyr96, Asp97 |
| Ala132 | Asn95, Tyr96, Asp97, Thr31, Phe36 |
| Gln133 | Thr31 |
| Ile134 | Thr31, Ser32, ASn34, Phe36 |

**Table 12: List of contacts PD1 of core sugar chain at Asn58- Fab PD1-0103 Heavy chain**

| Contacts identified by distance cutoff of 5Å | |
|---|---|
| **PD1 - N-Glycosylation at Asn58 (core sugar chain)** | **HC of PD-103** |
| First GlcNAc | Arg56, Asp57 |
| FUC | Ser30, Ser31, Tyr32, Gly53, Gly54, |
| Second GlcNAc | Gly54, Gly55, Arg56 |
| BMA | Gly54, Asn74 |
| **PD1 - N-Glycosylation at Asn58 (core sugar chain)** | **HC of PD-103** |
| MAN | Gly53, Gly54, Gly55, Arg72, Asp73, Asn74 |

Summary

- Epitope on PD1 resembles flat surface
  -> Binding mainly by front b-sheet and CDR3 of PD1

- Interactions involve polar and van der Waal contacts
- Large interaction surface area of PD1 with heavy chain **of Fab**
- Glycosylation at position Asn 58 participates in binding of PD1 to Fab fragment
- Fucose unit occupies pocket formed by PD1 and heavy chain of Fab PD1-0103

**Example 9:**

**Reduced antibody binding to human PD1 which is not glycosylated at Asn58 compared to the binding to human PD1 which is glycosylated at Asn58 (Biacore characzterization of anti-PD-1 antibodies to glycosylated and non-glycosylated recombinant PD1)**

A surface plasmon resonance (SPR) based assay has been used to determine the kinetic parameters of the binding between glycosylated PD1 and non-glycosylated recombinant human PD1. Therefore, an anti-human IgG was immobilized by amine coupling to the surface of a (Biacore) CM5 sensor chip. The samples were then captured and hu PD1-ECD was bound to them. The sensor chip surface was regenerated after each analysis cycle. The equilibrium constant and kinetic rate constants were finally gained by fitting the data to a 1:1 langmuir interaction model.

About 2000 response units (RU) of 20 μg/ml anti-human IgG (GE Healthcare #BR-1008-39) were coupled onto the flow cells 1 and 2 (alternatively: 3 and 4) of a CM5 sensor chip in a Biacore T200 at pH 5.0 by using an amine coupling kit supplied by GE Healthcare.

The sample and running buffer was HBS-EP+ (0.01 M HEPES, 0.15 M NaCl, 3 mM EDTA, 0.05 % v/v Surfactant P20, pH 7.4). Flow cell temperature was set to 25 °C and sample compartment temperature to 12 °C. The system was primed with running buffer.

The samples were injected for 20 seconds with a concentration of 10 nM and bound to the second flow cell. Then a complete set of human PD1-ECD glycosylated or non-glycosylated) concentrations (200 nM, 66.6 nM, 22.2 nM, 7.4 nM, 2.46 nM and 0 nM) was injected over each sample for 200s followed by a dissociation time of 0/2000s(66.6 nM & 22.2nM) and two 20s regeneration steps with 3 M $MgCl_2$, of which the last one contained an "extra wash after injection" with running buffer.

Finally the double referenced data was fitted to a 1:1 langmuir interaction model with the Biacore T200 Evaluation Software. Resulting $K_D$, $k_a$ and $k_d$ values are shown in Table 13.

**Table 13:** Kinetic rate constants and equilibrium constant determined by Biacore.

| Ligand | Sample | ka (1/Ms) | kd (1/s) | KD (M) |
|---|---|---|---|---|
| PD1-0103-0312 | PD1 aglycosylated at Asn58 | 3.36E+05 | 2.70E-02 | 8.02E-08 |
| PD1-0103-0312 | PD1 glycosylated at Asn58 | 7.77E+05 | 7.46E-05 | 9.61E-11 |
| pembrolizumab | PD1 aglycosylated at Asn58 | 1.51E+06 | 2.46E-03 | 1.63E-09 |
| pembrolizumab | PD1 glycosylated at Asn58 | 1.87E+06 | 4.50E-03 | 2.41E-09 |
| nivolumab | PD1 aglycosylated at Asn58 | 5.49E+05 | 3.66E-03 | 6.66E-09 |
| nivolumab | PD1 glycosylated at Asn58 | 4.44E+05 | 1.63E-03 | 3.68E-09 |

There is a clear differentiation between the binding of PD-103-0312 to aglycosylated and glycosylated PD-1 in contrast to pembrolizumab and nuvolumab (see also Figures 13A and 13B).

**Example 10:**

*In vivo* **anti-tumor efficacy of PD1 antibodies in combination with a T cell bispecific antibody against CEA**

Humanized animal were produced by conditioning NOG mice with subsequent adoptively transfer of human hematopoietic stem cells. The resulting mice display a chimeric ratio between human and mouse leukocytes ranging from 20 to 85% of human derived cells. In such model , T cells are functional and can be activated to kill tumor cells by the bispecofci antibody which binds to CEA and CD3 (which is described in WO2014/131712). Such humanized animals were then injected with one million CEA positive tumor cells, MKN45 gastric carcinoma, subcutaneously in the lateral location. Tumor growth could be assessed by measuring the 3 dimensional axis of the tumor by a operator directed caliper, 3 times a week (Figure 14A and B). At day 9 after tumor injection, the mice were randomized based of tumor size to have homogenous animal groups and the therapeutic treatment started. With the exception of the vehicle groups (figure xA and XB, circles), all the mouse groups were administered intravenously with CEACD3TCB at a dose of 2.5mh/Kg twice a week. In addition each mouse group was also treated with one combination partner: anti-PD1 (PD1-0103-0312) at either 0.15mg/Kg weekly (Figure 14A, squares) or 1.5mg/Kg (Figure 14B, squares) weekly intraperitoneally; Nivolumab at either 0.15mg/Kg weekly (Figure 14A, diamonds) or 1.5mg/Kg (Figure 14B, diamonds) weekly intraperitoneally. The mean of tumor size within one treatment group is displayed over time. The group were composed of 9-10 mice each and the measurement continue until there were at least 3 mice per group. The standardised Aerea under the curve (sAUC) has been calculated and the one way ANOVA analysis was use to calculate statistical significance.

## Claims

**1.** An isolated antibody that binds to human PD1, wherein the antibody

i) comprises a VH sequence of SEQ ID NO:57 and a VL sequence of SEQ ID NO:58, or
ii) comprises a VH sequence of SEQ ID NO:57 and a VL sequence of SEQ ID NO:59, or
iii) comprises a VH sequence of SEQ ID NO:57 and a VL sequence of SEQ ID NO:60, or
iv) comprises a VH sequence of SEQ ID NO:57 and a VL sequence of SEQ ID NO:61.

**2.** An isolated antibody that binds to human PD1, wherein the antibody comprises a VH sequence of SEQ ID NO:57 and a VL sequence of SEQ ID NO:58.

**3.** An isolated antibody that binds to human PD 1, wherein the antibody comprises a VH sequence of SEQ ID NO:57 and a VL sequence of SEQ ID NO:59.

**4.** An isolated antibody that binds to human PD1, wherein the antibody comprises a VH sequence of SEQ ID NO:57 and a VL sequence of SEQ ID NO:60.

5. An isolated antibody that binds to human PD1, wherein the antibody comprises a VH sequence of SEQ ID NO:57 and a VL sequence of SEQ ID NO:61.

6. The antibody of according to any one of the preceding claims, which is a full length IgG1 antibody with mutations L234A, L235A and P329G according to the EU index numbering.

7. Isolated nucleic acid encoding the antibody according to any one of the preceding claims.

8. A host cell comprising the nucleic acid of claim 7.

9. A method of producing an antibody comprising culturing the host cell of claim 8 so that the antibody is produced.

10. The method of claim 9, further comprising recovering the antibody from the host cell.

11. A pharmaceutical formulation comprising the antibody according any one of claims 1 to 6 and a pharmaceutically acceptable carrier.

12. The antibody according any one of claims 1 to 6 for use as a medicament.

13. The antibody according any one of claims 1 to 6 for use in treating cancer.

## Patentansprüche

1. Isolierter Antikörper, der an humanes PD1 bindet, wobei der Antikörper

i) eine VH-Sequenz von SEQ ID NO:57 und eine VL-Sequenz von SEQ ID NO:58 umfasst oder
ii) eine VH-Sequenz von SEQ ID NO:57 und eine VL-Sequenz von SEQ ID NO:59 umfasst oder
iii) eine VH-Sequenz von SEQ ID NO:57 und eine VL-Sequenz von SEQ ID NO:60 umfasst oder
iv) eine VH-Sequenz von SEQ ID NO:57 und eine VL-Sequenz von SEQ ID NO:61 umfasst.

2. Isolierter Antikörper, der an humanes PD1 bindet, wobei der Antikörper eine VH-Sequenz von SEQ ID NO:57 und eine VL-Sequenz von SEQ ID NO:58 umfasst.

3. Isolierter Antikörper, der an humanes PD1 bindet, wobei der Antikörper eine VH-Sequenz von SEQ ID NO:57 und eine VL-Sequenz von SEQ ID NO:59 umfasst.

4. Isolierter Antikörper, der an humanes PD1 bindet, wobei der Antikörper eine VH-Sequenz von SEQ ID NO:57 und eine VL-Sequenz von SEQ ID NO:60 umfasst.

5. Isolierter Antikörper, der an humanes PD1 bindet, wobei der Antikörper eine VH-Sequenz von SEQ ID NO:57 und eine VL-Sequenz von SEQ ID NO:61 umfasst.

6. Antikörper nach einem der vorstehenden Ansprüche, der ein IgG1-Antikörper voller Länge mit den Mutationen L234A, L235A und P329G nach der EU-Index-Nummerierung ist.

7. Isolierte Nukleinsäure, die für den Antikörper nach einem der vorstehenden Ansprüche kodiert.

8. Wirtszelle, umfassend die Nukleinsäure nach Anspruch 7.

9. Verfahren zum Produzieren eines Antikörpers, umfassend das Kultivieren der Wirtszelle nach Anspruch 8, so dass der Antikörper produziert wird.

10. Verfahren nach Anspruch 9, ferner umfassend das Gewinnen des Antikörpers aus der Wirtszelle.

11. Pharmazeutische Formulierung, umfassend den Antikörper nach einem der Ansprüche 1 bis 6 und einen pharmazeutisch unbedenklichen Träger.

**12.** Antikörper nach einem der Ansprüche 1 bis 6 zur Verwendung als ein Medikament.

**13.** Antikörper nach einem der Ansprüche 1 bis 6 zur Verwendung bei der Behandlung von Krebs.

**Revendications**

**1.** Anticorps isolé qui se lie à la PD1 humaine, dans lequel l'anticorps

i) comprend une séquence VH de SEQ ID NO : 57 et une séquence VL de SEQ ID NO : 58, ou
ii) comprend une séquence VH de SEQ ID NO : 57 et une séquence VL de SEQ ID NO : 59, ou
iii) comprend une séquence VH de SEQ ID NO : 57 et une séquence VL de SEQ ID NO : 60, ou
iv) comprend une séquence VH de SEQ ID NO : 57 et une séquence VL de SEQ ID NO : 61.

**2.** Anticorps isolé qui se lie à la PD1 humaine, dans lequel l'anticorps comprend une séquence VH de SEQ ID NO : 57 et une séquence VL de SEQ ID NO : 58.

**3.** Anticorps isolé qui se lie à la PD1 humaine, dans lequel l'anticorps comprend une séquence VH de SEQ ID NO : 57 et une séquence VL de SEQ ID NO : 59.

**4.** Anticorps isolé qui se lie à la PD1 humaine, dans lequel l'anticorps comprend une séquence VH de SEQ ID NO : 57 et une séquence VL de SEQ ID NO : 60.

**5.** Anticorps isolé qui se lie à la PD1 humaine, dans lequel l'anticorps comprend une séquence VH de SEQ ID NO : 57 et une séquence VL de SEQ ID NO : 61.

**6.** Anticorps selon l'une quelconque des revendications précédentes, qui est un anticorps IgG1 pleine longueur avec les mutations L234A, L235A et P329G conformément à la numérotation selon l'indice EU.

**7.** Acide nucléique isolé codant pour l'anticorps selon l'une quelconque des revendications précédentes.

**8.** Cellule hôte comprenant l'acide nucléique selon la revendication 7.

**9.** Procédé de production d'un anticorps comprenant la culture de la cellule hôte selon la revendication 8 de manière à produire l'anticorps.

**10.** Procédé selon la revendication 9, comprenant en outre la récupération de l'anticorps de la cellule hôte.

**11.** Formulation pharmaceutique comprenant l'anticorps selon l'une quelconque des revendications 1 à 6 et un véhicule pharmaceutiquement acceptable.

**12.** Anticorps selon l'une quelconque des revendications 1 à 6 pour une utilisation comme médicament.

**13.** Anticorps selon l'une quelconque des revendications 1 à 6 pour une utilisation dans le traitement d'un cancer.

Figure 1

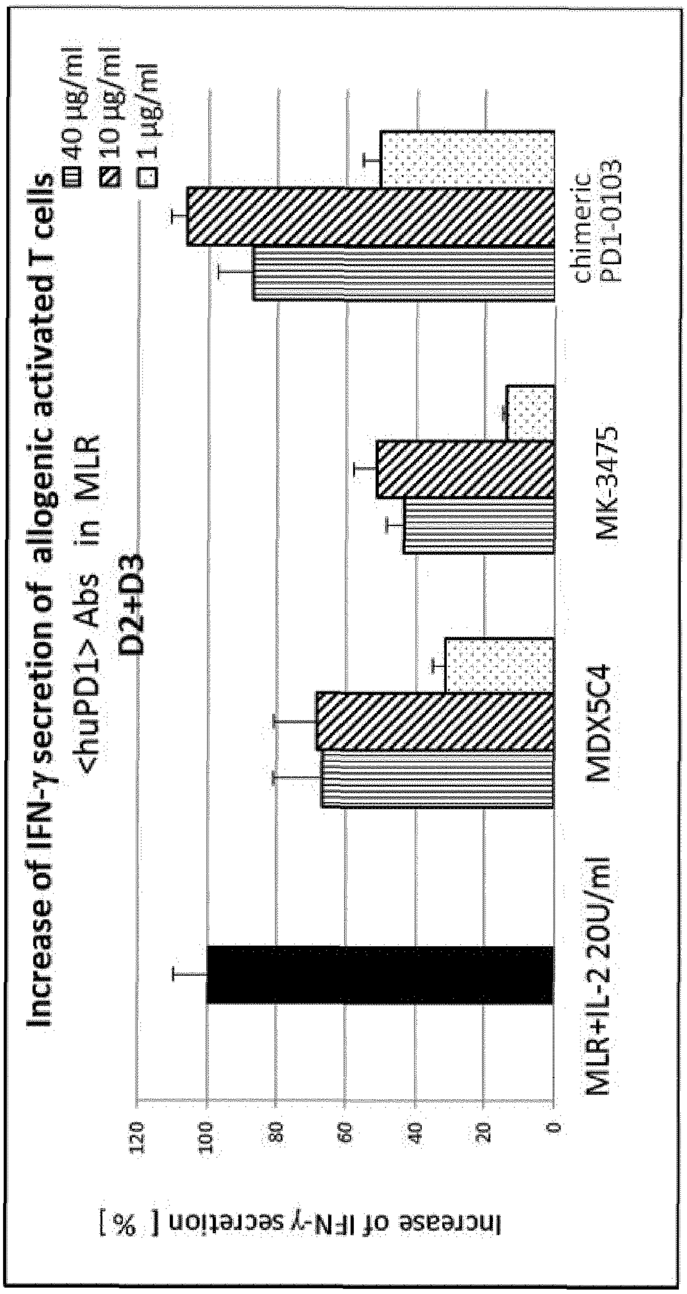
Increase of IFN-γ secretion of allogenic activated T cells
<huPD1> Abs in MLR
D2+D3
40 µg/ml
10 µg/ml
1 µg/ml
Increase of IFN-γ secretion [ % ]
120
100
80
60
40
20
0
MLR+IL-2 20U/ml
MDX5C4
MK-3475
chimeric PD1-0103

Figure 2

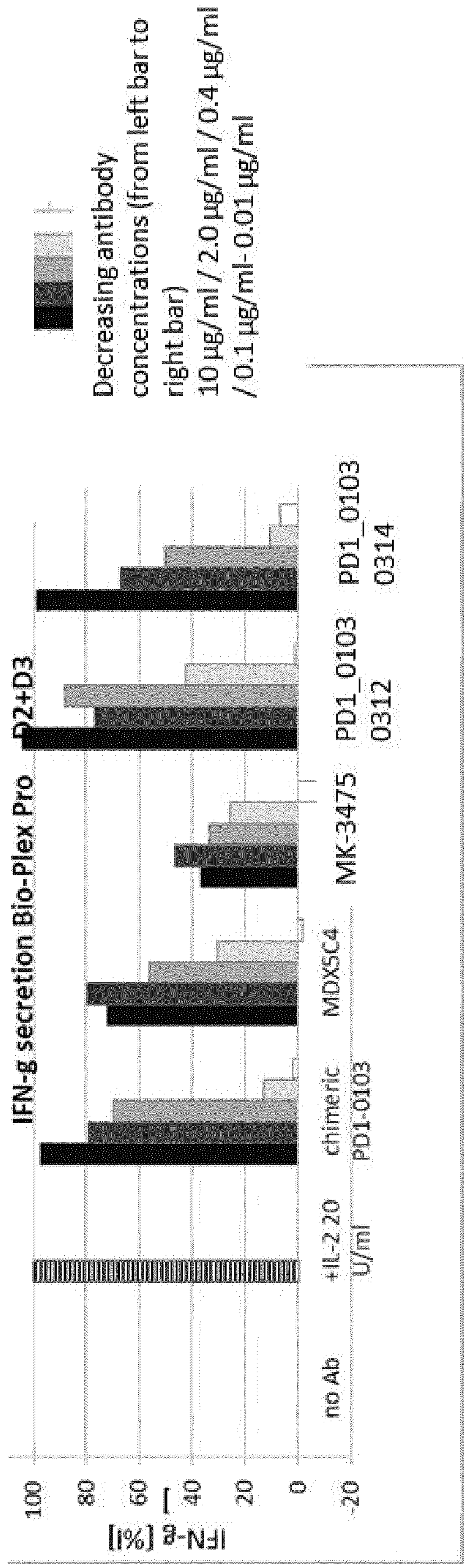
IFN-g secretion Bio-Plex Pro D2+D3
IFN-g [%]
100
80
60
40
20
0
-20
no Ab
+IL-2 20 U/ml
chimeric PD1-0103
MDX5C4
MK-3475
PD1_0103 0312
PD1_0103 0314
Decreasing antibody concentrations (from left bar to right bar)
10 µg/ml / 2.0 µg/ml / 0.4 µg/ml / 0.1 µg/ml-0.01 µg/ml

Figure 3

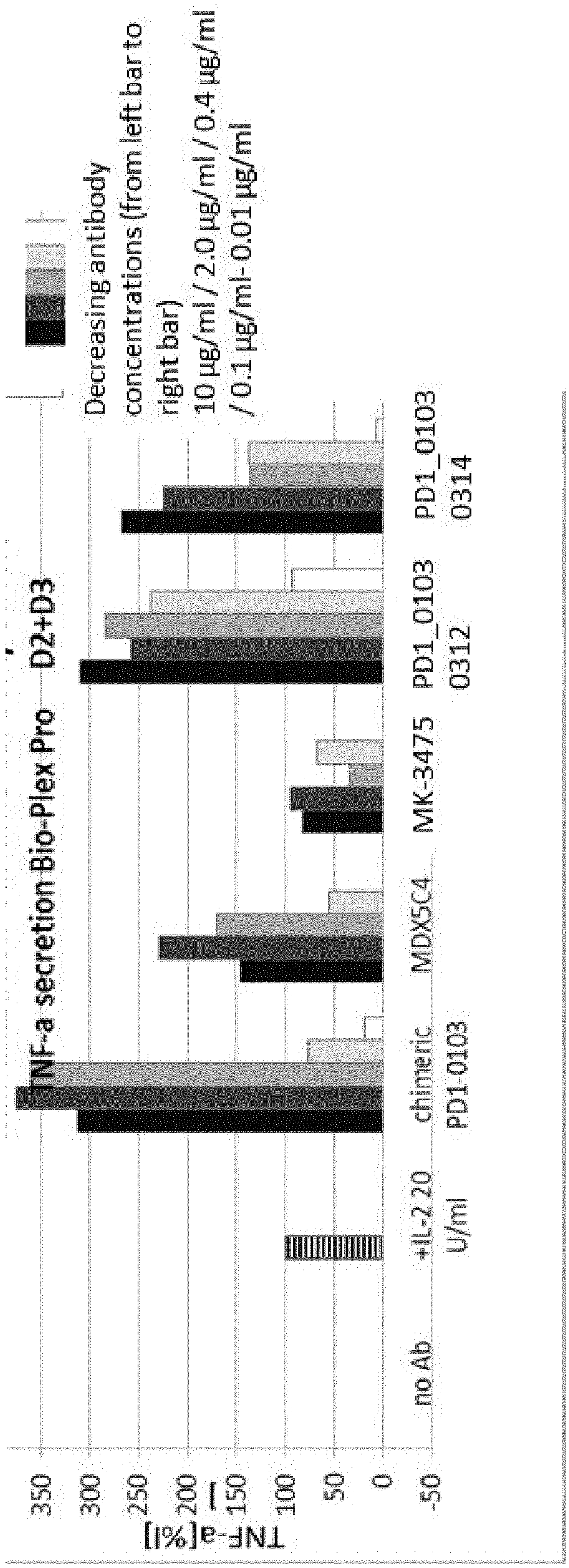
TNF-a secretion Bio-Plex Pro D2+D3
TNF-a[%l]
350
300
250
200
150
100
50
0
-50
no Ab
+IL-2 20 U/ml
chimeric PD1-0103
MDX5C4
MK-3475
PD1_0103 0312
PD1_0103 0314
Decreasing antibody concentrations (from left bar to right bar)
10 µg/ml / 2.0 µg/ml / 0.4 µg/ml / 0.1 µg/ml-0.01 µg/ml

Figure 4A

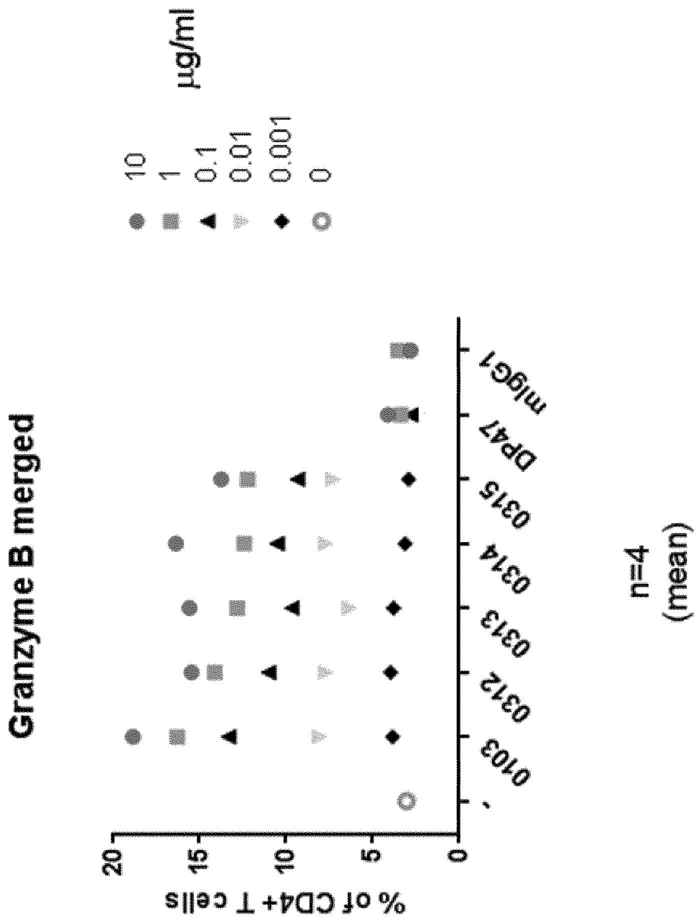
Granzyme B merged
% of CD4+ T cells
20
15
10
5
0
-
0103
0312
0313
0314
0315
DP47
mIgG1
n=4
(mean)
10
1
0.1
0.01
0.001
0
µg/ml

Figure 4B

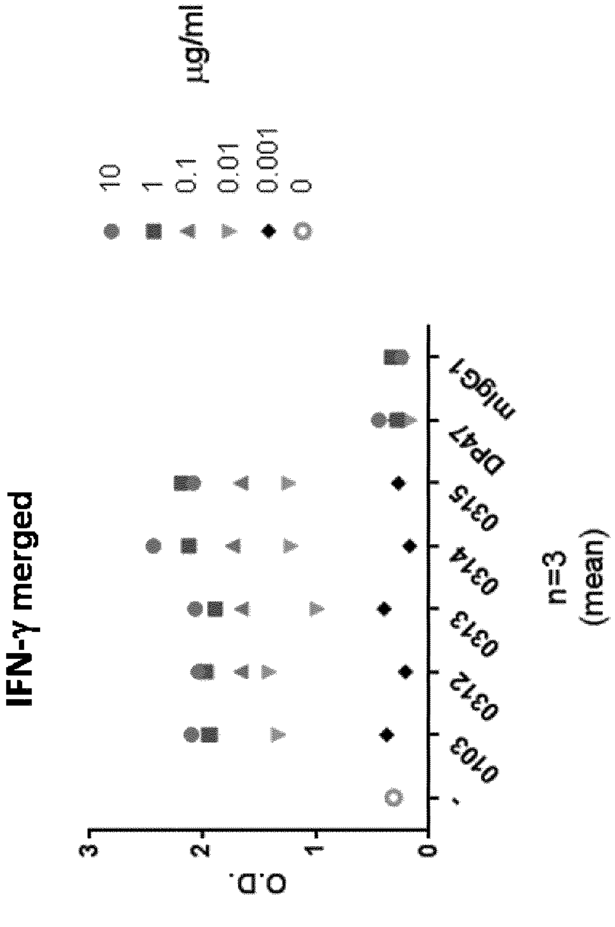
IFN-γ merged
O.D.
3
2
1
0
0103
0312
0313
0314
0315
DP47
mIgG1
n=3
(mean)
10
1
0.1
0.01
0.001
0
μg/ml

# Figure 5A and 5B

5A

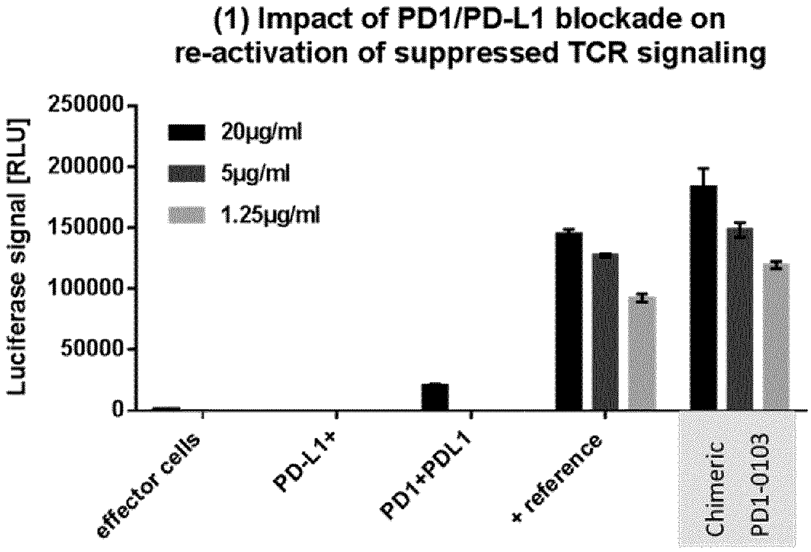


5B

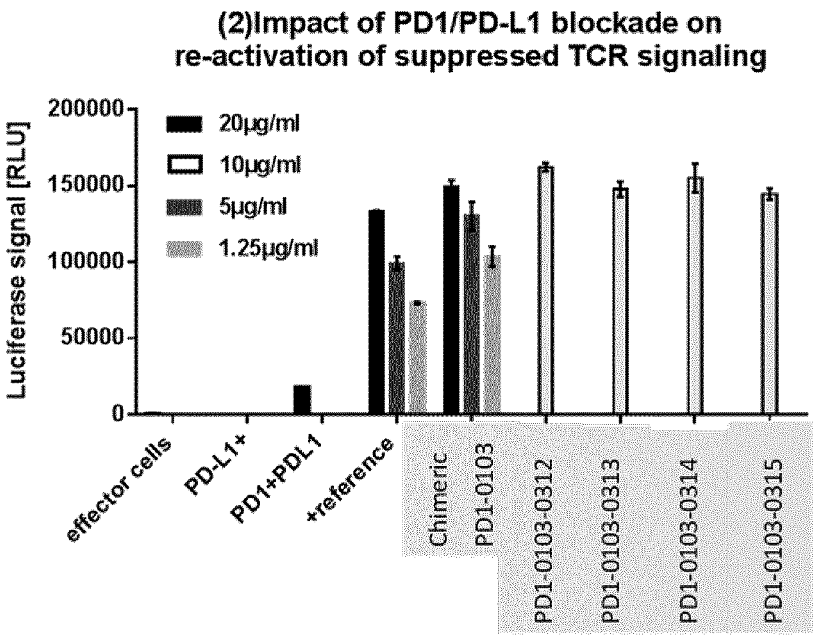

Figure 6

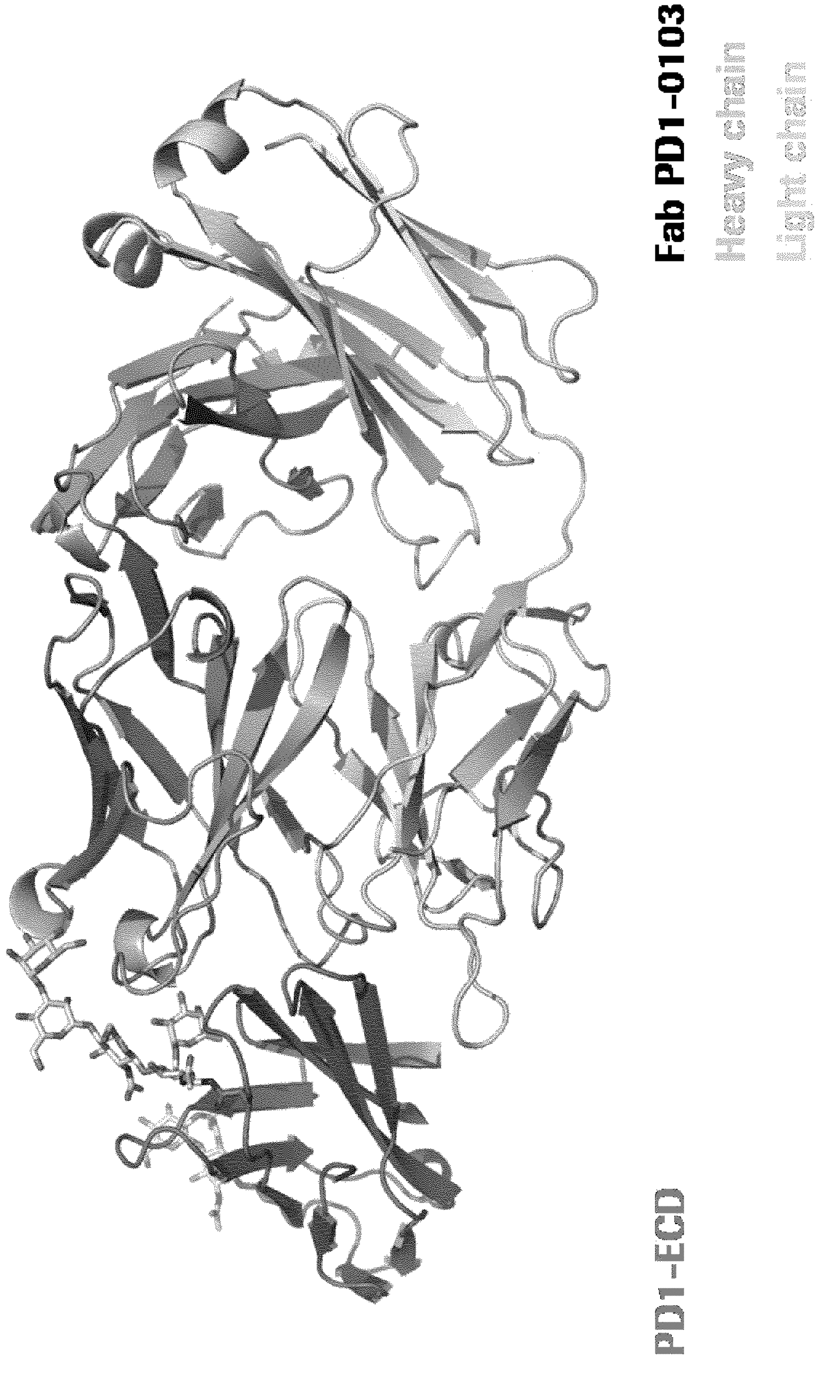

Figure 7

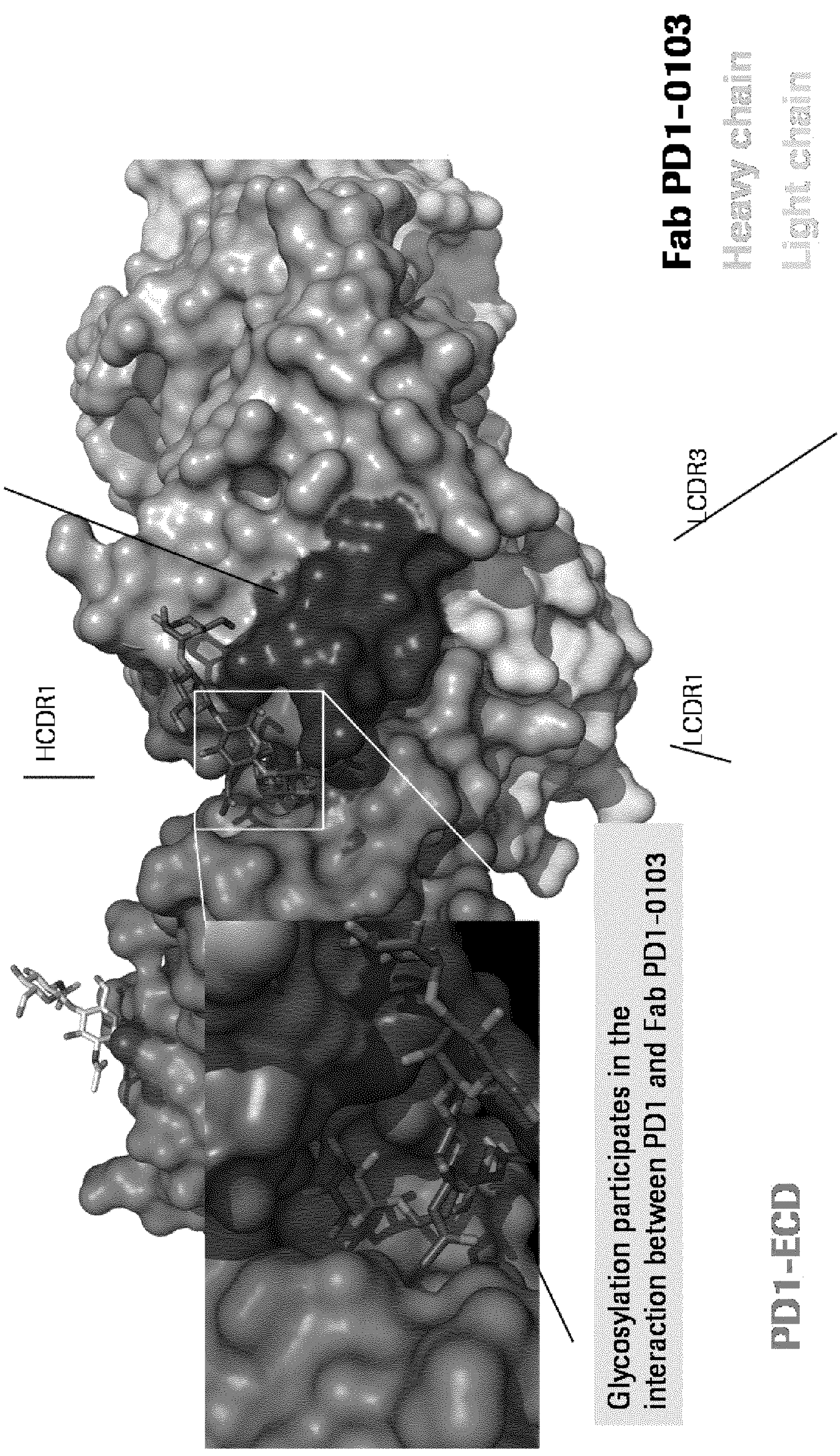
HCDR2
HCDR1
LCDR3
LCDR1
Fab PD1-0103
Heavy chain
Light chain
Glycosylation participates in the interaction between PD1 and Fab PD1-0103
PD1-ECD

# Figure 8

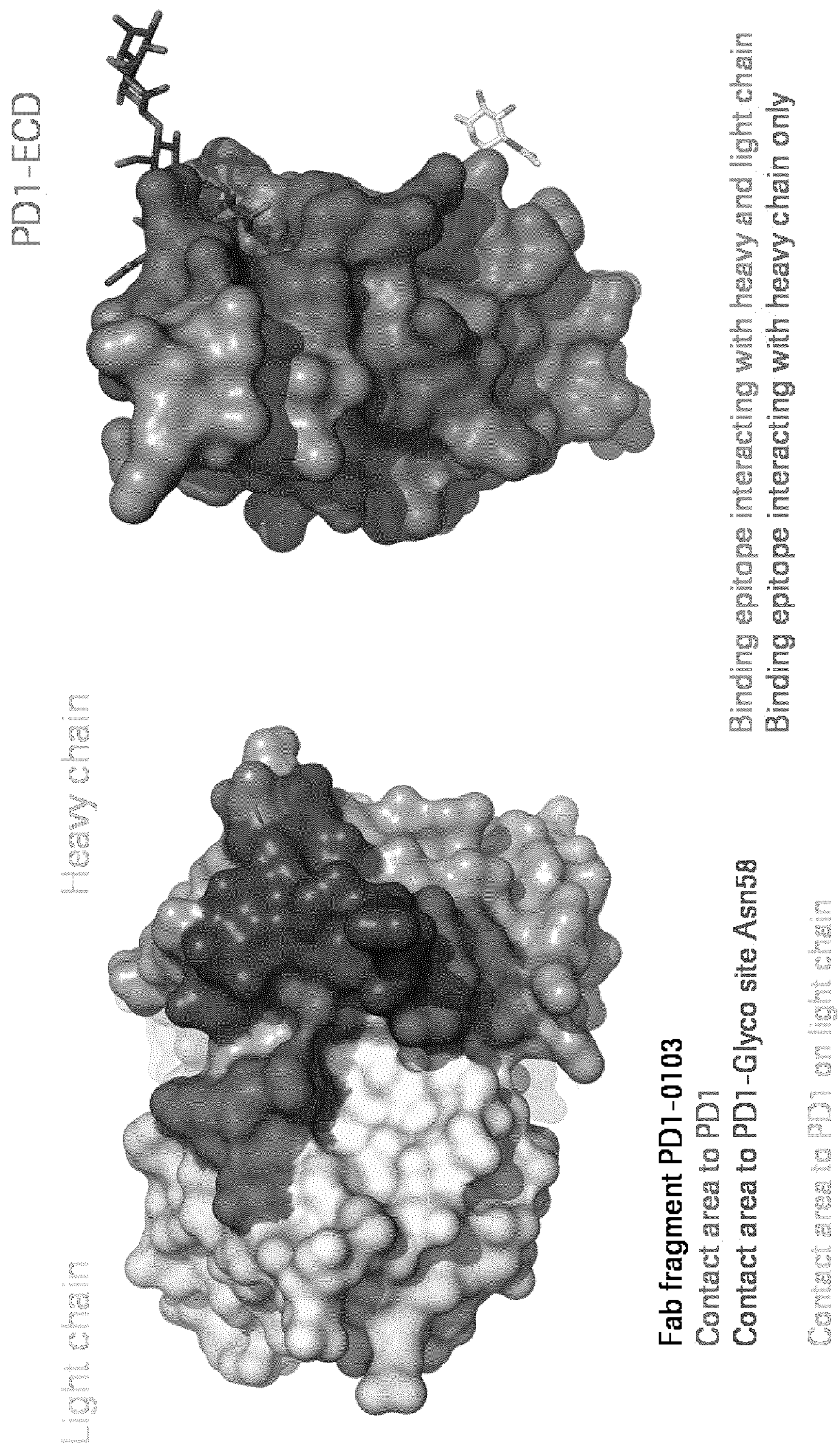
PD1-ECD
Heavy chain
Light chain
Binding epitope interacting with heavy and light chain
Binding epitope interacting with heavy chain only
Fab fragment PD1-0103
Contact area to PD1
Contact area to PD1-Glyco site Asn58
Contact area to PD1 on light chain

# Figure 9

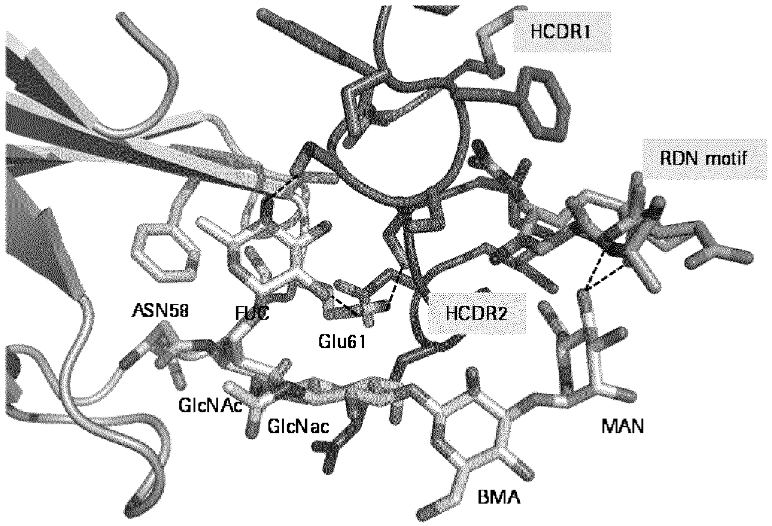


| PD1 – Glycosylation at Asn58 (core sugar chain) | HC |
|---|---|
| First GlcNAc | Arg56, Asp57 |
| FUC | Ser30, Ser31, Tyr32, Gly53, Gly54, |
| Second GlcNAc | Gly54, Gly55, Arg56 |
| BMA | Gly54, Asn74 |
| MAN | Gly53, Gly54, Gly55, Arg72, Asp73, Asn74 |

[GlcNAc]= NGA = N-acetyl-beta-D-galactosamine = 2-(acetylamino)-2-deoxy-beta-D-galactopyranose

[FUC] = alpha-L-fucose

[BMA] = beta-D-mannopyranose

[MAN] = alpha-D-mannopyranose

# Figure 10

- Contacts calculated by PISA
- Grey: PD-1 residues in contact with the antibody

➢ PD1

```
    .    30   .    40   .    50   .    60   .    70
PGWFLDSPDRPWNPPTFSPALLVVTEGDNATFTCSFSNTSESFVLNWYRM
                                        ▲▲ ▲

    .    80   .    90   .   100   .   110   .   120
SPSNQTDKLAAFPEDRSQPGQDCRFRVTQLPNGRDFHMSVVRARRNDSGT
            ▲

    .   130   .   140   .   150   .   160   .   170
YLCGAISLAPKAQIKESLRAELRVTERRAEVPTAHPSPSPRPAGQFQTLV
     ▲ ▲   ▲ ▲
           ■
```

▲ = side chain interaction
■ = main chain interaction

# Figure 11

- Yellow: CDRs
- Red: Antibody residues in contact with the antigen
- Contacts calculated by PISA

➢ PD1-0103 _HC

```
    .   10    .   20    .   30    .   40    .   50
EVILVESGGGLVKPGGSLKLSCAASGFSFSSYTMSWVRQTPEKRLDWVAT
                              ▲▲▲

    .   60    .   70    .   80    .   90    .  100
ISGGGRDIYYPDSVKGRFTISRDNAKNTLYLEMSSLMSEDTALYYCVLLT
 ▲ ■ ■▲ ▲             ★★★
  ★★★★

    .  110    .  120
GRVYFALDSWGQGTSVTVSS
■   ▲
```

★ = interaction with glycosylation

▲ = side chain interaction

■ = main chain interaction

# Figure 12

- Yellow: CDRs
- Red: Antibody residues in contact with the antigen
- Contacts calculated by PISA

➢ PD1-0103 _LC

```
    .   10    .   20    .   30    .   40    .   50
KIVLTQSPASLPVSLGQRATISCRASESVDTSDNSFIHWYQQRPGQSPKL
                                 ▲ ▲

    .   60    .   70    .   80    .   90    .  100
LIYRSSTLESGVPARFSGSGSRTDFTLTIDPVEADDVATYYCQQNYDVPW
                                              ■ ▲ ▲

    .  110
TFGGGTKLEIK
```

▲ = side chain interaction
■ = main chain interaction

# Figure 13A

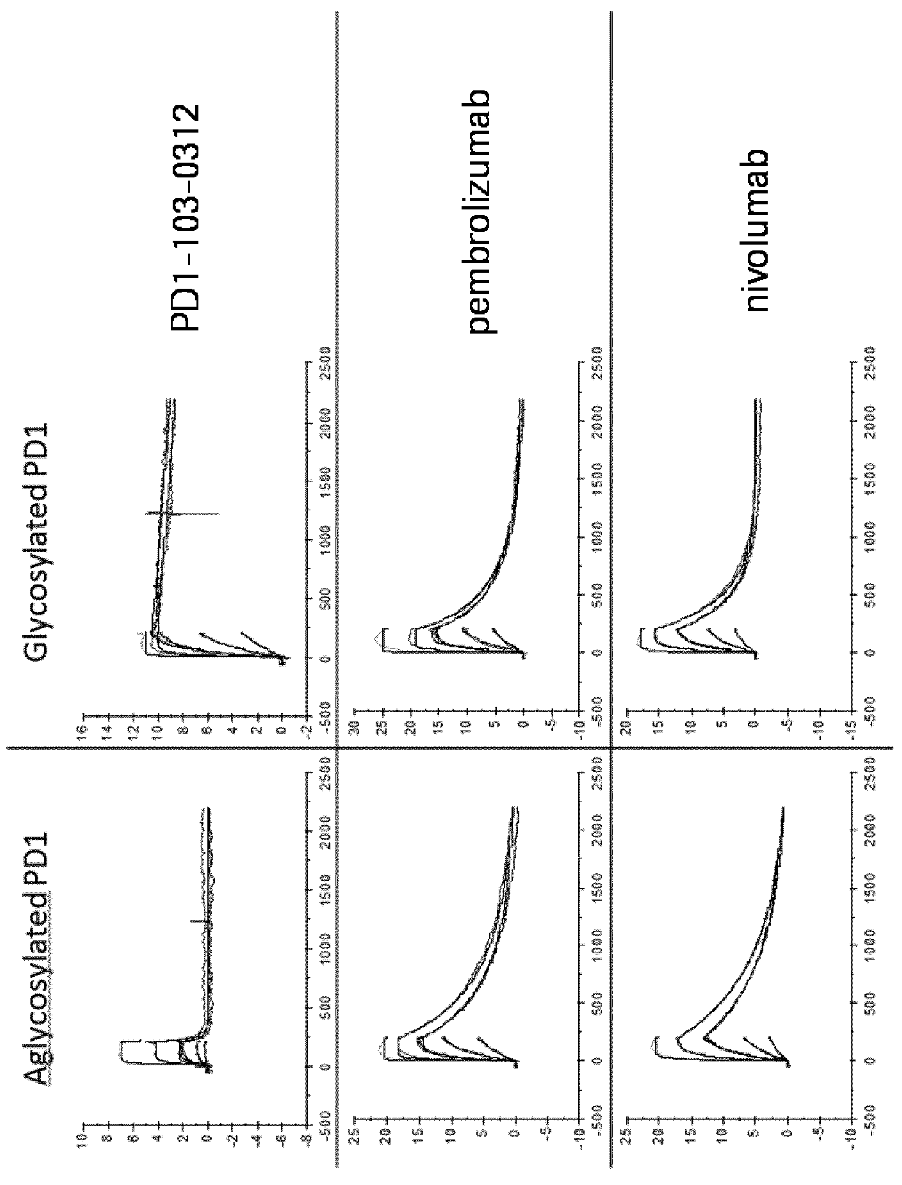
Aglycosylated PD1
Glycosylated PD1
PD1-103-0312
pembrolizumab
nivolumab

Figure 13B

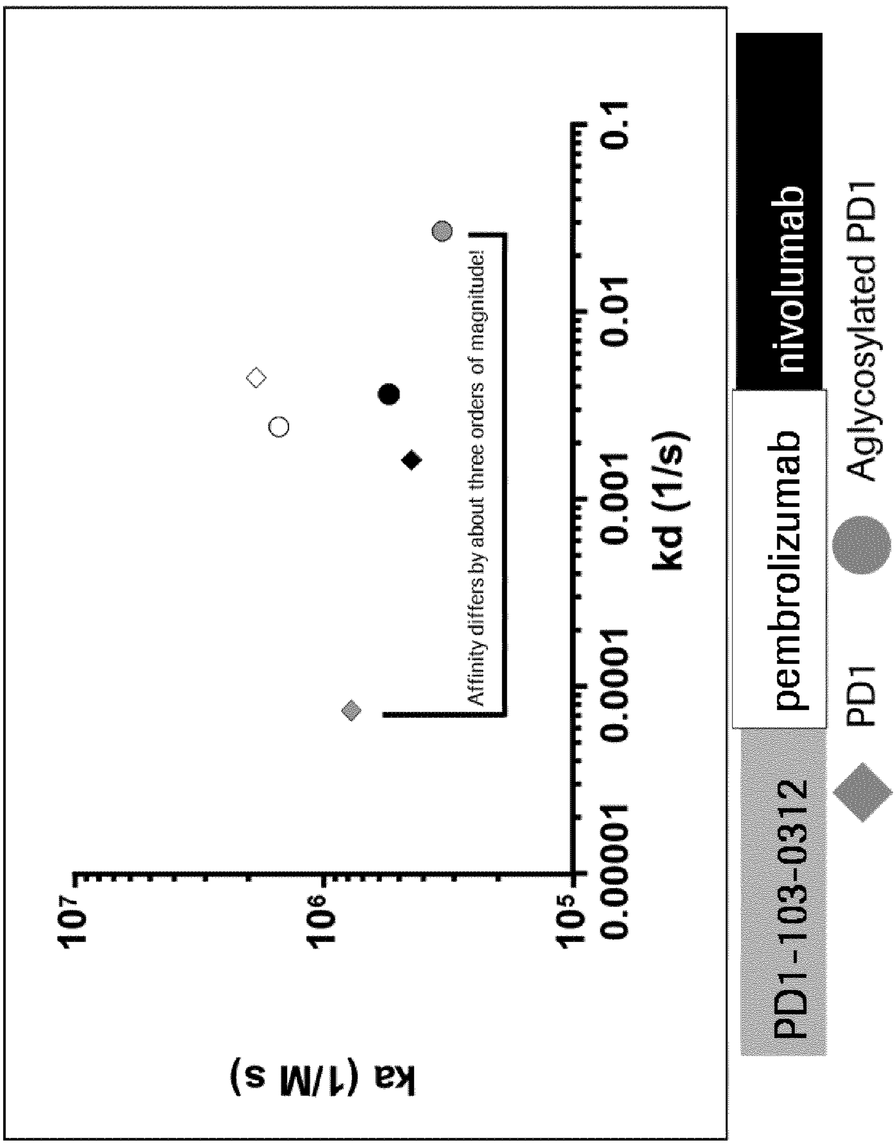
ka (1/M s)
$10^7$
$10^6$
$10^5$
0.00001
0.0001
0.001
0.01
0.1
kd (1/s)
Affinity differs by about three orders of magnitude!
PD1-103-0312
pembrolizumab
nivolumab
PD1
Aglycosylated PD1

Figure 14A

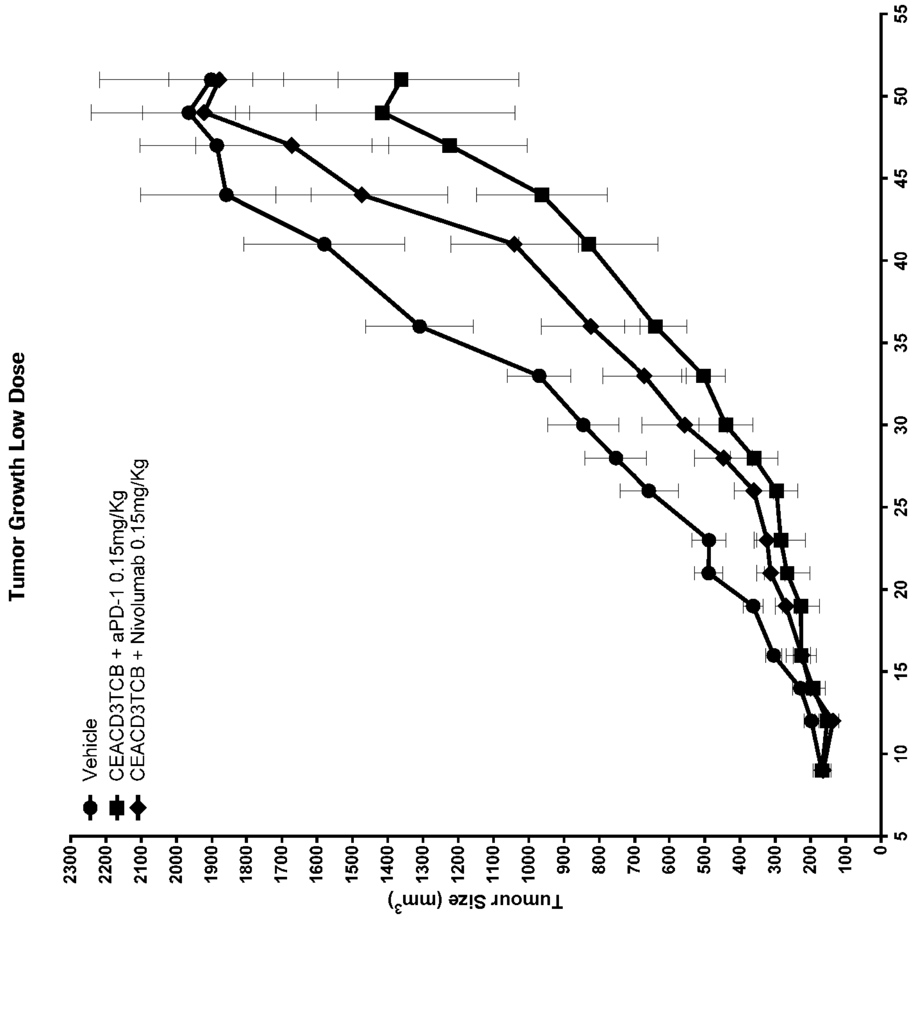
Tumor Growth Low Dose
Vehicle
CEACD3TCB + aPD-1 0.15mg/Kg
CEACD3TCB + Nivolumab 0.15mg/Kg
Tumour Size (mm³)
Days
0
100
200
300
400
500
600
700
800
900
1000
1100
1200
1300
1400
1500
1600
1700
1800
1900
2000
2100
2200
2300
5
10
15
20
25
30
35
40
45
50
55

# Figure 14B

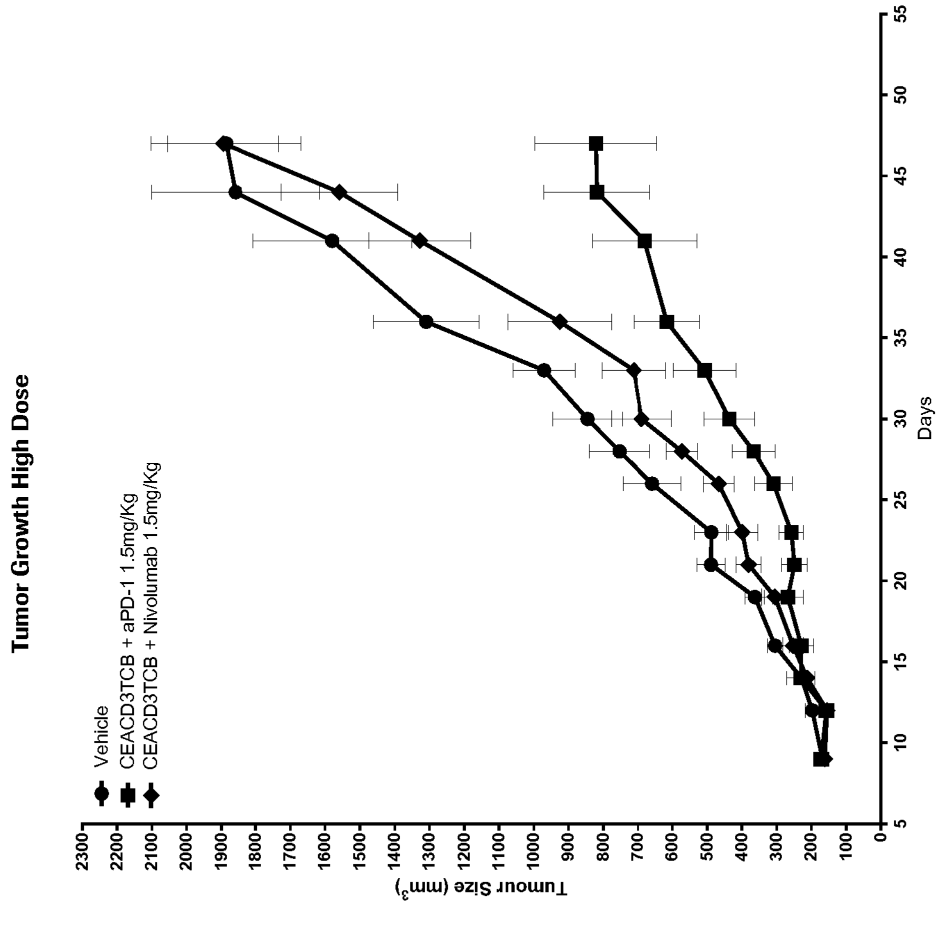
Tumor Growth High Dose
Vehicle
CEACD3TCB + aPD-1 1.5mg/Kg
CEACD3TCB + Nivolumab 1.5mg/Kg
Tumour Size (mm3)
Days
0
100
200
300
400
500
600
700
800
900
1000
1100
1200
1300
1400
1500
1600
1700
1800
1900
2000
2100
2200
2300
5
10
15
20
25
30
35
40
45
50
55

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20030039653 A
- US 20040213795 A
- US 20060110383 A
- US 20070065427 A
- US 20070122378 A
- US 2012237522 A
- WO 2004072286 A
- WO 2006121168 A
- WO 2006133396 A
- WO 2007005874 A
- WO 2008083174 A
- WO 2008156712 A
- WO 2009024531 A
- WO 2009014708 A
- WO 2009114335 A
- WO 2010027828 A
- WO 2010027423 A
- WO 2010036959 A
- WO 2010029435 A
- WO 2010029434 A
- WO 2010063011 A
- WO 2010089411 A
- WO 2011066342 A
- WO 2011110604 A
- WO 2011110621 A
- WO 2012145493 A
- WO 9316185 A
- US 5571894 A
- US 5587458 A
- US 5869046 A
- EP 0404097 A
- WO 199301161 A
- US 6248516 B1
- US 4816567 A
- US 5821337 A
- US 7527791 B
- US 6982321 B
- US 7087409 B
- US 6075181 A
- US 6150584 A
- US 5770429 A
- US 7041870 B
- US 20070061900 A
- US 7189826 B
- US 5750373 A
- US 20050079574 A
- US 20050119455 A
- US 20050266000 A
- US 20070117126 A
- US 20070160598 A
- US 20070237764 A
- US 20070292936 A
- US 20090002360 A
- WO 9308829 A
- US 5731168 A
- WO 2009089004 A
- US 4676980 A
- US 20060025576 A
- US 20080069820 A
- WO 2009080251 A
- WO 2009080252 A
- WO 2009080253 A
- WO 2009080254 A
- WO 2010112193 A
- WO 2010115589 A
- WO 2010136172 A
- WO 2010145792 A
- WO 2010145793 A
- WO 2011117330 A
- WO 2012025525 A
- WO 2012025530 A
- WO 2013026835 A
- WO 2013026831 A
- WO 2013164325 A
- WO 2013174873 A
- US 6737056 B
- US 7332581 B
- WO 2004056312 A
- US 20050014934 A
- US 7371826 B
- US 5648260 A
- US 5624821 A
- WO 9429351 A
- US 7521541 B
- US 5648237 A
- US 5789199 A
- US 5840523 A
- US 5959177 A
- US 6040498 A
- US 6420548 B
- US 7125978 B
- US 6417429 B
- US 5208020 A
- US 5416064 A
- EP 0425235 B1
- US 5635483 A
- US 5780588 A
- US 7498298 B

- US 5712374 A
- US 5714586 A
- US 5739116 A
- US 5767285 A
- US 5770701 A
- US 5770710 A
- US 5773001 A
- US 5877296 A
- US 6630579 B
- WO 9411026 A
- US 4737456 A
- US 20050260186 A
- US 20060104968 A
- US 6267958 B
- US 6171586 B
- WO 2006044908 A
- WO 2014131712 A
- EP 15188061


**Non-patent literature cited in the description**


- **LAFFERTY et al.** *Aust. J. Exp. Biol. Med. Sci.,* 1975, vol. 53, 27-42
- **BRETSCHER et al.** *Science,* 1970, vol. 169, 1042-1049
- **BRETSCHER, P.A.** *P.N.A.S. USA,* 1999, vol. 96, 185-190
- **JENKINS et al.** *J. Exp. Med.,* 1987, vol. 165, 302-319
- **LENSCHOW et al.** *Ann. Rev. Immunol.,* 1996, vol. 14, 233
- **VIOLA et al.** *Science,* 1996, vol. 273, 104-106
- **SLOAN-LANCASTER.** *Nature,* 1993, vol. 363, 156-159
- **OKAZAKI et al.** *Curr. Opin. Immunol.,* 2002, vol. 14, 391779-82
- **BENNETT et al.** *J Immunol,* 2003, vol. 170, 711-8
- **HUTLOFF et al.** *Nature,* 1999, vol. 397, 263-266
- **HANSEN et al.** *Immunogenics,* 1980, vol. 10, 247-260
- **ISHIDA et al.** *EMBO J,* 1992, vol. 11, 3887-95
- **AGATA et al.** *bit Immunol,* 1996, vol. 8, 765-72
- **THOMAS, MX.** J Exp A. 1953-6
- **VIVIER, E ; DAERON, M.** *Immunol Today,* 1997, vol. 18, 286-91
- **FREEMAN et al.** *J Exp Med,* 2000, vol. 192, 1027-34
- **LATCHMAN et al.** *Nat Immunol,* 2001, vol. 2, 261-8
- **CARTER et al.** *Eur J Immunol,* 2002, vol. 32, 634-43
- **DONG et al.** *Nat. Med,* 2002, vol. 8, 787-9
- **DONG et al.** *J. Mol. Med.,* 2003, vol. 81, 281-7
- **BLANK et al.** *Cancer Immunol. Immunother.,* 2005, vol. 54, 307-314
- **KONISHI et al.** *Clin. Cancer Res.,* 2004, vol. 10, 5094-100
- **IWAI et al.** *Proc. Nat 7. Acad. ScL USA,* 2002, vol. 99, 12293-7
- **BROWN et al.** *J. Immunol.,* 2003, vol. 170, 1257-66
- **OKAZAKI et al.** *Curr Opin Immunol,* 2002, vol. 14, 391779-82
- **BENNETT et al.** *J Immunol YWJ1,* 2003, 1-8
- **NISHIMURA et al.** *Immunity H,* 1999, 141-51
- **NISHIMURA et al.** *Science,* 2001, vol. 291, 319-22
- **SALAMA et al.** *J Exp Med,* 2003, vol. 198, 71-78
- **PROKUNINA ; ALARCON-RIQUELME.** *Hum Mol Genet,* 2004, vol. 13, R143
- **NIELSEN et al.** *Lupus,* 2004, vol. 11, 510
- **OKAZAKI et al.** *PNAS,* 2001, vol. 98, 13866-71
- **D.Y. LIN et al.** *PNAS,* 2008, vol. 105, 3011-3016
- **KABAT, E.A. et al.** Sequences of Proteins of Immunological Interest. Public Health Service, National Institutes of Health, 1991
- **KABAT, E.A. et al.** Sequences of Proteins of Immunological Interest. NIH, 1991, vol. 1-3
- **CHOTHIA ; LESK.** *J. Mol. Biol.,* 1987, vol. 196, 901-917
- **KABAT et al.** Sequences of Proteins of Immunological Interest. Public Health Service, National Institutes of Health, 1991
- **MACCALLUM et al.** *J. Mol. Biol.,* 1996, vol. 262, 732-745
- **FLATMAN, S. et al.** *J. Chromatogr. B,* 2007, vol. 848, 79-87
- **KINDT, T.J. et al.** Kuby Immunology. W.H. Freeman and Co, 2007, 91
- **PORTOLANO, S. et al.** *J. Immunol.,* 1993, vol. 150, 880-887
- **CLACKSON, T. et al.** *Nature,* 1991, vol. 352, 624-628
- **CHEN, Y. et al.** *J. Mol. Biol.,* 1999, vol. 293, 865-881
- **HUDSON, P.J. et al.** *Nat. Med.,* 2003, vol. 9, 129-134
- **PLUECKTHUN, A.** The Pharmacology of Monoclonal Antibodies. Springer-Verlag, 1994, vol. 113, 269-315
- **HOLLIGER, P. et al.** *Proc. Natl. Acad. Sci. USA,* 1993, vol. 90, 6444-6448
- **HUDSON, P.J. et al.** *Nat. Med.,* vol. 9 (20039), 129-134

- **MORRISON, S.L. et al.** *Proc. Natl. Acad. Sci. USA,* 1984, vol. 81, 6851-6855
- **ALMAGRO, J.C. ; FRANSSON, J.** *Front. Biosci.,* 2008, vol. 13, 1619-1633
- **RIECHMANN, I. et al.** *Nature,* 1988, vol. 332, 323-329
- **QUEEN, C. et al.** *Proc. Natl. Acad. Sci. USA,* 1989, vol. 86, 10029-10033
- **KASHMIRI, S.V. et al.** *Methods,* 2005, vol. 36, 25-34
- **PADLAN, E.A.** *Mol. Immunol.,* 1991, vol. 28, 489-498
- **DALL'ACQUA, W.F. et al.** *Methods,* 2005, vol. 36, 43-60
- **OSBOURN, J. et al.** *Methods,* 2005, vol. 36, 61-68
- **KLIMKA, A. et al.** *Br. J. Cancer,* 2000, vol. 83, 252-260
- **SIMS, M.J. et al.** *J. Immunol.,* 1993, vol. 151, 2296-2308
- **CARTER, P. et al.** *Proc. Natl. Acad. Sci. USA,* 1992, vol. 89, 4285-4289
- **PRESTA, L.G. et al.** *J. Immunol.,* 1993, vol. 151, 2623-2632
- **BACA, M. et al.** *J. Biol. Chem.,* 1997, vol. 272, 10678-10684
- **ROSOK, M.J. et al.** *J. Biol. Chem.,* vol. 271 (19969), 22611-22618
- **VAN DIJK, M.A. ; VAN DE WINKEL, J.G.** *Curr. Opin. Pharmacol.,* 2001, vol. 5, 368-374
- **LONBERG, N.** *Curr. Opin. Immunol.,* 2008, vol. 20, 450-459
- **LONBERG, N.** *Nat. Biotech.,* 2005, vol. 23, 1117-1125
- **KOZBOR, D.** *J. Immunol.,* 1984, vol. 133, 3001-3005
- **BRODEUR, B.R. et al.** Monoclonal Antibody Production Techniques and Applications. Marcel Dekker, Inc, 1987, 51-63
- **BOERNER, P. et al.** *J. Immunol.,* 1991, vol. 147, 86-95
- **LI, J. et al.** *Proc. Natl. Acad. Sci. USA,* 2006, vol. 103, 3557-3562
- **NI, J.** *Xiandai Mianyixue,* 2006, vol. 26, 265-268
- **VOLLMERS, H.P. ; BRANDLEIN, S.** *Histology and Histopathology,* 2005, vol. 20, 927-937
- **VOLLMERS, H.P. ; BRANDLEIN, S.** *Methods and Findings in Experimental and Clinical Pharmacology,* 2005, vol. 27, 185-191
- **HOOGENBOOM, H.R. et al.** *Methods in Molecular Biology,* 2001, vol. 178, 1-37
- **MCCAFFERTY, J. et al.** *Nature,* 1990, vol. 348, 552-554
- **MARKS, J.D. et al.** *J. Mol. Biol.,* 1992, vol. 222, 581-597
- **MARKS, J.D. ; BRADBURY, A.** *Methods in Molecular Biology,* 2003, vol. 248, 161-175
- **SIDHU, S.S. et al.** *J. Mol. Biol.,* 2004, vol. 338, 299-310
- **LEE, C.V. et al.** *J. Mol. Biol.,* 2004, vol. 340, 1073-1093
- **FELLOUSE, F.A.** *Proc. Natl. Acad. Sci. USA,* 2004, vol. 101, 12467-12472
- **LEE, C.V. et al.** *J. Immunol. Methods,* 2004, vol. 284, 119-132
- **WINTER, G. et al.** *Ann. Rev. Immunol.,* 1994, vol. 12, 433-455
- **GRIFFITHS, A.D. et al.** *EMBO J.,* 1993, vol. 12, 725-734
- **HOOGENBOOM, H.R. ; WINTER, G.** *J. Mol. Biol.,* 1992, vol. 227, 381-388
- **MILSTEIN, C. ; CUELLO, A.C.** *Nature,* 1983, vol. 305, 537-540
- **TRAUNECKER, A. et al.** *EMBO J.,* 1991, vol. 10, 3655-3659
- **BRENNAN, M. et al.** *Science,* 1985, vol. 229, 81-83
- **KOSTELNY, S.A. et al.** *J. Immunol.,* 1992, vol. 148, 1547-1553
- **GRUBER, M et al.** *J. Immunol.,* 1994, vol. 152, 5368-5374
- **TUTT, A. et al.** *J. Immunol.,* 1991, vol. 147, 60-69
- **CHOWDHURY, P.S.** *Methods Mol. Biol.,* 2008, vol. 207, 179-196
- **HOOGENBOOM, H.R. et al.** *Methods in Molecular Biology,* 2002, vol. 178, 1-37
- **CUNNINGHAM, B.C. ; WELLS, J.A.** *Science,* 1989, vol. 244, 1081-1085
- **SHIELDS, R.L. et al.** *J. Biol. Chem.,* 2001, vol. 276, 6591-6604
- **GUYER, R.L. et al.** *J. Immunol.,* 1976, vol. 117, 587-593
- **KIM, J.K. et al.** *J. Immunol.,* 1994, vol. 24, 2429-2434
- **DUNCAN, A.R. ; WINTER, G.** *Nature,* 1988, vol. 322, 738-740
- **KAM, N.W. et al.** *Proc. Natl. Acad. Sci. USA,* 2005, vol. 102, 11600-11605
- **CHARLTON, K.A.** Methods in Molecular Biology. Humana Press, 2003, vol. 248, 245-254
- **GERNGROSS, T.U.** *Nat. Biotech.,* 2004, vol. 22, 1409-1414
- **LI, H. et al.** *Nat. Biotech.,* 2006, vol. 24, 210-215
- **GRAHAM, F.L. et al.** *J. Gen Virol.,* 1977, vol. 36, 59-74
- **MATHER, J.P.** *Biol. Reprod.,* 1980, vol. 23, 243-252
- **MATHER, J.P et al.** *Annals N.Y. Acad. Sci.,* 1982, vol. 383, 44-68
- **URLAUB, G. et al.** *Proc. Natl. Acad. Sci. USA,* 1980, vol. 77, 4216-4220
- **YAZAKI, P. ; WU, A.M.** Methods in Molecular Biology. Humana Press, 2004, vol. 248, 255-268

- Epitope Mapping Protocols. Methods in Molecular Biology. Humana Press, 1996, vol. 66
- **HARLOW, E. ; LANE, D.** Antibodies: A Laboratory Manual. Cold Spring Harbor Laboratory, 1988
- **HINMAN, L.M. et al.** *Cancer Res.,* 1993, vol. 53, 3336-3342
- **LODE, H.N. et al.** *Cancer Res.,* 1998, vol. 58, 2925-2928
- **KRATZ, F. et al.** *Curr. Med. Chem.,* 2006, vol. 13, 477-523
- **JEFFREY, S.C. et al.** *Bioorg. Med. Chem. Lett.,* 2006, vol. 16, 358-362
- **TORGOV, M.Y. et al.** *Bioconjug. Chem.,* 2005, vol. 16, 717-721
- **NAGY, A. et al.** *Proc. Natl. Acad. Sci. USA,* 2000, vol. 97, 829-834
- **DUBOWCHIK, G.M. et al.** *Bioorg. & Med. Chem. Letters,* 2002, vol. 12, 1529-1532
- **KING, H.D. et al.** *J. Med. Chem.,* vol. 45 (20029), 4336-4343
- **VITETTA, E.S. et al.** *Science,* 1987, vol. 238, 1098-1104
- **CHARI, R.V. et al.** *Cancer Res.,* 1992, vol. 52, 127-131
- Remington's Pharmaceutical Sciences. 1980
- **KABSCH, W.** Automatic processing of rotation diffraction data from crystals of initially unknown symmetry and cell constants. *J. Appl. Cryst.,* 1993, vol. 26, 795-800
- Collaborative Computational Project, N. The CCP4 suite: programs for protein crystallography. *Acta Crystallogr. D,* 1994, 760-763
- **EMSLEY, P. ; LOHKAMP, B. ; SCOTT, WG. ; COWTAN, K.** Features and development of COOT. *Acta Crystallogr. D Biol. Crystallogr.,* 2010, vol. 60, 486-501
- **BRICOGNE G. ; BLANC E. ; BRANDL M. ; FLENSBURG C. ; KELLER P. ; PACIOREK W. ; ROVERSI P ; SHARFF A. ; SMART O.S. ; VONRHEIN C.** BUSTER version 2.11.6. Global Phasing Ltd, 2016

SEQUENCE LISTING

<110> F. Hoffmann-La Roche AG

<120> ANTI-PD1 ANTIBODIES AND METHODS OF USE

<130> P33103-WO

<150> EP15188061.4
<151> 2015-10-02

<160> 77

<170> PatentIn version 3.5

<210> 1
<211> 7
<212> PRT
<213> Mus Musculus

<400> 1

```
Gly Phe Ser Phe Ser Ser Tyr
1               5
```

<210> 2
<211> 3
<212> PRT
<213> Mus Musculus

<400> 2

```
Gly Gly Arg
1
```

<210> 3
<211> 9
<212> PRT
<213> Mus Musculus

<400> 3

```
Thr Gly Arg Val Tyr Phe Ala Leu Asp
1               5
```

<210> 4
<211> 11
<212> PRT
<213> Mus Musculus

<400> 4

```
Ser Glu Ser Val Asp Thr Ser Asp Asn Ser Phe
1               5                   10
```

<210> 5
<211> 3
<212> PRT
<213> Mus Musculus

<400> 5

```
Arg Ser Ser
1
```

<210> 6
<211> 6
<212> PRT
<213> Mus Musculus

<400> 6

```
Asn Tyr Asp Val Pro Trp
1               5
```

<210> 7
<211> 120
<212> PRT
<213> Mus Musculus

<400> 7

```
Glu Val Ile Leu Val Glu Ser Gly Gly Gly Leu Val Lys Pro Gly Gly
1               5                   10                  15

Ser Leu Lys Leu Ser Cys Ala Ala Ser Gly Phe Ser Phe Ser Ser Tyr
            20                  25                  30

Thr Met Ser Trp Val Arg Gln Thr Pro Glu Lys Arg Leu Asp Trp Val
        35                  40                  45

Ala Thr Ile Ser Gly Gly Gly Arg Asp Ile Tyr Tyr Pro Asp Ser Val
    50                  55                  60

Lys Gly Arg Phe Thr Ile Ser Arg Asp Asn Ala Lys Asn Thr Leu Tyr
65                  70                  75                  80

Leu Glu Met Ser Ser Leu Met Ser Glu Asp Thr Ala Leu Tyr Tyr Cys
                85                  90                  95

Val Leu Leu Thr Gly Arg Val Tyr Phe Ala Leu Asp Ser Trp Gly Gln
            100                 105                 110

Gly Thr Ser Val Thr Val Ser Ser
        115                 120
```

<210> 8
<211> 111
<212> PRT
<213> Mus Musculus

<400> 8

```
Lys Ile Val Leu Thr Gln Ser Pro Ala Ser Leu Pro Val Ser Leu Gly
1               5                   10                  15

Gln Arg Ala Thr Ile Ser Cys Arg Ala Ser Glu Ser Val Asp Thr Ser
            20                  25                  30

Asp Asn Ser Phe Ile His Trp Tyr Gln Gln Arg Pro Gly Gln Ser Pro
        35                  40                  45

Lys Leu Leu Ile Tyr Arg Ser Ser Thr Leu Glu Ser Gly Val Pro Ala
    50                  55                  60

Arg Phe Ser Gly Ser Gly Ser Arg Thr Asp Phe Thr Leu Thr Ile Asp
65                  70                  75                  80

Pro Val Glu Ala Asp Asp Val Ala Thr Tyr Tyr Cys Gln Gln Asn Tyr
                85                  90                  95

Asp Val Pro Trp Thr Phe Gly Gly Gly Thr Lys Leu Glu Ile Lys
            100                 105                 110
```

<210> 9
<211> 8
<212> PRT
<213> Mus Musculus

<400> 9

```
Gly Tyr Ser Ile Thr Ser Asp Tyr
1               5
```

<210> 10
<211> 3
<212> PRT
<213> Mus Musculus

<400> 10

```
Tyr Ser Gly
1
```

<210> 11
<211> 9
<212> PRT
<213> Mus Musculus

<400> 11

```
His Gly Ser Ala Pro Trp Tyr Phe Asp
1               5
```

<210> 12
<211> 12

<212> PRT
<213> Mus Musculus

<400> 12

Ser Gln Asn Ile Val His Ser Asp Gly Asn Thr Tyr
1               5                   10

<210> 13
<211> 3
<212> PRT
<213> Mus Musculus

<400> 13

Lys Val Ser
1

<210> 14
<211> 6
<212> **PRT**
<213> Mus Musculus

<400> 14

Gly Ser His Phe Pro Leu
1               5

<210> 15
<211> 120
<212> PRT
<213> Mus Musculus

<400> 15

Asp Val Gln Leu Gln Glu Ser Gly Pro Gly Leu Val Lys Pro Ser Gln
1               5                   10                  15

Ser Leu Ser Leu Thr Cys Thr Val Thr Gly Tyr Ser Ile Thr Ser Asp
            20                  25                  30

Tyr Ala Trp Asn Trp Ile Arg Gln Phe Pro Gly Asp Lys Leu Glu Trp
        35                  40                  45

Leu Gly Tyr Ile Thr Tyr Ser Gly Phe Thr Asn Tyr Asn Pro Ser Leu
    50                  55                  60

Lys Ser Arg Ile Ser Ile Ser Arg Asp Thr Ser Lys Asn Gln Phe Phe
65                  70                  75                  80

Leu Gln Leu Asn Ser Val Ala Thr Glu Asp Thr Ala Thr Tyr Tyr Cys
                85                  90                  95

Ala Arg Trp His Gly Ser Ala Pro Trp Tyr Phe Asp Tyr Trp Gly Arg
100 105 110

Gly Thr Thr Leu Thr Val Ser Ser
115 120

<210> 16
<211> 112
<212> PRT
<213> Mus Musculus

<400> 16

Asp Val Leu Met Thr Gln Thr Pro Leu Ser Leu Pro Val Ser Leu Gly
1 5 10 15

Asp Gln Ala Ser Ile Ser Cys Arg Ser Ser Gln Asn Ile Val His Ser
20 25 30

Asp Gly Asn Thr Tyr Leu Glu Trp Tyr Leu Gln Lys Pro Gly Gln Ser
35 40 45

Pro Asn Leu Leu Ile Tyr Lys Val Ser Arg Arg Phe Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Leu Gly Val Tyr Tyr Cys Phe Gln Gly
85 90 95

Ser His Phe Pro Leu Thr Phe Gly Ala Gly Thr Lys Leu Glu Leu Lys
100 105 110

<210> 17
<211> 8
<212> PRT
<213> Mus Musculus

<400> 17

Gly Tyr Ser Ile Thr Ser Asp Tyr
1 5

<210> 18
<211> 3
<212> PRT
<213> Mus Musculus

<400> 18

Tyr Thr Gly
1

<210> 19
<211> 9
<212> PRT
<213> Mus Musculus

<400> 19

Met Asp Tyr Tyr Gly Ser Thr Leu Asp
1 5

<210> 20
<211> 11
<212> PRT
<213> Mus Musculus

<400> 20

Ser Glu Ser Val Asp Arg Tyr Gly Asn Ser Phe
1 5 10

<210> 21
<211> 3
<212> PRT
<213> Mus Musculus

<400> 21

Arg Ala Ser
1

<210> 22
<211> 6
<212> PRT
<213> Mus Musculus

<400> 22

Asn Asn Glu Asp Pro Tyr
1 5

<210> 23
<211> 120
<212> PRT
<213> Mus Musculus

<400> 23

```
Asp Val Gln Leu Gln Glu Ser Gly Pro Gly Leu Val Lys Pro Ser Gln
1               5                   10                  15

Ser Leu Ser Leu Thr Cys Thr Val Thr Gly Tyr Ser Ile Thr Ser Asp
            20                  25                  30

Tyr Ala Trp Asn Trp Ile Arg Gln Phe Pro Gly Asn Lys Leu Glu Trp
        35                  40                  45

Met Gly Tyr Ile Thr Tyr Thr Gly Arg Thr Ser Tyr Asn Pro Ser Leu
    50                  55                  60

Lys Ser Arg Ile Ser Ile Thr Arg Asp Thr Ser Lys Asn Gln Phe Phe
65                  70                  75                  80

Leu Gln Leu Asn Ser Val Thr Thr Glu Asp Thr Ala Thr Tyr Tyr Cys
                85                  90                  95

Ala Arg Glu Met Asp Tyr Tyr Gly Ser Thr Leu Asp Tyr Trp Gly Gln
            100                 105                 110

Gly Thr Thr Leu Thr Val Ser Ser
        115                 120
```

<210> 24
<211> 111
<212> PRT
<213> Mus Musculus

<400> 24

```
Lys Ile Val Leu Thr Gln Ser Pro Ala Ser Leu Ala Val Ser Leu Arg
1               5                   10                  15

Gln Arg Ala Thr Ile Ser Cys Arg Ala Ser Glu Ser Val Asp Arg Tyr
            20                  25                  30

Gly Asn Ser Phe Ile His Trp Tyr Gln Gln Lys Pro Gly Gln Pro Pro
        35                  40                  45

Lys Val Leu Ile Tyr Arg Ala Ser Asn Leu Glu Ser Gly Phe Pro Ala
    50                  55                  60

Arg Phe Ser Gly Ser Gly Ser Arg Thr Asp Phe Thr Leu Thr Ile Asp
65                  70                  75                  80

Pro Val Glu Ala Asp Asp Ala Ala Thr Tyr Tyr Cys Gln Gln Asn Asn
                85                  90                  95

Glu Asp Pro Tyr Thr Phe Gly Ser Gly Thr Lys Leu Glu Ile Lys
            100                 105                 110
```

<210> 25
<211> 7
<212> PRT
<213> Mus Musculus

<400> 25

```
Gly Tyr Thr Phe Thr Asp Tyr
1               5
```

<210> 26
<211> 3
<212> PRT
<213> Mus Musculus

<400> 26

```
Tyr Ser Gly
1
```

<210> 27
<211> 7
<212> PRT
<213> Mus Musculus

<400> 27

```
Gly Ile Thr Thr Gly Phe Ala
1               5
```

<210> 28
<211> 11

<212> PRT
<213> Mus Musculus

<400> 28

Ser Lys Gly Val Ser Thr Ser Ser Tyr Ser Phe
1 5 10

<210> 29
<211> 3
<212> PRT
<213> Mus Musculus

<400> 29

Tyr Ala Ser
1

<210> 30
<211> 6
<212> PRT
<213> Mus Musculus

<400> 30

Ser Arg Glu Phe Pro Trp
1 5

<210> 31
<211> 118
<212> PRT
<213> Mus Musculus

<400> 31

Gln Val Gln Leu Gln Gln Ser Gly Pro Glu Leu Val Arg Pro Gly Val
1               5                   10                  15

Ser Val Lys Ile Ser Cys Lys Gly Ser Gly Tyr Thr Phe Thr Asp Tyr
            20                  25                  30

Ala Met His Trp Val Lys Gln Ser His Ala Arg Thr Leu Glu Trp Ile
        35                  40                  45

Gly Val Ile Ser Thr Tyr Ser Gly Asp Thr Asn Tyr Asn Gln Lys Phe
    50                  55                  60

Lys Asp Lys Ala Thr Met Thr Val Asp Lys Ser Ser Ser Thr Ala Tyr
65                  70                  75                  80

Leu Glu Leu Ala Arg Met Thr Ser Glu Asp Ser Ala Ile Tyr Tyr Cys
                85                  90                  95

Ala Arg Leu Gly Ile Thr Thr Gly Phe Ala Tyr Trp Gly Gln Gly Thr
            100                 105                 110

Leu Val Thr Val Ser Ala
        115

<210> 32
<211> 111
<212> PRT
<213> Mus Musculus

<400> 32

Asp Ile Val Leu Thr Gln Ser Pro Ala Ser Leu Ala Val Ser Leu Gly
1               5                   10                  15

Gln Arg Ala Thr Ile Ser Cys Arg Ala Ser Lys Gly Val Ser Thr Ser
            20                  25                  30

Ser Tyr Ser Phe Met His Trp Tyr Gln Gln Lys Pro Arg Gln Pro Pro
        35                  40                  45

Lys Leu Leu Ile Lys Tyr Ala Ser Tyr Leu Glu Ser Gly Val Pro Ala
    50                  55                  60

Arg Phe Ser Gly Ser Gly Ser Gly Thr Asp Phe Thr Leu Asn Ile His
65                  70                  75                  80

```
Pro Val Glu Glu Glu Asp Ala Ala Thr Tyr Tyr Cys His His Ser Arg
                85                  90                  95

Glu Phe Pro Trp Thr Phe Gly Gly Gly Thr Lys Leu Glu Ile Lys
            100                 105                 110
```

<210> 33
<211> 7
<212> PRT
<213> Mus Musculus

<400> 33

```
Gly Phe Thr Phe Ser Asn Tyr
1               5
```

<210> 34
<211> 3
<212> PRT
<213> Mus Musculus

<400> 34

```
Gly Gly Arg
1
```

<210> 35
<211> 5
<212> PRT
<213> Mus Musculus

<400> 35

```
Tyr Tyr Gly Ile Asp
1               5
```

<210> 36
<211> 7
<212> PRT
<213> Mus Musculus

<400> 36

```
Ser Gln Asp Val Thr Thr Ala
1               5
```

<210> 37
<211> 3
<212> PRT
<213> Mus Musculus

<400> 37

```
Trp Ala Ser
1
```

<210> 38

<211> 6
<212> PRT
<213> Mus Musculus

<400> 38

```
His Tyr Ser Ile Pro Trp
1               5
```

<210> 39
<211> 116
<212> PRT
<213> Mus Musculus

<400> 39

```
Glu Val Lys Leu Val Glu Ser Gly Gly Gly Leu Val Lys Pro Gly Gly
1               5                   10                  15

Ser Leu Lys Leu Ser Cys Ala Ala Ser Gly Phe Thr Phe Ser Asn Tyr
            20                  25                  30

Gly Met Ser Trp Ile Arg Gln Thr Pro Glu Lys Gly Leu Glu Trp Val
        35                  40                  45

Ala Thr Ile Ser Gly Gly Gly Arg Asp Thr Tyr Tyr Pro Asp Ser Val
    50                  55                  60

Lys Gly Arg Phe Thr Ile Ser Arg Asp Asn Val Lys Asn Asn Leu Tyr
65                  70                  75                  80

Leu Gln Met Ser Ser Leu Arg Ser Glu Asp Thr Ala Phe Tyr Tyr Cys
                85                  90                  95

Ala Ser Tyr Tyr Tyr Gly Ile Asp Tyr Trp Gly Gln Gly Thr Ser Val
            100                 105                 110

Thr Val Ser Ser
        115
```

<210> 40
<211> 107
<212> PRT
<213> Mus Musculus

<400> 40

Asp Ile Val Met Thr Gln Pro His Lys Phe Met Ser Thr Ser Val Gly
1 5 10 15

Asp Arg Val Arg Ile Thr Cys Lys Ala Ser Gln Asp Val Thr Thr Ala
20 25 30

Val Ala Trp Tyr Gln Gln Lys Pro Gly Gln Ser Pro Lys Leu Leu Ile
35 40 45

Tyr Trp Ala Ser Thr Arg His Thr Gly Val Pro Asp Arg Phe Thr Gly
50 55 60

Ser Gly Ser Gly Thr Glu Phe Thr Leu Thr Ile Ser Ser Val Gln Ala
65 70 75 80

Glu Asp Leu Ala Leu Tyr Tyr Cys Gln Gln His Tyr Ser Ile Pro Trp
85 90 95

Thr Phe Gly Gly Gly Thr Lys Leu Glu Ile Lys
100 105

<210> 41
<211> 7
<212> PRT
<213> Mus Musculus

<400> 41

Gly Tyr Thr Phe Thr Ser Thr
1 5

<210> 42
<211> 3
<212> PRT
<213> Mus Musculus

<400> 42

Ser Asp Ser
1

<210> 43
<211> 3
<212> PRT
<213> Mus Musculus

<400> 43

Pro Phe Asp
1

<210> 44
<211> 7
<212> PRT

<213> Mus Musculus

<400> 44

Ser Gln Asp Val Ser Thr Ala
1 5

<210> 45
<211> 3
<212> PRT
<213> Mus Musculus

<400> 45

Ser Ala Ser
1

<210> 46
<211> 6
<212> PRT
<213> Mus Musculus

<400> 46

His Tyr Ser His Pro Phe
1 5

<210> 47
<211> 114
<212> PRT
<213> Mus Musculus

<400> 47

Gln Val Gln Leu Gln Gln Pro Gly Ala Glu Leu Val Lys Pro Gly Ala
1               5                   10                  15

Ser Val Lys Met Ser Cys Lys Ala Ser Gly Tyr Thr Phe Thr Ser Thr
            20                  25                  30

Trp Met His Trp Val Lys Gln Arg Pro Gly Gln Gly Leu Glu Trp Ile
        35                  40                  45

Gly Ala Ile Asp Pro Ser Asp Ser Tyr Thr Thr Tyr Asn Gln Lys Phe
    50                  55                  60

Lys Gly Lys Ala Thr Leu Thr Val Asp Thr Ser Ser Thr Thr Ala Tyr
65                  70                  75                  80

Met Gln Leu Ser Ser Leu Thr Ser Glu Asp Ser Ala Val Tyr Tyr Cys
                85                  90                  95

Thr Arg Ser Pro Phe Asp Tyr Trp Gly Gln Gly Thr Thr Leu Thr Val
            100                 105                 110

Ser Ser

<210> 48
<211> 107
<212> PRT
<213> Mus Musculus

<400> 48

```
Asp Ile Val Met Thr Gln Ser His Lys Phe Met Ser Thr Ser Val Gly
1               5                   10                  15

Asp Arg Val Ser Ile Thr Cys Lys Ala Ser Gln Asp Val Ser Thr Ala
            20                  25                  30

Val Ala Trp Tyr Gln Gln Lys Pro Gly Gln Ser Pro Lys Leu Leu Ile
        35                  40                  45

Tyr Ser Ala Ser Tyr Arg Tyr Thr Gly Val Pro Asp Arg Phe Thr Gly
    50                  55                  60

Ser Gly Ser Gly Thr Asp Phe Thr Phe Ala Ile Ser Ser Val Gln Ala
65                  70                  75                  80

Glu Asp Leu Ala Val Tyr Tyr Cys Gln Gln His Tyr Ser His Pro Phe
                85                  90                  95

Thr Phe Gly Ser Gly Thr Lys Leu Glu Ile Lys
            100                 105
```

<210> 49
<211> 8
<212> PRT
<213> Mus Musculus

<400> 49

```
Gly Tyr Ser Ile Thr Ser Gly Tyr
1               5
```

<210> 50
<211> 3
<212> PRT
<213> Mus Musculus

<400> 50

```
Ser Ser Gly
1
```

<210> 51
<211> 6
<212> PRT
<213> Mus Musculus

<400> 51

```
Arg Asn Trp Tyr Phe Asp
1               5
```

<210> 52
<211> 13

<212> PRT
<213> Mus Musculus

<400> 52

Ser Gln Ser Leu Leu Asn Ser Gly Thr Gln Lys Asn Tyr
1 5 10

<210> 53
<211> 3
<212> PRT
<213> Mus Musculus

<400> 53

Trp Ala Ser
1

<210> 54
<211> 6
<212> PRT
<213> Mus Musculus

<400> 54

Asp Tyr Thr Phe Pro Leu
1 5

<210> 55
<211> 117
<212> PRT
<213> Mus Musculus

<400> 55

Asp Val Gln Leu Gln Glu Ser Gly Pro Asp Leu Val Lys Pro Ser Gln
1 5 10 15

Ser Leu Ser Leu Thr Cys Thr Val Thr Gly Tyr Ser Ile Thr Ser Gly
20 25 30

Tyr Ser Trp His Trp Ile Arg Gln Phe Pro Gly Asn Lys Leu Glu Trp
35 40 45

Met Gly Phe Ile His Ser Ser Gly Asp Thr Asn Tyr Asn Pro Ser Leu
50 55 60

Lys Ser Arg Ile Ser Phe Thr Arg Asp Thr Ser Lys Asn Gln Phe Phe

65 70 75 80

Leu Gln Leu Ser Ser Leu Thr Asp Glu Asp Thr Ala Thr Tyr Tyr Cys
85 90 95

Ala Thr Tyr Arg Asn Trp Tyr Phe Asp Val Trp Gly Ala Gly Thr Thr
100 105 110

Val Thr Val Ser Ser
115

<210> 56
<211> 113
<212> PRT
<213> Mus Musculus

<400> 56

Asp Ile Val Met Thr Gln Ser Pro Ser Ser Leu Thr Val Thr Ala Gly
1 5 10 15

Glu Lys Val Thr Met Arg Cys Lys Ser Ser Gln Ser Leu Leu Asn Ser
20 25 30

Gly Thr Gln Lys Asn Tyr Leu Thr Trp Tyr Gln Gln Lys Pro Gly Gln
35 40 45

Pro Pro Lys Leu Leu Ile Tyr Trp Ala Ser Thr Arg Glu Ser Gly Val
50 55 60

Pro Asn Arg Phe Thr Gly Ser Gly Ser Gly Thr Asp Phe Thr Leu Thr
65 70 75 80

Ile Ser Ser Val Gln Ala Glu Asp Leu Ser Val Tyr Tyr Cys Gln Ser
85 90 95

Asp Tyr Thr Phe Pro Leu Thr Phe Gly Gly Gly Thr Lys Leu Glu Leu
100 105 110

Lys

<210> 57
<211> 120
<212> PRT
<213> Artificial

<220>
<223> humanized variant -heavy chain variable domain VH of PD1-0103_01

<400> 57

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Ser Phe Ser Ser Tyr
            20                  25                  30

Thr Met Ser Trp Val Arg Gln Ala Pro Gly Lys Gly Leu Glu Trp Val
        35                  40                  45

Ala Thr Ile Ser Gly Gly Gly Arg Asp Ile Tyr Tyr Pro Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Val Leu Leu Thr Gly Arg Val Tyr Phe Ala Leu Asp Ser Trp Gly Gln
            100                 105                 110

Gly Thr Leu Val Thr Val Ser Ser
        115                 120
```

<210> 58
<211> 111
<212> PRT
<213> Artificial

<220>
<223> humanized variant -light chain variable domain VL of PD1-0103_01

<400> 58

Asp Ile Val Met Thr Gln Ser Pro Asp Ser Leu Ala Val Ser Leu Gly
1 5 10 15

Glu Arg Ala Thr Ile Asn Cys Lys Ala Ser Glu Ser Val Asp Thr Ser
20 25 30

Asp Asn Ser Phe Ile His Trp Tyr Gln Gln Lys Pro Gly Gln Ser Pro
35 40 45

Lys Leu Leu Ile Tyr Arg Ser Ser Thr Leu Glu Ser Gly Val Pro Asp
50 55 60

Arg Phe Ser Gly Ser Gly Ser Gly Thr Asp Phe Thr Leu Thr Ile Ser
65 70 75 80

Ser Leu Gln Ala Glu Asp Val Ala Val Tyr Tyr Cys Gln Gln Asn Tyr
85 90 95

Asp Val Pro Trp Thr Phe Gly Gln Gly Thr Lys Val Glu Ile Lys
100 105 110

<210> 59
<211> 111
<212> PRT
<213> Artificial

<220>
<223> humanized variant -light chain variable domain VL of PD1-0103_02

<400> 59

```
Asp Val Val Met Thr Gln Ser Pro Leu Ser Leu Pro Val Thr Leu Gly
1               5                   10                  15

Gln Pro Ala Ser Ile Ser Cys Arg Ala Ser Glu Ser Val Asp Thr Ser
            20                  25                  30

Asp Asn Ser Phe Ile His Trp Tyr Gln Gln Arg Pro Gly Gln Ser Pro
        35                  40                  45

Arg Leu Leu Ile Tyr Arg Ser Ser Thr Leu Glu Ser Gly Val Pro Asp
    50                  55                  60

Arg Phe Ser Gly Ser Gly Ser Gly Thr Asp Phe Thr Leu Lys Ile Ser
65                  70                  75                  80

Arg Val Glu Ala Glu Asp Val Gly Val Tyr Tyr Cys Gln Gln Asn Tyr
                85                  90                  95

Asp Val Pro Trp Thr Phe Gly Gln Gly Thr Lys Val Glu Ile Lys
            100                 105                 110
```

<210> 60
<211> 111
<212> PRT
<213> Artificial

<220>
<223> humanized variant -light chain variable domain VL of PD1-0103_03

<400> 60

```
Glu Ile Val Leu Thr Gln Ser Pro Ala Thr Leu Ser Leu Ser Pro Gly
1               5                   10                  15

Glu Arg Ala Thr Leu Ser Cys Arg Ala Ser Glu Ser Val Asp Thr Ser
            20                  25                  30
```

Asp Asn Ser Phe Ile His Trp Tyr Gln Gln Lys Pro Gly Gln Ser Pro
35 40 45

Arg Leu Leu Ile Tyr Arg Ser Ser Thr Leu Glu Ser Gly Ile Pro Ala
50 55 60

Arg Phe Ser Gly Ser Gly Ser Gly Thr Asp Phe Thr Leu Thr Ile Ser
65 70 75 80

Ser Leu Glu Pro Glu Asp Phe Ala Val Tyr Tyr Cys Gln Gln Asn Tyr
85 90 95

Asp Val Pro Trp Thr Phe Gly Gln Gly Thr Lys Val Glu Ile Lys
100 105 110

<210> 61
<211> 111
<212> PRT
<213> Artificial

<220>
<223> humanized variant -light chain variable domain VL of PD1-0103_04

<400> 61

Glu Ile Val Leu Thr Gln Ser Pro Ala Thr Leu Ser Leu Ser Pro Gly
1 5 10 15

Glu Arg Ala Thr Leu Ser Cys Arg Ala Ser Glu Ser Val Asp Thr Ser
20 25 30

Asp Asn Ser Phe Ile His Trp Tyr Gln Gln Lys Pro Gly Gln Ser Pro
35 40 45

Arg Leu Leu Ile Tyr Arg Ser Ser Thr Leu Glu Ser Gly Ile Pro Ala
50 55 60

Arg Phe Ser Gly Ser Gly Ser Gly Thr Asp Phe Thr Leu Thr Ile Ser
65 70 75 80

Ser Leu Glu Pro Glu Asp Phe Ala Val Tyr Tyr Cys Gln Gln Asn Tyr
85 90 95

Asp Val Pro Trp Thr Phe Gly Gln Gly Thr Lys Val Glu Ile Lys
100 105 110

<210> 62
<211> 107
<212> PRT
<213> Homo Sapiens

<400> 62

```
Arg Thr Val Ala Ala Pro Ser Val Phe Ile Phe Pro Pro Ser Asp Glu
1               5                   10                  15

Gln Leu Lys Ser Gly Thr Ala Ser Val Val Cys Leu Leu Asn Asn Phe
            20                  25                  30

Tyr Pro Arg Glu Ala Lys Val Gln Trp Lys Val Asp Asn Ala Leu Gln
        35                  40                  45

Ser Gly Asn Ser Gln Glu Ser Val Thr Glu Gln Asp Ser Lys Asp Ser
    50                  55                  60

Thr Tyr Ser Leu Ser Ser Thr Leu Thr Leu Ser Lys Ala Asp Tyr Glu
65                  70                  75                  80

Lys His Lys Val Tyr Ala Cys Glu Val Thr His Gln Gly Leu Ser Ser
                85                  90                  95

Pro Val Thr Lys Ser Phe Asn Arg Gly Glu Cys
            100                 105
```

<210> 63
<211> 105
<212> PRT
<213> Homo Sapiens

<400> 63

```
Gln Pro Lys Ala Ala Pro Ser Val Thr Leu Phe Pro Pro Ser Ser Glu
1               5                   10                  15

Glu Leu Gln Ala Asn Lys Ala Thr Leu Val Cys Leu Ile Ser Asp Phe
            20                  25                  30

Tyr Pro Gly Ala Val Thr Val Ala Trp Lys Ala Asp Ser Ser Pro Val
        35                  40                  45

Lys Ala Gly Val Glu Thr Thr Thr Pro Ser Lys Gln Ser Asn Asn Lys
    50                  55                  60

Tyr Ala Ala Ser Ser Tyr Leu Ser Leu Thr Pro Glu Gln Trp Lys Ser
65                  70                  75                  80

His Arg Ser Tyr Ser Cys Gln Val Thr His Glu Gly Ser Thr Val Glu
                85                  90                  95

Lys Thr Val Ala Pro Thr Glu Cys Ser

                    100                 105
```

<210> 64
<211> 330
<212> PRT
<213> Homo Sapiens

<400> 64

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
```

```
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330
```

<210> 65
<211> 330
<212> PRT
<213> homo sapiens

<400> 65

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
```

```
                100                 105                 110

Pro Ala Pro Glu Ala Ala Gly Gly Pro Ser Val Phe Leu Phe Pro Pro
            115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330
```

<210> 66
<211> 330
<212> PRT

<213> homo sapiens

<400> 66

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Ala Ala Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Gly Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240
```

```
Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330
```

<210> 67
<211> 327
<212> PRT
<213> Homo Sapiens

<400> 67

```
Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Ser Cys Pro Ala Pro
            100                 105                 110

Glu Phe Leu Gly Gly Pro Ser Val Phe Leu Phe Pro Pro Lys Pro Lys
        115                 120                 125
```

Asp Thr Leu Met Ile Ser Arg Thr Pro Glu Val Thr Cys Val Val Val
130 135 140

Asp Val Ser Gln Glu Asp Pro Glu Val Gln Phe Asn Trp Tyr Val Asp
145 150 155 160

Gly Val Glu Val His Asn Ala Lys Thr Lys Pro Arg Glu Glu Gln Phe
165 170 175

Asn Ser Thr Tyr Arg Val Val Ser Val Leu Thr Val Leu His Gln Asp
180 185 190

Trp Leu Asn Gly Lys Glu Tyr Lys Cys Lys Val Ser Asn Lys Gly Leu
195 200 205

Pro Ser Ser Ile Glu Lys Thr Ile Ser Lys Ala Lys Gly Gln Pro Arg
210 215 220

Glu Pro Gln Val Tyr Thr Leu Pro Pro Ser Gln Glu Glu Met Thr Lys
225 230 235 240

Asn Gln Val Ser Leu Thr Cys Leu Val Lys Gly Phe Tyr Pro Ser Asp
245 250 255

Ile Ala Val Glu Trp Glu Ser Asn Gly Gln Pro Glu Asn Asn Tyr Lys
260 265 270

Thr Thr Pro Pro Val Leu Asp Ser Asp Gly Ser Phe Phe Leu Tyr Ser
275 280 285

Arg Leu Thr Val Asp Lys Ser Arg Trp Gln Glu Gly Asn Val Phe Ser
290 295 300

Cys Ser Val Met His Glu Ala Leu His Asn His Tyr Thr Gln Lys Ser
305 310 315 320

Leu Ser Leu Ser Leu Gly Lys
325

<210> 68
<211> 268
<212> PRT
<213> Homo Sapiens

<400> 68

Pro Gly Trp Phe Leu Asp Ser Pro Asp Arg Pro Trp Asn Pro Pro Thr
1 5 10 15

```
Phe Ser Pro Ala Leu Leu Val Val Thr Glu Gly Asp Asn Ala Thr Phe
            20                  25                  30

Thr Cys Ser Phe Ser Asn Thr Ser Glu Ser Phe Val Leu Asn Trp Tyr
        35                  40                  45

Arg Met Ser Pro Ser Asn Gln Thr Asp Lys Leu Ala Ala Phe Pro Glu
    50                  55                  60

Asp Arg Ser Gln Pro Gly Gln Asp Cys Arg Phe Arg Val Thr Gln Leu
65                  70                  75                  80

Pro Asn Gly Arg Asp Phe His Met Ser Val Val Arg Ala Arg Arg Asn
                85                  90                  95

Asp Ser Gly Thr Tyr Leu Cys Gly Ala Ile Ser Leu Ala Pro Lys Ala
            100                 105                 110

Gln Ile Lys Glu Ser Leu Arg Ala Glu Leu Arg Val Thr Glu Arg Arg
        115                 120                 125

Ala Glu Val Pro Thr Ala His Pro Ser Pro Ser Pro Arg Pro Ala Gly
    130                 135                 140

Gln Phe Gln Thr Leu Val Val Gly Val Val Gly Gly Leu Leu Gly Ser
145                 150                 155                 160

Leu Val Leu Leu Val Trp Val Leu Ala Val Ile Cys Ser Arg Ala Ala
                165                 170                 175

Arg Gly Thr Ile Gly Ala Arg Arg Thr Gly Gln Pro Leu Lys Glu Asp
            180                 185                 190

Pro Ser Ala Val Pro Val Phe Ser Val Asp Tyr Gly Glu Leu Asp Phe
        195                 200                 205

Gln Trp Arg Glu Lys Thr Pro Glu Pro Pro Val Pro Cys Val Pro Glu
    210                 215                 220

Gln Thr Glu Tyr Ala Thr Ile Val Phe Pro Ser Gly Met Gly Thr Ser
225                 230                 235                 240

Ser Pro Ala Arg Arg Gly Ser Ala Asp Gly Pro Arg Ser Ala Gln Pro
                245                 250                 255

Leu Arg Pro Glu Asp Gly His Cys Ser Trp Pro Leu
            260                 265
```

<210> 69
<211> 150
<212> PRT
<213> Homo Sapiens

<400> 69

```
Pro Gly Trp Phe Leu Asp Ser Pro Asp Arg Pro Trp Asn Pro Pro Thr
1               5                   10                  15

Phe Ser Pro Ala Leu Leu Val Val Thr Glu Gly Asp Asn Ala Thr Phe
            20                  25                  30

Thr Cys Ser Phe Ser Asn Thr Ser Glu Ser Phe Val Leu Asn Trp Tyr
        35                  40                  45

Arg Met Ser Pro Ser Asn Gln Thr Asp Lys Leu Ala Ala Phe Pro Glu
    50                  55                  60

Asp Arg Ser Gln Pro Gly Gln Asp Cys Arg Phe Arg Val Thr Gln Leu
65                  70                  75                  80

Pro Asn Gly Arg Asp Phe His Met Ser Val Val Arg Ala Arg Arg Asn
                85                  90                  95

Asp Ser Gly Thr Tyr Leu Cys Gly Ala Ile Ser Leu Ala Pro Lys Ala
            100                 105                 110

Gln Ile Lys Glu Ser Leu Arg Ala Glu Leu Arg Val Thr Glu Arg Arg
        115                 120                 125

Ala Glu Val Pro Thr Ala His Pro Ser Pro Ser Pro Arg Pro Ala Gly
    130                 135                 140

Gln Phe Gln Thr Leu Val
145                 150
```

<210> 70
<211> 288
<212> PRT
<213> homo sapiens

<400> 70

```
Met Gln Ile Pro Gln Ala Pro Trp Pro Val Val Trp Ala Val Leu Gln
1               5                   10                  15

Leu Gly Trp Arg Pro Gly Trp Phe Leu Asp Ser Pro Asp Arg Pro Trp
            20                  25                  30
```

```
Asn Pro Pro Thr Phe Ser Pro Ala Leu Leu Val Val Thr Glu Gly Asp
        35                  40                  45

Asn Ala Thr Phe Thr Cys Ser Phe Ser Asn Thr Ser Glu Ser Phe Val
    50                  55                  60

Leu Asn Trp Tyr Arg Met Ser Pro Ser Asn Gln Thr Asp Lys Leu Ala
65                  70                  75                  80

Ala Phe Pro Glu Asp Arg Ser Gln Pro Gly Gln Asp Cys Arg Phe Arg
                85                  90                  95

Val Thr Gln Leu Pro Asn Gly Arg Asp Phe His Met Ser Val Val Arg
            100                 105                 110

Ala Arg Arg Asn Asp Ser Gly Thr Tyr Leu Cys Gly Ala Ile Ser Leu
        115                 120                 125

Ala Pro Lys Ala Gln Ile Lys Glu Ser Leu Arg Ala Glu Leu Arg Val
    130                 135                 140

Thr Glu Arg Arg Ala Glu Val Pro Thr Ala His Pro Ser Pro Ser Pro
145                 150                 155                 160

Arg Pro Ala Gly Gln Phe Gln Thr Leu Val Val Gly Val Val Gly Gly
                165                 170                 175

Leu Leu Gly Ser Leu Val Leu Leu Val Trp Val Leu Ala Val Ile Cys
            180                 185                 190

Ser Arg Ala Ala Arg Gly Thr Ile Gly Ala Arg Arg Thr Gly Gln Pro
        195                 200                 205

Leu Lys Glu Asp Pro Ser Ala Val Pro Val Phe Ser Val Asp Tyr Gly
    210                 215                 220

Glu Leu Asp Phe Gln Trp Arg Glu Lys Thr Pro Glu Pro Pro Val Pro
225                 230                 235                 240

Cys Val Pro Glu Gln Thr Glu Tyr Ala Thr Ile Val Phe Pro Ser Gly
                245                 250                 255

Met Gly Thr Ser Ser Pro Ala Arg Arg Gly Ser Ala Asp Gly Pro Arg
            260                 265                 270

Ser Ala Gln Pro Leu Arg Pro Glu Asp Gly His Cys Ser Trp Pro Leu
        275                 280                 285
```

<210> 71
<211> 4
<212> PRT
<213> Artificial

<220>
<223> Minimal HVR1 of PD1-0103 and PD1-0103 humanized variant PD1-0103-0312, PD1-0103-0313, PD1-0103-0314 , and PD1-0103-0315

<400> 71

Ser Ser Tyr Thr
1

<210> 72
<211> 8
<212> PRT
<213> Artificial

<220>
<223> Minimal HVR2 of PD1-0103 and PD1-0103 humanized variant PD1-0103-0312, PD1-0103-0313, PD1-0103-0314 , and PD1-0103-0315

<400> 72

Ser Gly Gly Gly Arg Asp Ile Tyr
1               5

<210> 73
<211> 5
<212> PRT
<213> Artificial

<220>
<223> Minimal HVR3 of PD1-0103 and PD1-0103 humanized variant PD1-0103-0312, PD1-0103-0313, PD1-0103-0314 , and PD1-0103-0315

<400> 73

Gly Arg Val Tyr Phe
1               5

<210> 74
<211> 6
<212> PRT
<213> Artificial

<220>
<223> Minimal LVR1 of PD1-0103 and PD1-0103 humanized variant PD1-0103-0312, PD1-0103-0313, PD1-0103-0314 , and PD1-0103-0315

<400> 74

Thr Ser Asp Asn Ser Phe
1               5

<210> 75
<211> 7

<212> PRT
<213> Artificial

<220>
<223> Minimal LVR2 of PD1-0103 and PD1-0103 humanized variant PD1-0103-0312, PD1-0103-0313, PD1-0103-0314 , and PD1-0103-0315

<400> 75

```
Arg Ser Ser Thr Leu Glu Ser
1               5
```

<210> 76
<211> 6
<212> PRT
<213> Artificial

<220>
<223> Minimal LVR3 of PD1-0103 and PD1-0103 humanized variant PD1-0103-0312, PD1-0103-0313, PD1-0103-0314 , and PD1-0103-0315

<400> 76

```
Asn Tyr Asp Val Pro Trp
1               5
```

<210> 77
<211> 3
<212> PRT
<213> Artificial

<220>
<223> fragment of FR-H3 comprising the amino acid sequence RDN at positions of 71, 72, 73 according to Kabat numbering

<400> 77

```
Arg Asp Asn
1
```